# EUROPEAN PATENT APPLICATION

(11) **EP 4 737 436 A1**
(43) Date of publication of application: **06.05.2026**
(21) Application number: 24830348.9
(22) Date of filing: 28.05.2024
(51) Int. Cl.: C07C 229/26, A61K 47/18, A61K 9/127, A61K 39/00

(54) **CATIONIC LIPID CONTAINING TWO TERTIARY AMINE GROUPS, AND USE THEREOF**

(30) Priority: 30.06.2023 CN 202310795706
(71) Applicant: Xiamen Sinopeg Biotech Co., Ltd, Xiamen, Fujian 361100 (CN)
(72) Inventor: LIN, Sheng, Xiamen, Fujian 361100 (CN); LIN, Minggui, Xiamen, Fujian 361100 (CN); WANG, Linlin, Xiamen, Fujian 361100 (CN); WANG, Ailan, Xiamen, Fujian 361100 (CN); WEI, Guohua, Xiamen, Fujian 361100 (CN); ZHU, Qi, Xiamen, Fujian 361100 (CN); WENG, Wengui, Xiamen, Fujian 361100 (CN); LIU, Chao, Xiamen, Fujian 361100 (CN)
(74) Representative: Algemeen Octrooi- en Merkenbureau B.V.
(86) International application number: PCT/CN2024/095788
(87) International publication number: WO 2025/001700

(57) **Abstract**

The present application provides a novel cationic lipid containing two tertiary amine groups, represented by the general formula (1), wherein each symbol is defined as described herein. This cationic lipid containing two tertiary amine groups is pharmaceutically acceptable, biodegradable, or highly biocompatible, offering advantages such as low toxicity, low immunogenicity, and high biocompatibility. One or more biodegradable groups are incorporated between the hydrophilic head and lipophilic tail chains of the cationic lipid containing two tertiary amine groups in the present application, which prevents endosomal accumulation in the LNPs prepared therefrom, enhances the endosomal escape efficiency of LNPs within cells, and promotes the release of drugs into the cytoplasm to exert their effects.

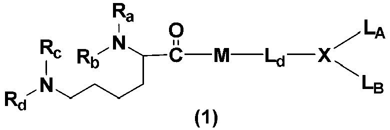

## Description

### TECHNICAL FIELD

The present application belongs to the field of drug delivery, relating to a cationic lipid in general, in particular to a cationic lipid containing two tertiary amine groups, which can be used as a drug carrier component, and a lipid composition containing the cationic lipid, a lipid pharmaceutical composition, and formulations and applications thereof.

### BACKGROUND

Lipid nanoparticles (LNPs) are spherical particles made of homogeneous lipids, which enter cells through endocytosis and are transported to endosomes. Then, through the endosomal escape process, the contents of the LNP, such as nucleic acids, are released into the cytoplasm. In the field of delivery, LNP has the advantages of easy preparation, modularity, good biocompatibility and strong payload capacity, and is widely used to deliver various drugs, such as small molecule drug, peptide, protein, nucleic acid, etc., in order to improve the stability and solubility of drugs, enhance the delivery effect, achieve directional delivery and reduce the toxic and side effects of drugs. LNP drug delivery systems also have the advantage of being highly tunable in nature, and their stability, uptake, toxicity, side effects, drug release rates, and targeting properties can be adjusted by changing lipid component contents or lipid species, adding targeting functional molecules, and surface modifications. In addition, LNPs support both re-administration and transient administration, and the safety has been validated through the widespread use of COVID-19 vaccines.

LNPs typically contain four lipid components, including a phospholipid, a cationic lipid, a cholesterol, and a PEGylated lipid; wherein, the cationic lipid interacts with drug molecules (e.g., negatively charged nucleic acids) through electrostatic interaction; the phospholipid lipid prevents lipid oxidation or link ligands to the surface of lipid nanoparticles. The steroid lipid has strong membrane fusion ability, which promotes the intracellular uptake and cytosolic entry of drug molecules. The PEGylated lipid is located on the surface of lipid nanoparticles, which can improve the hydrophilicity, avoid rapid removal by the immune system, prevent particle aggregation, and increase stability.

Despite recent advances in the use of cationic lipid for drug delivery, there is a need for alternative cationic lipids suitable for conventional therapeutic use. In literature CN114751835A, a series of ionizable tertiary amine lipids containing multiple ester bonds were prepared, and the preparation method was complex and cumbersome, requiring at least seven chemical reactions to obtain a cationic lipid with asymmetrical tail chains and two tertiary amine structures. A number of lipids featuring an amino acid core have been disclosed in CN101674853A; wherein, the amino acid-based lipid with a lysine core contains only one tertiary amine structure and two saturated and unsaturated tails, which is not conducive to the formation of conical geometry, incapable of promoting the destabilization of endosomal membranes and the intracytoplasmic release of nucleic acids.

The object of the present application is to provide a class of cationic lipids containing two tertiary amine groups with simple preparation methods, low cytotoxicity, and high gene transfection efficiency in vitro, and synthesis methods and application thereof.

### SUMMARY

To solve the problems above, the application provides a novel cationic lipid and preparation methods thereof, a lipid composition containing the cationic lipid, a lipid pharmaceutical composition containing the lipid composition and formulation thereof, and a liposome or a lipid nanoparticle containing the lipid composition; especially, the LNP-nucleic acid pharmaceutical composition containing the cationic lipid and formulation thereof are provided, with the advantages of high delivery efficiency, safety and low toxicity, and high biocompatibility, which can improve the therapeutic and/or preventive effects of drugs.

The above-described purposes of this application can be realized via the embodiments below.

In one embodiment, provided herein is a cationic lipid:
A cationic lipid, wherein the structure is represented by the general formula (1): 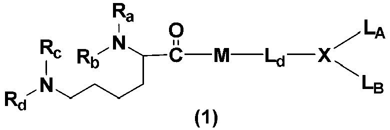
wherein, Rₐ, R_{b}, R_{c} and R_{d} are each independently a C₁₋₃ alkyl group; or, Rₐ and R_{b}, together with the nitrogen atom to which they are connected, form a nitrogen-containing heterocyclic group; or, R_{c} and R_{d}, together with the nitrogen atom to which they are connected, form a nitrogen-containing heterocyclic group;
M is O, S, or NH;
L_{d} is a linking bond or a divalent linking group;
X is N or CRₘ, and the Rₘ is H or a C₁₋₁₂ alkyl group;
L_{A} is represented by -B₃-L₃-B₁-L₁-R₁, and L_{B} is represented by -B₄-L₄-B₂-L₂-R₂; wherein, L₁, L₂, L₃, and L₄ are each independently a linking bond or a divalent linking group L; B₁, B₂, B₃, and B₄ are each independently a linking bond or a C₁₋₂₀ alkylene group; R₁ and R₂ are each independently a C₁₋₃₀ hydrocarbon group or a C₁₋₃₀ hydrocarbon derivative residue containing 1 to 4 units of -O-;
or a pharmaceutically acceptable salt, tautomer, stereoisomer, deuterated derivative, or solvate thereof.

The present application also provides a lipid composition, embodied as follows:
A lipid composition containing a cationic lipid with the structure represented by the formula (1).

The present application also provides a lipid pharmaceutical composition, embodied as follows:
A lipid pharmaceutical composition containing a lipid composition and a drug, and the lipid composition contains a cationic lipid with the structure represented by the formula (1). The drug is selected from any one of a nucleic acid drug, a gene vaccine, an anti-tumor drug, a small molecule drug, a peptide drug, or a protein drug.

The present application also provides a formulation of lipid pharmaceutical composition, embodied as follows:
A formulation of lipid pharmaceutical composition containing the aforementioned lipid pharmaceutical composition and a pharmaceutically acceptable diluent or excipient.

The present application also provides a liposome or lipid nanoparticle, embodied as follows:
A liposome or lipid nanoparticle containing a lipid composition, wherein the lipid composition contains a cationic lipid with the structure represented by formula (1).

### Compared with the prior art, the present application brings the following beneficial effects:

The cationic lipid containing two tertiary amine groups of the present application has a hydrophilic head and lipophilic tails. The hydrophilic head can be derived from the tertiary amine groups of the amino acid residue, and the lipophilic tails contain one or more types of saturated or unsaturated lipophilic tail chains. The cationic lipid can be used to prepare liposomes or lipid nanoparticles, and the unsaturated group in the lipophilic tail chain can regulate the fluidity of the membrane to affect the delivery of nucleic acids. The lipid nanoparticles of the present application, prepared from the cationic lipid with lipophilic tail chains containing alkenyl or alkynyl groups, exhibit more effective transfection.

The hydrophilic head of the cationic lipid containing two tertiary amine groups of the present application features tertiary amine groups of a lysine derivative, exhibiting high biocompatibility. The lysine or lysine derivatives used as starting materials in the preparation process are easy to obtain, either from natural sources or through straightforward synthesis, offering the advantages of simplicity, safety, and cost-effectiveness in production.

Compared with the lysine-branched cationic lipid which contains only one tertiary amine group, the cationic lipid of the present application, which contains two tertiary amine groups, exhibits higher nucleic acid delivery efficiency, and requires a smaller amount of lipid to deliver the same nucleic acid, offering the advantages of increased efficiency and reduced production cost.

The cationic lipid containing two tertiary amine groups of the present application contains one or more biodegradable groups between the hydrophilic head and the lipophilic tail chains. The biodegradable groups are stable at physiological pH, and can be enzymatically hydrolyzed in the cell to form a plurality of low- or non-toxic small-molecule acids, alcohols and amines; consequently, the cytotoxicity of the lipid is further reduced, and the accumulation of LNPs in endosomes is avoided, thereby promoting the endosome escape and improving the delivery efficiency.

The preparation process of the cationic lipid containing two tertiary amine groups of the present application adopts lipase-catalyzed reactions, wherein the lipases are highly active, highly selective, readily available, and can be recycled and reused.

### DETAILED DESCRIPTION OF THE APPLICATION

### 1. Description of Terms

In the present application, unless otherwise described, terms have the following meanings.

In the present application, unless otherwise specified, when the structure involved has isomers, it can refer to any one of the isomers. For example, when *cis-* and *trans-* isomers are present, it can refer to either a *cis*-structure or a *trans*-structure; when *E*/*Z* isomers are present, it can refer to either an (*E*)-structure or a (*Z*)-structure; and when optical activity is present, it can refer to either a laevoisomer or a dextroisomer.

In the present application, the numerical interval includes both the numerical interval marked by a hyphen (e.g., 1-6) and the numerical interval marked by a tilde (e.g., 1~6). Unless otherwise specified, an integer interval represents the group of all integers within the range of the interval, and the range includes two endpoints. For example, the integer range 1-6 represents the group consisting of 1, 2, 3, 4, 5, and 6. The numerical range in the present application includes but is not limited to the numerical intervals represented by integers, non-integers, percentages, and fractions, and the numerical intervals include two endpoints unless otherwise specified.

In the present application, "about" or "approximately" followed by a numerical value generally suggests a numerical range of ±10%, which may, in some cases, be magnified to ±15%, but not exceeding ±20%, based on the preset value. For example, when the molar percentage of steroid lipid in the total lipids is about 40%, it is generally assumed that the molar percentage of steroid lipid is 30% to 50%.

In the present application, "pharmaceutically acceptable salt" refers to a relatively non-toxic, inorganic acid or organic acid addition salt of the compound of the present application. See, e.g., S. M. Berge et al., "Pharmaceutical Salts", J. Pharm. Sci. 1977, 66, 1-19. Wherein, exemplary inorganic acids include hydrochloric acid, hydrobromic acid, hydroiodic acid, sulfuric acid, phosphoric acid, or nitric acid, etc.; exemplary organic acids include formic acid, acetic acid, acetoacetic acid, pyruvate, trifluoroacetic acid, propionic acid, butyric acid, caproic acid, heptanoic acid, undecanoic acid, lauric acid, benzoic acid, salicylic acid, 2-(4-hydroxybenzoyl)-benzoic acid, camphoric acid, cinnamic acid, cyclopentanepropionic acid, digluconic acid, 3-hydroxy-2-naphthoic acid, niacin, bamoic acid, pectin ester acid, 3-phenylpropionic acid, picric acid, pivalic acid, 2-hydroxyethanesulfonic acid, itaconic acid, aminesulfonic acid, trifluoromethanesulfonic acid, lauryl sulfuric acid, ethanesulfonic acid, benzenesulfonic acid, p-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptonic acid, glycerophosphoric acid, aspartic acid, sulfosalicylic acid, etc. For example, HCl (or hydrochloric acid), HBr (or hydrobromic acid solution), methanesulfonic acid, sulfuric acid, tartaric acid or fumaric acid may be used to form a pharmaceutically acceptable salt with the compound represented by the formula (1).

In the present application, the "solvate" refers to a complex formed by the compound of formula (1) or a pharmaceutically acceptable salt thereof with a solvent (e.g., ethanol or water). It should be understood that any solvate of formula (1) used in the treatment of a disease or condition, although it may provide different properties (including pharmacokinetic properties), once absorbed into the subject, will result in the compound of formula (1), such that the use of a compound of formula (1) covers the use of any solvate of compound of formula (1), respectively. It should be further understood that the compound of formula (1) or a pharmaceutically acceptable salt thereof may be isolated in the form of a solvate, and therefore any said solvate is included within the scope of the present application. For example, a compound of formula (1) or a pharmaceutically acceptable salt thereof may exist in unsolvated form or in solvated form with a pharmaceutically acceptable solvent (e.g., water, ethanol, etc.).

In the present application, unless otherwise specified, the terms "include", "contain", "comprise", and similar expressions shall be interpreted in an open and inclusive manner as "including but not limited to" in the description and claims.

In the present application, when two or more objects are "each independently preferably" selected from multiple levels of preferable options, the objects are not necessarily selected from preferable options of the same level. It is allowed that one is selected from a wider range of preferable options while another one is selected from a narrower range of preferable options. It is also allowed that one is selected from the maximum range of preferable options while another is selected from any allowable preferable options. It is also allowed that the objects are selected from preferable options of the same level.

In the present application and unless otherwise specified, for divalent linking group, e.g., a hydrocarbylene group, an alkylene group, an arylene group, an amide bond, and the like, either one of the two connection ends may be chosen to be connected to another group. For example, when an amide bond serves as a divalent linking group between C-CH₂CH₂- and -CH₂-D, both C-CH₂CH₂-C(=O)NH-CH₂-D and C-CH₂CH₂-NHC(=O)-CH₂-D are allowable.

In the present application, when distinguishing the terminus from the substituents of a linking group becomes questionable, 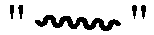 is used to indicate the connection location between the linking group and the other group. For example, in structural formulas 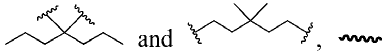 are used to indicate the connection locations between the divalent linking groups and the other groups; the two structural formulas mentioned above represent -CH(CH₂CH₂CH₃)₂- and -CH₂CH₂CH(CH₃)₂-CH₂CH₂-, respectively.

In the present application, a numerical interval written in the subscript of "C" can be used to indicate the number of carbon atoms in a group. For example, a C₁₋₁₂ indicates "having 1 to 12 carbon atoms"; C₁₋₃₀ indicates "having 1 to 30 carbon atoms". "substituted C₁₋₁₂ alkyl group" means a C₁₋₁₂ alkyl group with one or more hydrogen atoms being substituted. "C₁₋₁₂ substituted alkyl group" means an alkyl group with one or more hydrogen atoms being substituted, with 1 to 12 carbon atoms remaining. As another example, when a group can be selected from C₁₋₁₂ alkylene groups, it can be any alkylene group with the number of carbon atoms in the range indicated by the subscript, that is, the group can be selected from the group consisting of C₁, C₂, C₃, C₄, C₅, C₆, C₇, C₈, C₉, C₁₀, C₁₁, and C₁₂ alkylene groups. In the present application and unless otherwise specified, a subscript being a numerical interval indicates that the subscript can be any integer within the interval which includes two endpoints.

In the present application, heteroatoms are not particularly limited, including but not limited to O, S, N, P, Si, F, Cl, Br, I, B, etc.

In the present application, the heteroatom used for substitution is referred to as "substituent atom", and the group used for substitution is referred to as "substituent group".

In the present application, the term "substituted" indicates that at least one hydrogen atom of any group (e.g., a hydrocarbon group, an alkyl group, an alkenyl group, an alkynyl group, an alkoxy group, or an alkylene group) is replaced by a bond connected to a non-hydrogen atom, the non-hydrogen atom including but not limited to a C₁₋₁₂ alkyl group, a C₁₋₁₂ cycloalkyl group, a C₂₋₁₂ alkenyl group, a C₂₋₁₂ alkynyl group, halogen atom (F, Cl, Br, or I), an oxo group (=O), a hydroxyl group (-OH), a hydrocarbyloxy group (-ORₙ; wherein, Rₙ is a C₁₋₁₂ alkyl group), a carboxyl group (-COOH), or an amine group (-NRₘRₘ; wherein, both Rₘ are each independently H or a C₁₋₁₂ alkyl group). In some embodiments, the substituent group is preferably selected from the group consisting of a C₁₋₁₂ alkyl group, a C₁₋₁₂ cycloalkyl group, F, a hydroxyl group, an alkoxy group, a carboxyl group and an amino group.

In the present application, "optional" or "optionally" (e.g., optionally substituted) means that the event of the situation described thereafter may or may not occur, and the description includes instances in which the event or situation occurs as well as instances in which the event or situation does not occur. For example, "optionally substituted hydrocarbon groups" means that the hydrocarbon groups may or may not be substituted, and the description includes both substituted and unsubstituted hydrocarbon groups.

In the present application, a compound or a group can be substituted and heteroatom-incorporated simultaneously, e.g., by replacing a hydrogen atom with a nitrophenyl group, or by replacing -CH₂-CH₂-CH₂- with -CH₂-S-CH(CH₃)-.

In the present application, a "linking bond" is for connection only and does not contain any atoms. When the definition of a group includes a linking bond, it indicates that the group may be absent.

In the present application, "each independently at each occurrence" not only means that different groups can be each independently selected from the definitions but also means that the same group at different positions can be independently selected from the definitions. For example, two R_{g} in "-NR_{g}C(=O)NR_{g}-" can be the same or different, which are independently a hydrogen atom or a C₁₋₁₂ alkyl group.

In the present application, a "group" contains at least one atom, referring to the radical formed by a compound losing one or more atoms. Relative to a compound, the remaining group formed by the removal of other groups is also referred to as "residue". The valence of groups is not particularly limited, and examples include a monovalent group, a divalent group, a trivalent group, a tetravalent group, ..., a hectovalent group, etc. Wherein, groups with valence being equal to or greater than two are collectively defined as linking groups. The linking group can also contain only one atom, such as oxo group or thioxo group.

In the present application, "hydrocarbon" refers to a class of compounds that contain only carbon atoms and hydrogen atoms.

In the present application, hydrocarbons are classified into aliphatic hydrocarbons and aromatic hydrocarbons in terms of the type of hydrocarbon groups. Hydrocarbons containing neither phenyl rings nor hydrocarbyl-substituted phenyl rings are defined as aliphatic hydrocarbons. Hydrocarbons containing at least one phenyl ring or hydrocarbyl-substituted phenyl ring are defined as aromatic hydrocarbons. In addition, aromatic hydrocarbon can contain aliphatic hydrocarbon structures, such as toluene, diphenylmethane, 2,3-dihydroindene, etc.

In the present application, hydrocarbons are classified into saturated hydrocarbons and unsaturated hydrocarbons in terms of the degree of saturation. All aromatic hydrocarbons are unsaturated hydrocarbons. Saturated aliphatic hydrocarbons are also termed alkanes. There is no specific limit to the degree of unsaturation of unsaturated aliphatic hydrocarbons. For example, unsaturated aliphatic hydrocarbons include but are not limited to alkenes (containing carbon-carbon double bonds), alkynes (containing carbon-carbon triple bonds), dienes (containing conjugated carbon-carbon double bonds), and the like. When the aliphatic moieties of aromatic hydrocarbons are saturated, the aromatic hydrocarbons are also termed aralkanes, such as toluene.

In the present application, the structures of hydrocarbons are not particularly limited, including linear structures without pendant groups, branched structures with pendant groups, ring-containing structures, dendritic structures, comb structures, hyperbranched structures, etc. Unless otherwise specified, preferable structures include linear structures without pendant groups, branched structures with pendant groups, and ring-containing structures, corresponding to linear hydrocarbons, branched hydrocarbons and cyclic hydrocarbons, respectively. Wherein, hydrocarbons that contain no rings are termed open-chain hydrocarbons, including but not limited to linear structures without pendant groups, and branched structures with pendant groups. Open-chain hydrocarbons belong to aliphatic hydrocarbons. Therefore, linear hydrocarbons are also referred to as linear aliphatic hydrocarbons. Branched hydrocarbons are also referred to as branched aliphatic hydrocarbons.

In the present application, hydrocarbons with any carbon atom replaced by heteroatom are generally referred to as heterohydrocarbons.

In the present application, "hydrocarbon group" refers to the residue of a hydrocarbon molecule with at least one hydrogen atom removed, including an alkyl group, an alkenyl group and an alkynyl group. According to the number of removed hydrogen atoms, hydrocarbon groups can be classified into monovalent hydrocarbon groups (with one hydrogen atom removed), divalent hydrocarbon groups (with two hydrogen atoms removed; also termed hydrocarbylene groups), trivalent hydrocarbon groups (with three hydrogen atoms removed), and the like. Accordingly, when n hydrogen atoms are lost, the valence of the formed hydrocarbon group is n. Unless otherwise specified, hydrocarbon groups in the present application specifically refer to monovalent hydrocarbon groups. Unless otherwise expressly stated in this specification, a hydrocarbon group is optionally substituted.

In the present application, the source of hydrocarbon group is not particularly limited; for example, hydrocarbon groups can be derived from aliphatic hydrocarbons or aromatic hydrocarbons, from saturated hydrocarbons or unsaturated hydrocarbons, from linear hydrocarbons, branched hydrocarbons or cyclic hydrocarbons, or from hydrocarbons or heterohydrocarbons, etc. According to the degree of saturation, hydrocarbon groups can be derived from alkanes, alkenes, alkynes, dienes, etc. Cyclic hydrocarbons can be derived, e.g., from alicyclic hydrocarbons, aromatic hydrocarbons, monocyclic hydrocarbons, or polycyclic hydrocarbons. Heterocyclic hydrocarbons can be derived, e.g., from aliphatic heterocyclic hydrocarbons or aromatic heterocyclic hydrocarbons.

In the present application, "alkyl group" refers to a hydrocarbon group obtained from an alkane losing a hydrogen atom at any location, unless otherwise specified; the alkyl group can be linear or branched, substituted or unsubstituted. Specific examples include that a propyl group refers to either a 1-propyl group or an isopropyl group, and that a propylene group refers to a 1,3-propylene group, a 1,2-propylene group, or an isopropylidene group. Unless otherwise expressly stated in this specification, alkyl groups are optionally substituted.

In the present application, "unsaturated hydrocarbon group" refers to the hydrocarbon group obtained from an unsaturated hydrocarbon losing hydrogen atoms. The hydrocarbon groups obtained from unsaturated hydrocarbons losing hydrogen atoms bonded to unsaturated carbon atoms, can include alkenyl groups, alkynyl groups, dienyl groups, and the like, and specifically, e.g., propenyl groups and propynyl groups. According to the type of unsaturated bond, the hydrocarbon groups formed by removing hydrogen atoms bonded to saturated carbon atoms of unsaturated hydrocarbons include alkene, alkyne, diene groups, and the like, and specifically, e.g., allyl groups and propargyl groups.

In the present application, "alkenyl group" refers to a substituted or unsubstituted alkenyl group with a linear structure or branched structure, containing two or more carbon atoms (e.g., 2, 3, 4, 5, 6, 7, 8, 9, 10, 11, 12, 13, 14, 15, 16, 17, 18, 19, 20 or more carbon atoms) and at least one carbon-carbon double bond. "C₂₋₁₅ alkenyl group" refers to a substituted or unsubstituted alkenyl group with a linear or branched structure containing 2 to 15 carbon atoms and at least one carbon-carbon double bond, that is, an alkenyl group can contain one, two, three, four, or more carbon-carbon double bonds. Unless otherwise specified, alkenyl groups include substituted and unsubstituted alkenyl groups in the present application. Unless otherwise expressly stated in this specification, alkenyl groups are optionally substituted.

In the present application, "alkynyl group" refers to an optionally substituted hydrocarbon with a linear or branched structure, containing two or more carbon atoms (such as 2, 3, 4, 5, 6, 7, 8, 9, 10, 11, 12, 13, 14, 15, 16, 17, 18, 19, 20, or more carbon atoms) and at least one carbon-carbon triple bond. "C₂₋₁₅ alkynyl group" refers to a substituted or unsubstituted alkynyl group with a linear or branched structure, containing 2 to 15 carbon atoms and at least one carbon-carbon triple bond, that is, an alkynyl group can contain one, two, three, four, or more carbon-carbon triple bonds. Unless otherwise specified, alkynyl groups include substituted and unsubstituted alkynyl groups in the present application. Unless otherwise expressly stated in this specification, alkynyl groups are optionally substituted.

In the present application, "hydrocarbylene group" or "hydrocarbylene chain" refers to a linear or branched divalent hydrocarbon chain that connects the remainder of a molecule to free radicals, consisting only carbon and hydrogen, being saturated or unsaturated. For example, a hydrocarbylene group with one to twenty-four carbon atoms (a C₁₋₂₄ hydrocarbylene group), a hydrocarbylene group with one to twelve carbon atoms (a C₁₋₁₂ hydrocarbylene group), and specifically, a methylene group, an ethylene group, a propylene group, n-butylene group, a vinylidene group, a propenylene group, an n-butenylene group, a propynylene group, an n-butynylene group, etc. Unless otherwise explicitly stated in this specification, hydrocarbylene groups are optionally substituted.

In the present application, "alkylene group" is also a divalent alkyl group, including an open-chain alkylene group and a divalent cycloalkyl group. The open-chain alkylene group refers to a divalent alkyl group without any cyclic structure. The divalent cycloalkyl group refers to a divalent alkyl group containing a cyclic structure. Unless otherwise expressly stated in this specification, alkylene groups are optionally substituted.

In the present application, aliphatic hydrocarbon derivatives are preferably ether-derived aliphatic hydrocarbons, preferably aliphatic hydrocarbon derivatives containing one or two ether bonds, and more preferably aliphatic hydrocarbon derivatives containing two ether bonds.

In the present application, "molecular weight" refers to the mass of a compound. The measuring unit of "molecular weight" is Dalton (Da), unless otherwise specified.

In the present application, the term "about" before a percentage refers to a range of ±0.5%.

In the present application, the terms "stable" and "degradable" are a pair of relative concepts. Detailed examples of stable groups and degradable groups are given in paragraphs [0134]-[0145] in CN113402405A.

In the present application, "hydroxyl protecting group" includes all the groups that can be used as common hydroxyl protecting groups. A hydroxyl protecting group is preferably selected from the group consisting of an alkanoyl group (e.g., an acetyl group, a butyryl group), an aromatic alkanoyl group (e.g., a benzoyl group), a benzyl group, a triphenylmethyl group, a trimethylsilyl group, a *t*-butyldimethylsilyl group, an allyl group, an acetal group, or a ketal group. Removal of acetyl groups is generally carried out under basic conditions, most commonly by the ammonolysis with NH₃/MeOH or by the methanol anion-catalyzed methanolysis. The benzyl group can be easily removed via palladium-catalyzed hydrogenolysis in a neutral solution at room temperature, or via a reduction reaction by metallic sodium in ethanol or liquid ammonia. The triphenylmethyl group is generally removed via catalytic hydrogenolysis. The trimethylsilyl group is generally removed using reagents containing fluoride ions (e.g., tetrabutylammonium fluoride/anhydrous THF, etc.). The t-butyldimethylsilyl ether is relatively stable and can withstand ester hydrolysis conditions with alcoholic potassium hydroxide and mild reduction conditions (e.g., Zn/CH₃OH and the like), which can be removed by fluoride ions (e.g., Bu₄N⁺F⁻) in THF solution or by aqueous acetic acid at room temperature.

In the present application, "carboxyl protecting group" refers to the protecting group which can be transformed into a carboxyl group via the hydrolysis or the deprotection reaction of a carboxyl protecting group itself. A carboxyl protecting group is preferably selected from the group consisting of an alkyl group (e.g., a methyl group, an ethyl group, and a butyl group) and an aralkyl group (e.g., a benzyl group), and more preferably selected from the group consisting of a butyl group (tBu), a methyl group (Me) and an ethyl group (Et). In the present application, "protected carboxyl group" refers to the group protected by an appropriate carboxyl protecting group, preferably selected from the group consisting of a methoxycarbonyl group, an ethoxycarbonyl group, a *t*-butoxycarbonyl group, and a benzyloxycarbonyl group. The carboxyl protecting groups can be removed through hydrolysis catalyzed by acids or bases, or through pyrolysis reactions occasionally; for example, the *t*-butyl groups can be removed under mild acidic conditions, and the benzyl group can be removed by hydrogenolysis. The reagent used for removal of carboxyl protecting groups is selected from the group consisting of TFA, H₂O, LiOH, NaOH, KOH, MeOH, EtOH and combinations thereof, preferably selected from the group consisting of the combination of TFA and H₂O, the combination of LiOH and MeOH, and the combination of LiOH and EtOH. A protected carboxyl group can undergo deprotection and then produce the corresponding free acid; the deprotection is conducted in the presence of a base, and the base forms a pharmaceutically acceptable salt with the free acid produced via the deprotection.

In the present application, "amino protecting group" includes all the groups which are used as amino protecting groups generally, such as an aryl C₁₋₆ alkyl group, a C₁₋₆ alkoxy C₁₋₆ alkyl group, a C₁₋₆ alkoxycarbonyl group, an aryloxycarbonyl group, a C₁₋₆ alkylsulfonyl group, an arylsulfonyl group, a methylsilyl group, etc. An amino protecting group is preferably selected from the group consisting of a t-butoxycarbonyl group (Boc), a p-methoxybenzyloxycarbonyl group (Moz), and a 9-fluorenylmethyloxycarbonyl (Fmoc). The reagent used for removal of amino protecting groups is selected from the group consisting of TFA, H₂O, LiOH, MeOH, EtOH and combinations thereof, preferably selected from the group consisting of the combination of TFA and H₂O, the combination of LiOH and MeOH, and the combination of LiOH and EtOH. The reagent used for removal of the Boc protecting group can be TFA or HCl/EA, preferably TFA. The reagent used for the removal of the Fmoc protecting group can be the N,N-dimethylformamide (DMF) solution containing 20% piperidine.

In the present application, "cation" refers to the corresponding structure bearing a positive charge, either permanently, or non-permanently but in response to certain conditions such as pH. Therefore, the cations include permanent cations and cationizable substances. Permanent cations refer to the corresponding compounds, groups, or atoms that bear positive charges under conditions of any pH value or hydrogen ion activity of their environment; typically, the presence of a quaternary nitrogen atom is accompanied by a positive charge. When a compound carries multiple such positive charges, it can be termed a permanent cation. A cationizable substance refers to a compound, group, or atom that is positively charged at a lower pH and uncharged at a higher pH of its environment. Moreover, in non-aqueous environments where pH cannot be determined, a cationizable compound, group, or atom is positively charged at high hydrogen ion concentration and uncharged at low concentration or activity of hydrogen ions. It depends on the individual properties of the cationizable or polycationizble compound, in particular the pKa of the respective cationizable group or atom, at which pH or hydrogen ion concentration said compound is charged or uncharged. In the diluted aqueous environment, the Henderson-Hasselbalch equation can be used to estimate the fraction of positively charged cationizable compounds, groups, or atoms, which is well-known to those skilled in the art. For example, in some embodiments, if a compound or moiety is cationizable, it is preferred that it is positively charged at a pH value of about 1 to 9, preferably 4 to 9, 5 to 8, or even 6 to 8, more preferably equal to or below 9, equal to or below 8, equal to or below 7, and most preferably at physiological pH values (e.g., about 7.3 to 7.4), i.e., under physiological conditions, especially under normal saline conditions in cells in vivo. In other embodiments, it is preferable that the cationizable compound or moiety is predominantly neutral at physiological pH values, e.g., about 7.0 to 7.4, but becomes positively charged at lower pH values. In some embodiments, pKa of the cationizable compound or moiety is preferably about 5 to about 7.

In the present application, "cationic lipid" refers to a lipid having a positive charge or being ionizable. In addition to the structural general formula (1) of the present application, cationic lipids include, but are not limited to, N,N-dioleyl-N,N-dimethylammonium chloride (DODAC), *N,N*-distearyl-*N,N*-dimethylammonium bromide (DDAB), N-(1-(2,3-dioleoyloxy)propyl)-N,N,N-trimethylammonium chloride (DOTAP), N-(1-(2,3-dioleyloxy)propyl)-N,N,N-trimethylammonium chloride (DOTMA), N,N-dimethyl-2, 3-Dioilyloxypropylamine (DODMA), 3-(didodecylamino)-*N1,N1*,4-tridodecyl-1-piperazineethanamine (KL10), *N*-[2(didodecylamino)ethyl]*-N1,N4,N4*-tridodecyl-1,4-piperazinediethanamine (KL22), 14,25-ditridecyl-15,18,21,24-tetraaza-octatriacontane (KL25), 1,2-Dileutheroxy-N, N-Dimethylaminopropane (DLin-DMA), 2,2-Dilinoleyl-4-dimethylaminomethyl-[1,3 ]-dioxolane (DLin-K-DMA), heptatriaconta-6,9,28,31-tetraen-19-yl 4-(dimethylamino)butanoate (DLin-MC3-DMA) and 2,2-dilinoleyl-4-(2dimethylaminoethyl)-[1,3]-dioxolane (DLin-KC2-DMA), ((4-hydroxybutyl)azanediyl)bis(hexane-6,1-diyl)bis(2-hexyldecanoate) (ALC-0315), heptadecan-9-yl 8-((2-hydroxyethyl) (6-oxo-6-(undecyloxy) hexyl) amino) octanoate) (SM102) and any mixture thereof.

In the present application, "PEGylated lipid" refers to the molecules containing both lipid and PEG moieties.

In the present application, "neutral lipid" refers to any lipid substance that is uncharged or exists in the form of neutral zwitterion at the chosen pH, preferably phospholipid. The neutral lipid can be synthetic or natural.

In the present application, "steroid lipid" refers to a steroid or steroid analog.

In the present application, "variant form" refers to a structure that can be transformed into the target reactive group after any process of chemical change selected from the group consisting of oxidation, reduction, hydration, dehydration, electronic rearrangement, structural rearrangement, salt complexation and decomplexation, ionization, protonation, deprotonation, substitution, deprotection, leaving group transformation, etc.

In the present application, "variant form of reactive group" refers to a form which is still active (still a reactive group) after at least one process of chemical change such as oxidation, reduction, hydration, dehydration, electronic rearrangement, structural rearrangement, salt complexation and decomplexation, ionization, protonation, deprotonation, substitution, deprotection, leaving group transformation, etc., or an inactive form after being protected.

In the present application, "N/P ratio" refers to a molar ratio of cationizable nitrogen atoms in cationic lipids to phosphates in nucleic acids.

In the present application, "nucleic acid" refers to DNA, RNA, or their modified form.

In the present application, "RNA" refers to a ribonucleic acid that may be naturally or non-naturally occurring. For example, RNA may include modified and/or non-naturally occurring components such as one or more nucleobases, nucleosides, nucleotides, or linkers. RNA may include a cap structure, a chain-terminating nucleoside, a stem-loop, a polyA sequence, and/or a polyadenylation signal. RNA may have a nucleotide sequence encoding a polypeptide of interest. For example, RNA may be a messenger RNA (mRNA). Translation of mRNA encoding a particular polypeptide, for example, in vivo translation of mRNA inside a mammalian cell, may produce the encoded polypeptide. RNAs may be selected from the non-limiting group consisting of small interfering RNA (siRNA), asymmetrical interfering RNA (aiRNA), microRNA (miRNA), Dicer-substrate RNA (dsRNA), small hairpin RNA (shRNA), mRNA (messenger RNA), single guide RNA (sgRNA), self-amplifying RNA (saran), circular RNA, cas9 mRNA, and mixtures thereof.

In the present application, FLuc mRNA can express luciferase protein which emits bioluminescence in the presence of fluorescein substrate, so FLuc is commonly used in mammalian cell culture to measure gene expression and cell viability.

In the present application, suitable assays for determining the level of target gene expression include but are not limited to dot blot, northern blot, in situ hybridization, ELISA, immunoprecipitation, enzyme function, and phenotypic assays.

In the present application, "transfection" refers to the introduction of a species (e.g., RNA) into a cell. Transfection may occur, for example, in vitro, ex vivo, or in vivo.

In the present application, "antigen" typically refers to a substance that can be recognized by the immune system, preferably recognized by the adaptive immune system, and trigger an antigen-specific immune response, for example, forming antibodies and/or antigen-specific T cells as a part of the adaptive immune response. Typically, the antigen may be or may contain a peptide or a protein that can be presented to T cells by MHC. In the present application, the antigen may be a translation product of the provided nucleic acid molecule (preferably mRNA as defined herein). In this context, fragments, variants, and derivatives of peptides and proteins containing at least one epitope are also defined as antigens.

In the present application, "delivery" refers to delivering an entity to the target, for example, delivering drugs and/or therapeutic agents and/or prophylactic agents to subjects, wherein the subjects are tissues and/or cells of humans and/or other animals.

In the present application, "pharmaceutically acceptable carrier" refers to a diluent, adjuvant, excipient, or vehicle administered together with the therapeutic agent, which is, within the scope of reasonable medical judgment, suitable for contact with tissues of human and/or other animals without causing excessive toxicity, irritation, allergic reaction, or other problems or complications corresponding to reasonable benefit/risk ratio. Pharmaceutically acceptable carriers that can be used in the pharmaceutical composition in the present application include but are not limited to sterile liquids, such as water and oil, including oils from petroleum, animal, vegetable, or synthesis, e.g., peanut oil, soybean oil, mineral oil, sesame oil, etc. When the pharmaceutical composition is administered intravenously, water is an exemplary carrier. Physiological saline, glucose, and aqueous glycerol solution can also be used as liquid carriers, especially as an injection. Suitable pharmaceutical excipients include starch, glucose, lactose, sucrose, gelatin, maltose, chalk, silica gel, sodium stearate, glyceryl monostearate, talc, sodium chloride, defatted milk powder, glycerol, propylene glycol, water, ethanol, etc. The composition can also contain a small amount of humectant, emulsifier, or pH buffer as needed. Oral preparations can contain standard carriers, such as pharmaceutical-grade mannitol, lactose, starch, magnesium stearate, sodium saccharin, cellulose, magnesium carbonate, etc. Specifically, excipients include but are not limited to anti-adherents, antioxidants, binders, coatings, compression aids, disintegrants, dyes (pigments), emollients, emulsifiers, fillers (diluents), film formers or coatings, flavors, fragrances, glidants (flow enhancers), lubricants, preservatives, printing inks, sorbents, suspending or dispersing agents, sweeteners, and water for hydration. More specifically, excipients include but are not limited to butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E (α-tocopherol), vitamin C, and xylitol.

In the present application, pharmaceutical compositions can act systematically and/or locally. For this purpose, they can be administered by appropriate routes such as injection (e.g., intravenous, intraarterial, subcutaneous, intraperitoneal, or intramuscular injection, including instillation) and transdermal delivery, and can also be administered by oral, buccal, transnasal, transmucosal, or topical routes, or in the form of ophthalmic preparation, or by inhalation. Regarding these routes of administration, the pharmaceutical compositions of the present application can be administered in suitable dosage forms. The dosage forms include but are not limited to, tablets, capsules, lozenges, hard sugar agents, powders, sprays, creams, ointments, suppositories, gels, pastes, lotions, ointments, aqueous suspensions, injectable solutions, elixirs, and syrups.

In the present application, "vaccines" are preventive or therapeutic materials that provide at least one antigen or antigenic function. An antigen or antigenic function can stimulate the body's adaptive immune system to provide an adaptive immune response.

In the present application, "treatment" refers to the management and care of patients to resist diseases, obstacles, or symptoms, which is intended to delay the development of diseases, obstacles, or symptoms, reduce or alleviate symptoms and complications, and/or cure or eliminate diseases, obstacles, or symptoms. The patients to be treated are preferably mammals, especially humans.

### An embodiment of the present application:

**1. A cationic lipid, wherein the structure is represented by the general formula (1):** 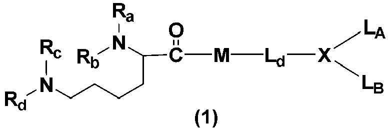
   wherein, Rₐ, R_{b}, R_{c} and R_{d} are each independently a C₁₋₃ alkyl group; or, Rₐ and R_{b}, together with the nitrogen atom to which they are connected, form a nitrogen-containing heterocyclic group; or, R_{c} and R_{d}, together with the nitrogen atom to which they are connected, form a nitrogen-containing heterocyclic group;
   M is O, S, or -NH-;
   L_{d} is a linking bond or a divalent linking group;
   X is N or CRₘ, and the Rₘ is H or a C₁₋₁₂ alkyl group;
   L_{A} is represented by -B₃-L₃-B₁-L₁-R₁, and L_{B} is represented by -B₄-L₄-B₂-L₂-R₂; wherein, L₁, L₂, L₃, and L₄ are each independently a linking bond or a divalent linking group L; B₁, B₂, B₃, and B₄ are each independently a linking bond or a C₁₋₂₀ alkylene group; R₁ and R₂ are each independently a C₁₋₃₀ hydrocarbon group or a C₁₋₃₀ hydrocarbon derivative residue containing 1 to 4 units of -O-;
   or a pharmaceutically acceptable salt, tautomer, stereoisomer, deuterated derivative, or solvate thereof.
**1.1. M**
   In the present application, M is O, S, or -NH-.
**1.2. X**
   In the present application, X is N or CRₘ; wherein, the Rₘ is H or a C₁₋₁₂ alkyl group.
**1.3. L_{d}**

In the present application, L_{d} is a linking bond or a divalent linking group.

In a specific embodiment of the present application, L_{d} is selected from the group consisting of a linking bond, -(CH₂)ₜ-, -(CH₂)ₜZ-, -(CH₂)ₜZ-(CH₂)ₜZ-, and -(CH₂)ₜZ(CH₂)ₜZ-; wherein, t is independently an integer from 1 to 12 at each occurrence; Z is independently selected from the group consisting of -C(=O)-, -OC(=O)-, -C(=O)O-, -OC(=O)O-, -O-, -S-, -C(=O)S-, -SC(=O)-, -NRC(=O)-, -C(=O)NR_{g}, -NR_{g}C(=O)NR_{g}-, -OC(=O)NR_{g}-, -NR_{g}C(=O)O-, -SC(=O)NR_{g}-, and -NR_{g}C(=O)S- at each occurrence; wherein, R_{g} is independently H or a C₁₋₁₂ alkyl group at each occurrence; L_{g} is preferably selected from the group consisting of a linking bond, -(CH₂)ₜ-, -(CH₂)ₜO-, -(CH₂)ₜC(=O)-, -(CH₂)ₜC(=O)O-, -(CH₂)ₜOC(=O)-, -(CH₂)ₜC(=O)NH-, -(CH₂)ₜNHC(=O)-, -(CH₂)ₜOC(=O)O-, -(CH₂)ₜNHC(=O)O-, -(CH₂)ₜOC(=O)NH-, -(CH₂)ₜNHC(=O)NH-, -(CH₂)ₜO(CH₂)ₜ-, -(CH₂)ₜC(=O)(CH₂)ₜ-, -(CH₂)ₜC(=O)O(CH₂)ₜ-, -(CH₂)ₜOC(=O)(CH₂)ₜ-, -(CH₂)ₜC(=O)NH(CH₂)ₜ-, -(CH₂)ₜNHC(=O)(CH₂)ₜ-, -(CH₂)ₜOC(=O)O(CH₂)ₜ-, -(CH₂)ₜNHC(=O)O(CH₂)ₜ-, -(CH₂)ₜOC(=O)NH(CH₂)ₜ-, and -(CH₂)ₜNHC(=O)NH(CH₂)ₜ-; most preferably, L_{d} is a linking bond or -(CH₂)ₜ-.

### 1.4. L₁, L₂, L₃, L₄

In the present application, L₁, L₂, L₃, and L₄ are each independently a linking bond or a divalent linking group.

In a specific embodiment of the present application, the L₁, L₂, L₃, and L₄ are each independently a linking bond or a divalent linking group L. The L is independently selected from the group consisting of -OC(=O)-, -C(=O)O-, -OC(=O)O-, -C(=O)-, -O-, -NH-, -O(CR_{g}R_{g})ₛO-, -S-, -C(=O)S-, -SC(=O)-, -NR_{g}C(=O)-, -C(=O)NR_{g}-, -NR_{g}C(=O)NR_{g}-, -OC(=O)NR_{g}-, -NR_{g}C(=O)O-, -SC(=O)NR_{g}-, -NR_{g}C(=O)S-, -C(=S)-, -OC(=S)-, -C(=S)O-, -OC(=S)O-, -NR_{g}C(=S)-, -C(=S)NR_{g}-, -NR_{g}C(=S)NR_{g}-, -OC(=S)NR_{g}-, and -NR_{g}C(=S)O- at each occurrence; wherein, R_{g} is independently a hydrogen atom or a C₁₋₁₂ alkyl group at each occurrence, and s is 2, 3 or 4; preferably, L₁, L₂, L₃, and L₄ are selected from any of the following cases:
Case (1): L₁, L₂, L₃, and L₄ are each independently L;
Case (2): any one of L₁, L₂, L₃, and L₄ is a linking bond, and the other three are each independently L; preferably, L₃ is a linking bond, and L₁, L₂, and L₄ are each independently L;
Case (3): any two of L₁, L₂, L₃, and L₄ are linking bonds, and the other two are each independently L; preferably, L₃ and L₄ are linking bonds, and L₁ and L₂ are each independently L;
Case (4): any three of L₁, L₂, L₃, and L₄ are linking bonds, and the other is L;
Case (5): L₁, L₂, L₃, and L₄ are all linking bonds.

In a specific embodiment of the present application, L₁, L₂, L₃, and L₄ are more preferably selected from any one of the aforementioned case (1) to case (3).

In a specific embodiment of the present application, L₁, L₂, L₃, and L₄ are most preferably selected from any of the following cases:
Case (a): L₁, L₂, L₃, and L₄ are each independently selected from the group consisting of -OC(=O)-, -C(=O)O-, -C(=O)NH-, -OC(=O)O-, and -NHC(=O)O-;
Case (b): L₃ is a linking bond, and L₁, L₂, and L₄ are each independently selected from the group consisting of -OC(=O)-, -C(=O)O-, -C(=O)NH-, -OC(=O)O-, and -NHC(=O)O-;
Case (c): L₃ and L₄ are linking bonds; L₁ and L₂ are each independently selected from the group consisting of -OC(=O)-, -C(=O)O-, -C(=O)NH-, -OC(=O)O-, and -NHC(=O)O-.

### 1.5. B₁, B₂, B₃, B₄

In the present application, B₁, B₂, B₃, and B₄ are each independently a linking bond or a C₁₋₂₀ alkylene group.

In a specific embodiment of the present application, B₁, B₂, B₃, and B₄ are selected from any of the following cases:
Case (1): B₁, B₂, B₃, and B₄ are each independently a C₁₋₂₀ alkylene group, more preferably a C₁₋₁₀ alkylene group;
Case (2): any one of B₁, B₂, B₃, and B₄ is a linking bond, and the other three are C₁₋₁₀ alkylene groups; preferably, B₃ is a linking bond, with B₁, B₂, and B₄ each independently being a C₁₋₁₀ alkylene group;
Case (3): any two of B₁, B₂, B₃, and B₄ are linking bonds, and the other two are C₁₋₁₀ alkylene groups; preferably, B₃ and B₄ are linking bonds, with B₁ and B₂ each independently being a C₁₋₁₀ alkylene group;
Case (4): any three of B₁, B₂, B₃, and B₄ are linking bonds, and the other is a C₁₋₁₀ alkylene group;
Case (5): B₁, B₂, B₃, and B₄ are all linking bonds.

In a specific embodiment of the present application, B₁, B₂, B₃, and B₄ are more preferably selected from any one of the aforementioned case (1) to case (3); wherein, the aforementioned C₁₋₁₀ alkylene group is preferably selected from the group consisting of a methylene group, an ethylene group, a propylene group, a butylene group, a pentylene group, a hexylene group, a heptylene group, and an octylene group.

### 1.6. R₁, R₂

In the present application, R₁ and R₂ are each independently a C₁₋₃₀ hydrocarbon group or a C₁₋₃₀ hydrocarbon derivative residue containing 1 to 4 -O-.

In a specific embodiment of the present application, R₁ and R₂ are preferably selected from any of the following cases:
Case (1): both R₁ and R₂ are C₁₋₃₀ hydrocarbon groups;
Case (2): both R₁ and R₂ are C₁₋₃₀ hydrocarbon derivative residues;
Case (3): one of R₁ and R₂ is a C₁₋₃₀ hydrocarbon group, and the other is a C₁₋₃₀ hydrocarbon derivative residue;
the C₁₋₃₀ hydrocarbon derivative residue is represented by 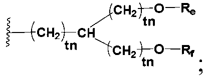 wherein, the tn is independently an integer from 0 to 12 at each occurrence; Rₑ and R_{f} are each independently selected from the group consisting of a C₁₋₁₅ alkyl group, a C₂₋₁₅ alkenyl group, and a C₂₋₁₅ alkynyl group.

In a specific embodiment of the present application, the aforementioned C₁₋₃₀ hydrocarbon group is preferably selected from the group consisting of a linear C₁₋₃₀ hydrocarbon group or a branched C₁₋₃₀ hydrocarbon group.

In a specific embodiment of the present application, the linear C₁₋₃₀ hydrocarbon group is selected from the group consisting of a linear C₁₋₃₀ alkyl group, a linear C₂₋₃₀ alkenyl group, and a linear C₂₋₃₀ alkynyl group; preferably, the linear C₁₋₃₀ hydrocarbon group is selected from the group consisting of a linear C₁₋₂₅ alkyl group, a linear C₂₋₂₅ alkenyl group, and a linear C₂₋₂₅ alkynyl group; more preferably, the linear C₁₋₃₀ hydrocarbon group is a linear C₂₋₂₅ alkenyl group.

In a specific embodiment of the present application, the branched C₁₋₃₀ hydrocarbon group is selected from the group consisting of a branched C₁₋₃₀ alkyl group, a branched C₂₋₃₀ alkenyl group, and a branched C₂₋₃₀ alkynyl group, each independently represented by 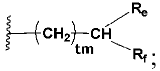 wherein, tm is an integer from 0 to 12; Rₑ and R_{f} are each independently selected from the group consisting of a C₁₋₁₅ alkyl group, a C₂₋₁₅ alkenyl group, and a C₂₋₁₅ alkynyl group; the branched C₁₋₃₀ hydrocarbon group is preferably selected from the group consisting of the following structures:

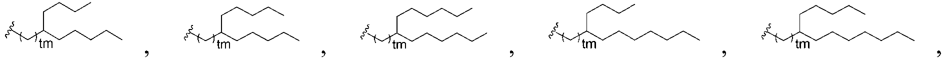

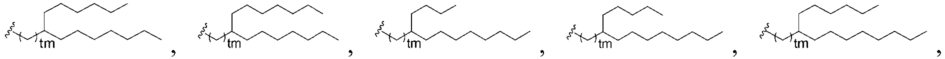

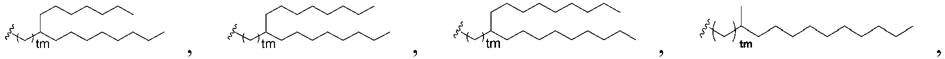

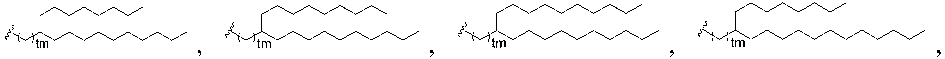

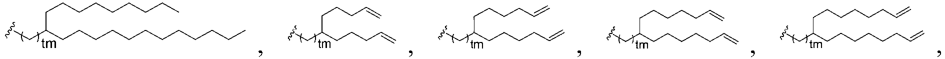

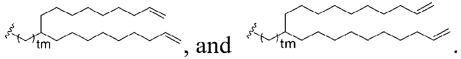

In a specific embodiment of the present application, the 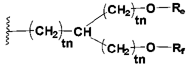 is preferably selected from the group consisting of 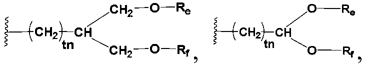 and 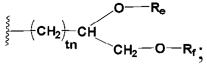 further, the 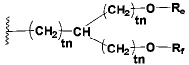 is preferably selected from the group consisting of the following structures: 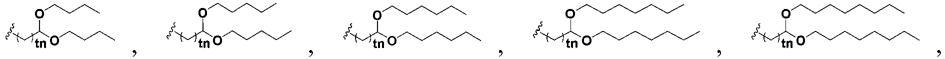 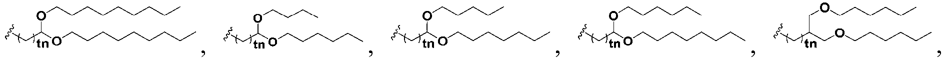 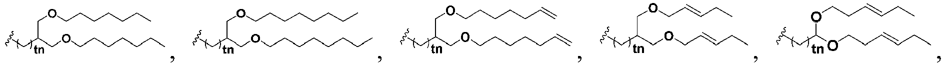 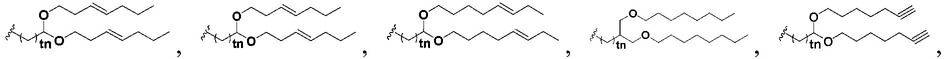 and

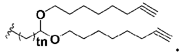

### 1.7. L_{A}, L_{B}

In a specific embodiment of the present application, L_{A} is represented by -B₃-L₃-B₁-L₁-R₁, and L_{A} is represented by -B₄-L₄-B₂-L₂-R₂; L_{A} and L_{B} are each independently selected from the group consisting of the following structures: 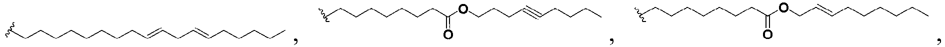 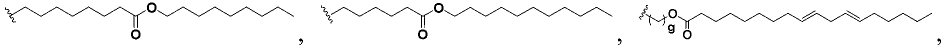 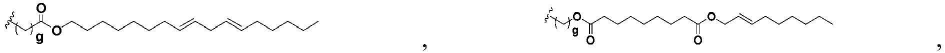 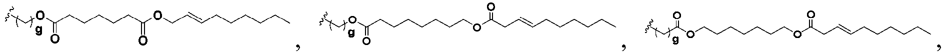 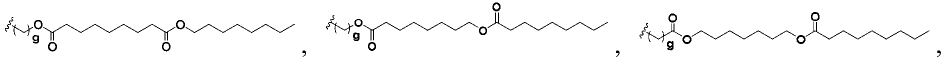 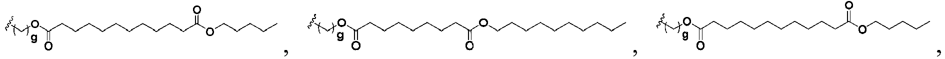 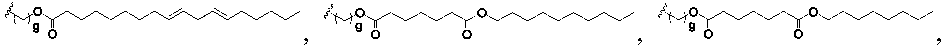 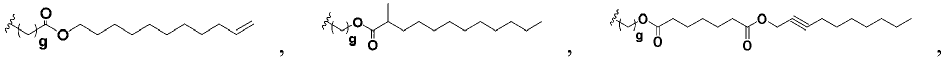 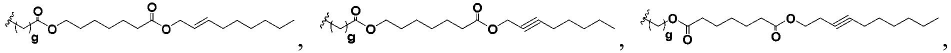 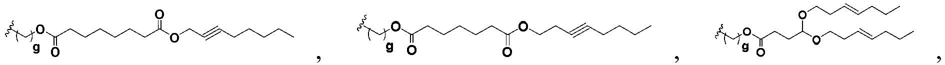 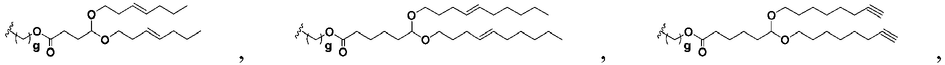 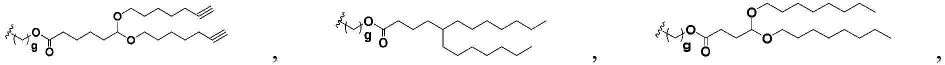 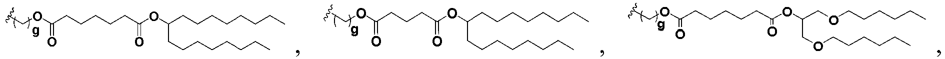 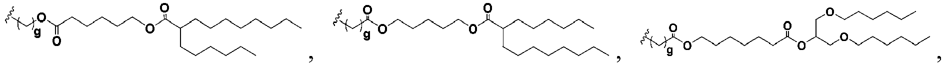 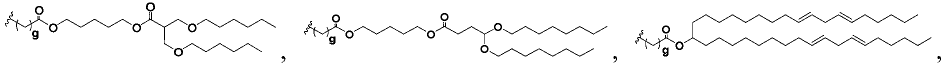 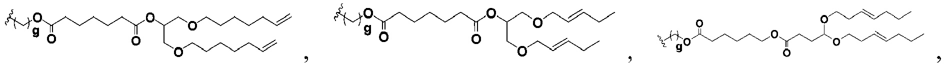 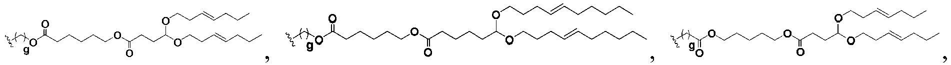 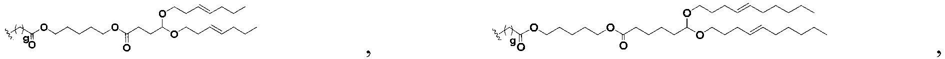 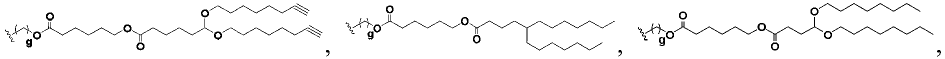 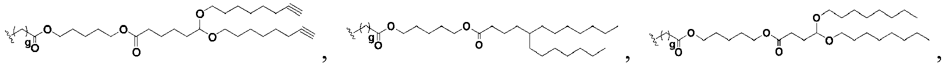 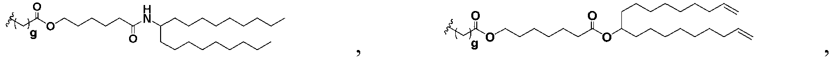 and 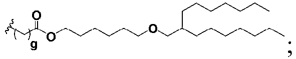 wherein, g is independently an integer from 1 to 10 at each occurrence, more preferably 1, 2, or 3.

### 1.8. Rₐ, R_{b}, R_{c}, R_{d}

In a specific embodiment of the present application, Rₐ and R_{b} are identical and are both methyl groups or ethyl groups; R_{c} and R_{d} are identical and are both methyl groups or ethyl groups.

In a specific embodiment of the present application, Rₐ and R_{b}, together with the nitrogen atom to which they are connected, form a nitrogen-containing heterocyclic group; R_{c} and R_{d}, together with the nitrogen atom to which they are connected, form a nitrogen-containing heterocyclic group; the nitrogen-containing heterocyclic group is

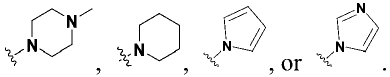

In a specific embodiment of the present application, it is preferred that Rₐ, R_{b}, R_{c}, and R_{d} are all methyl groups.

### 1.9. Examples of general formulas of structures

In a specific embodiment of the present application, the structure of cationic lipid containing two tertiary amine groups is represented by the following general formula (1-A) or (1-B): 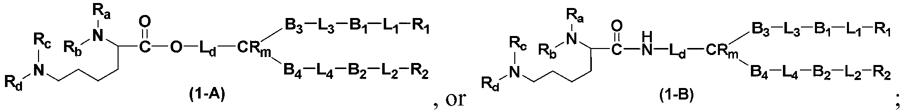 preferably, the structure of cationic lipid is represented by general formula (2-1) or (2-2): 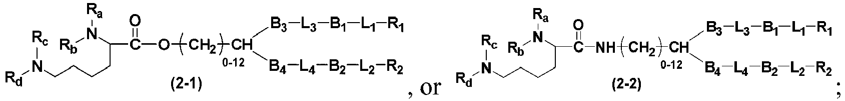 more preferably, the structure of cationic lipid is selected from the group consisting of the following general formulas: 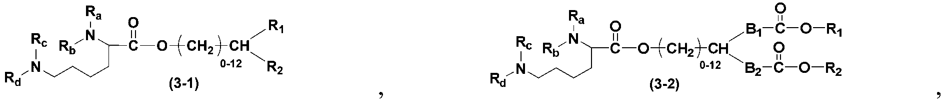 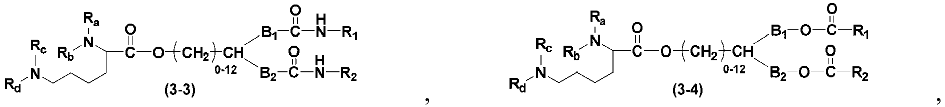 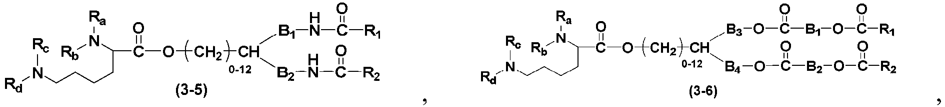 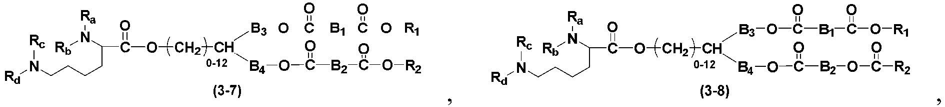 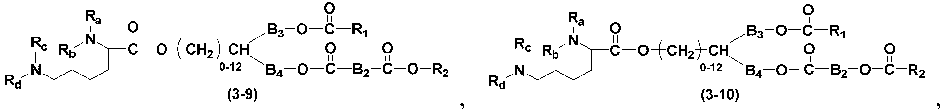 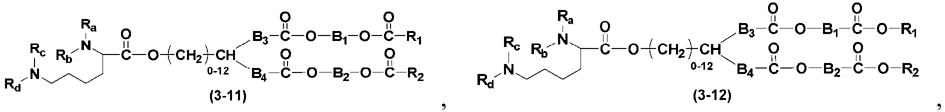 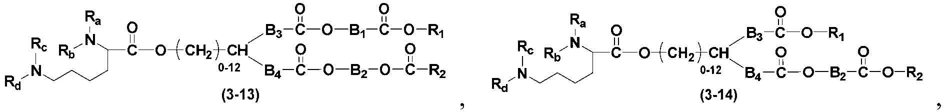 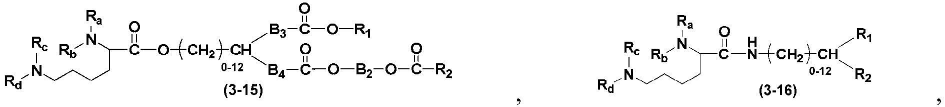 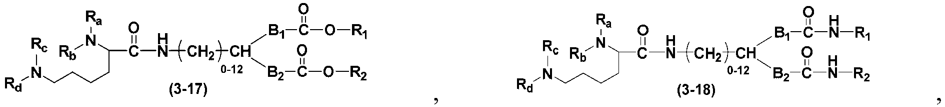 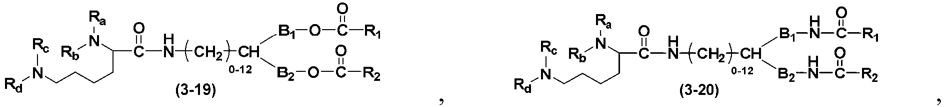 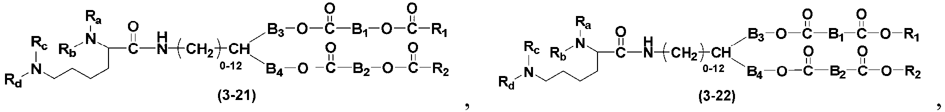 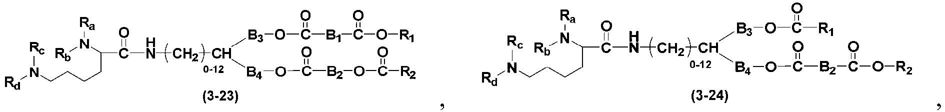 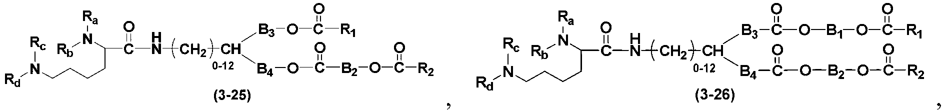 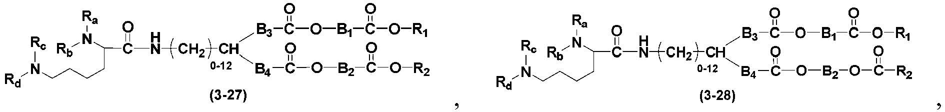 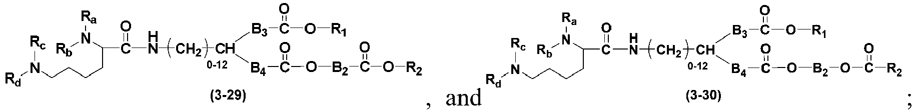 in the formulas (3-1) to (3-30), B₁, B₂, B₃, and B₄ are not linking bonds; more preferably, B₁, B₂, B₃, and B₄ are each independently a C₁₋₁₀ alkylene group at each occurrence.

### 1.10. Examples of specific structures

In a specific embodiment of the present application, the cationic lipid is selected from the group consisting of the following structures: 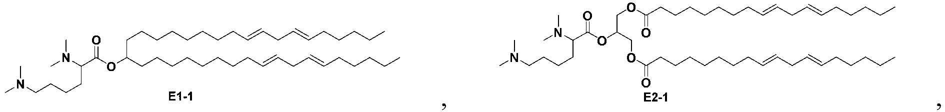 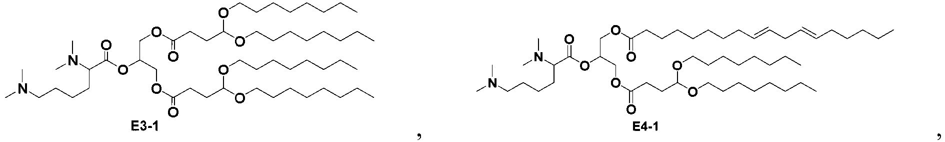 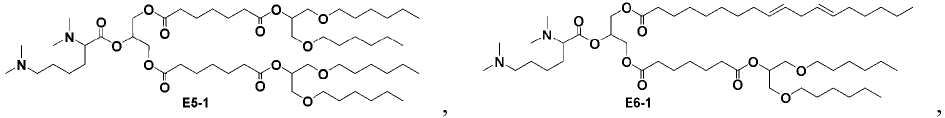 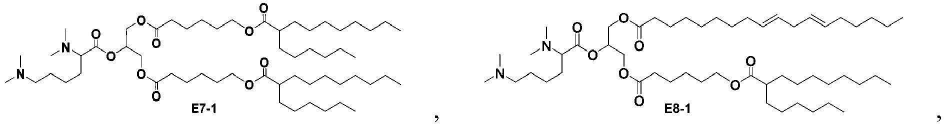 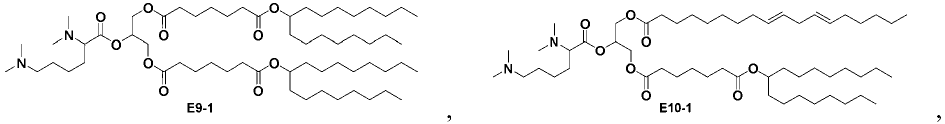 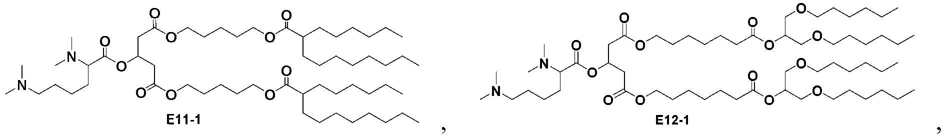 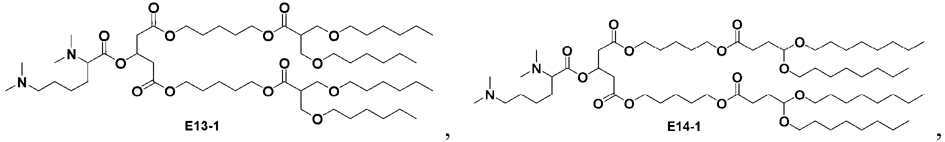 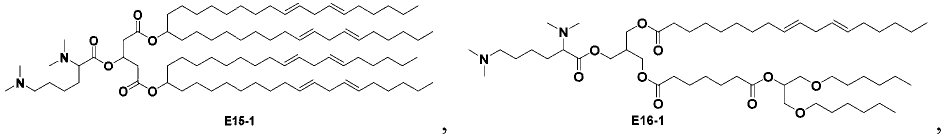 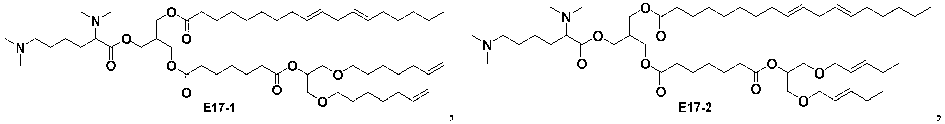 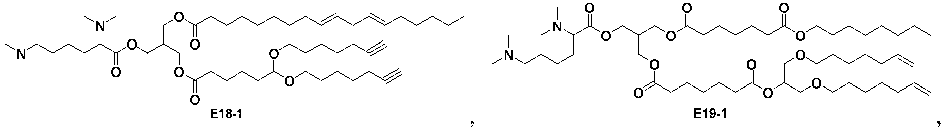 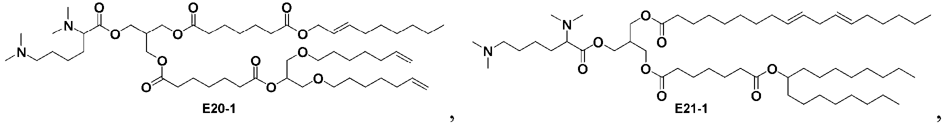 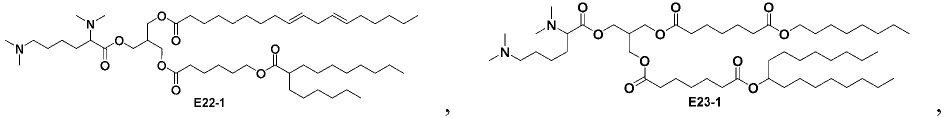 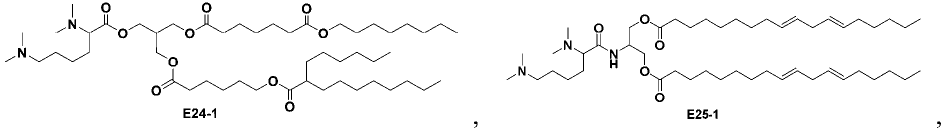 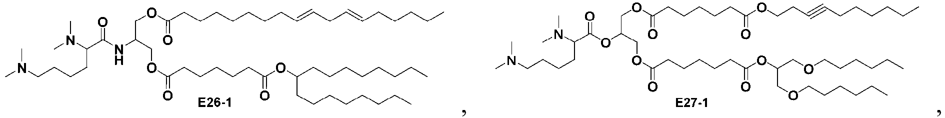 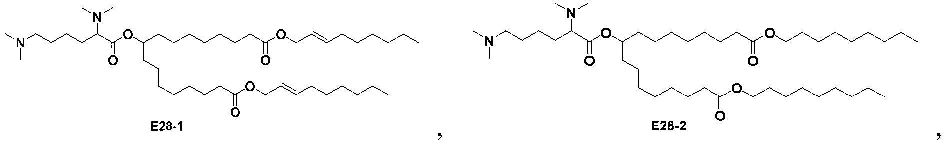 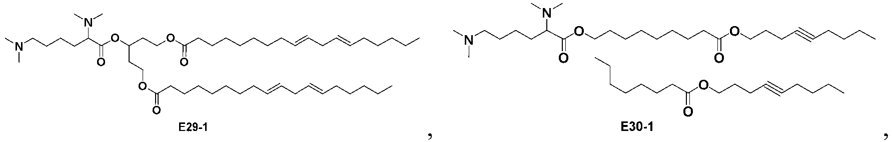 and 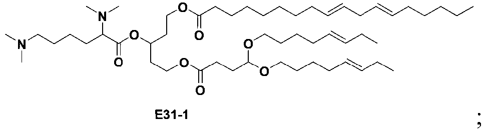 or the cationic lipid is selected from the group consisting of the following structures:

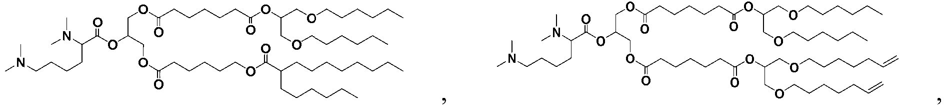

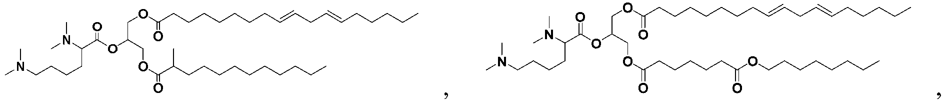

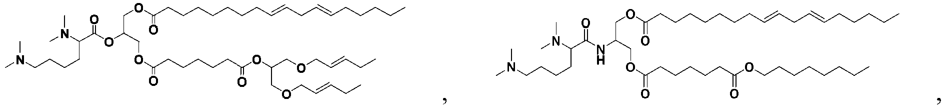

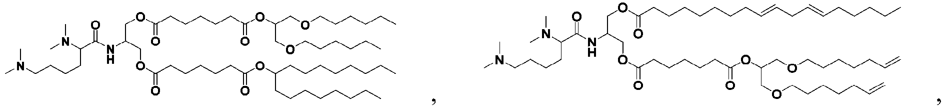

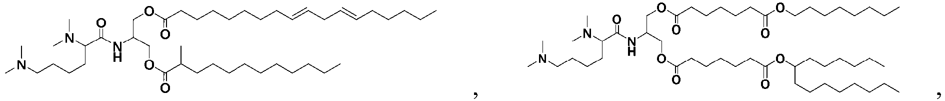

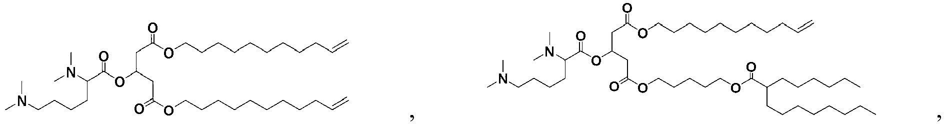

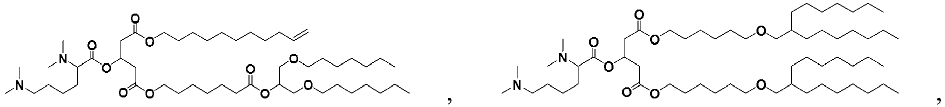

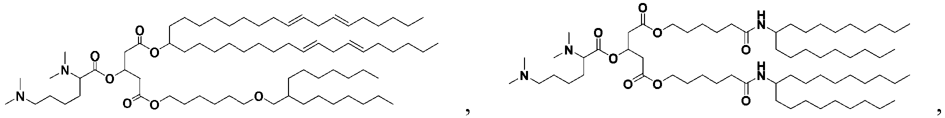

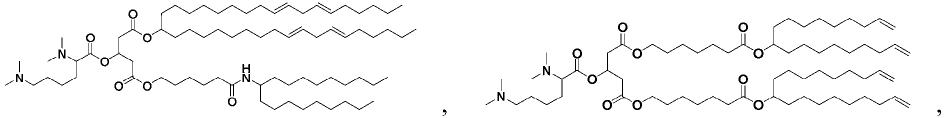

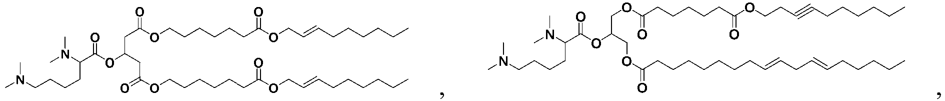

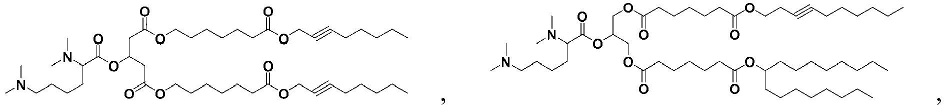

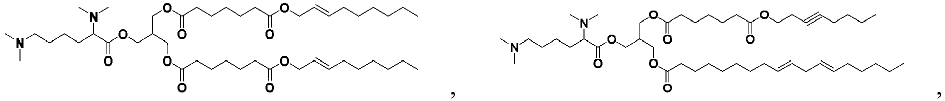

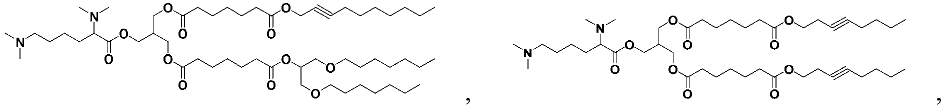

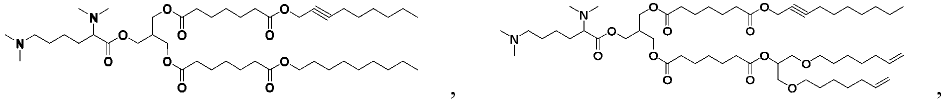

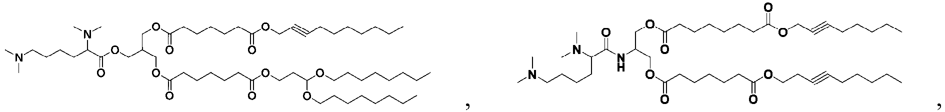

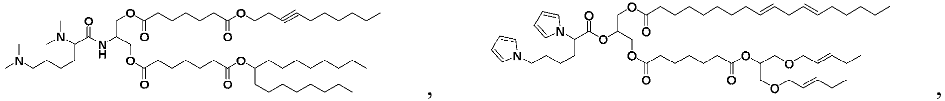

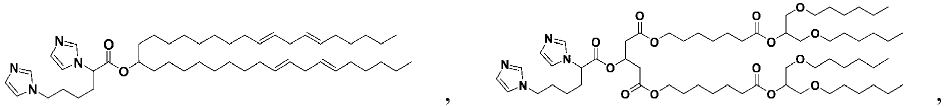

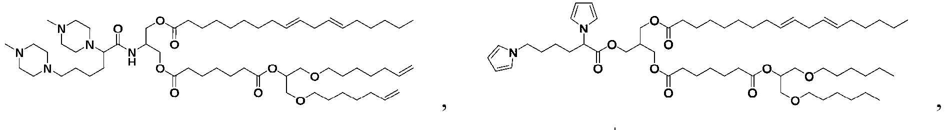

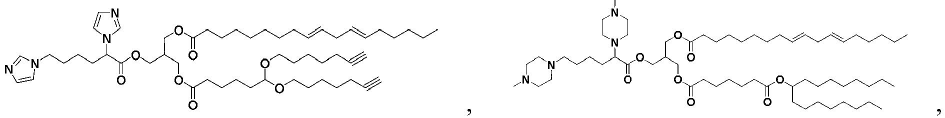

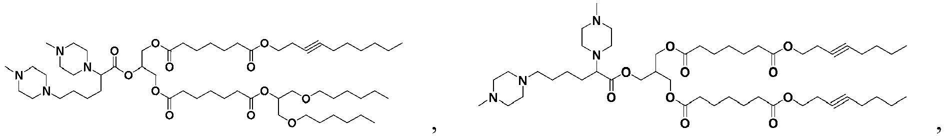

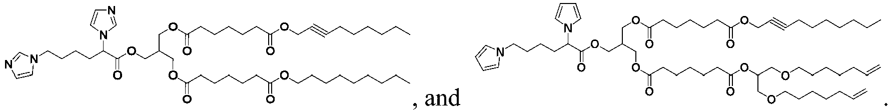

### 2. Preparation of cationic lipids

**2.1.** In a specific embodiment of the present application, the cationic lipid represented by formula (1) is prepared by the following method:
The intermediates/starting materials involved in the preparation process of cationic lipids of the present application include but are not limited to LYS₀, LYS-C, IM-C', IM-C and IM-N.

The preparation process of cationic lipid of the present application involves an initial starting material LYS₀ containing a lysine core with one reactive group F₀; wherein, F₀ is the protected or unprotected -COOH.

In the present application, LYS₀ is selected from the group consisting of the following structures: 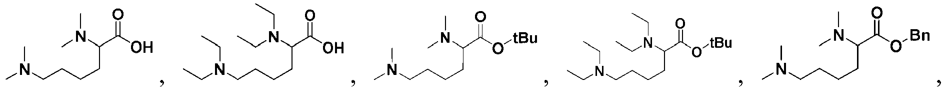 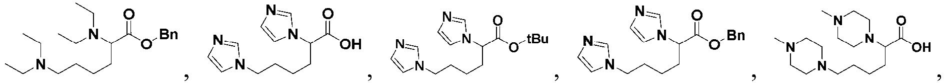 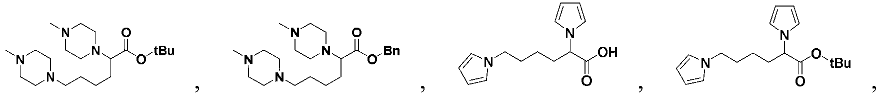 and

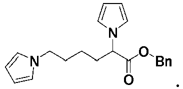

The preparation process of cationic lipids of the present application may involve a small molecule intermediate IM-C' containing three identical or different reactive groups, and the structure of IM-C' is represented by 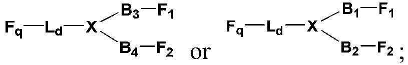 wherein, F_{q} is a protected or unprotected group that can react with F₀ in LYS₀, and is selected from the group consisting of -OH, -NH₂, and -SH; the functional group F₁ and F₂ are selected from the group consisting of -OH, -NH₂, and -COOH. The definitions of the remaining symbols are the same as those described in general formula (1). IM-C' can be obtained by purchase or synthesis.

In the present application, IM-C' is selected from the group consisting of the following structures:

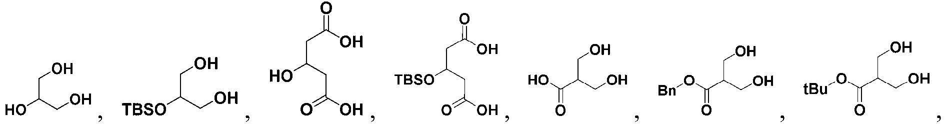

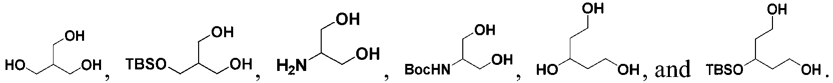

The preparation process of cationic lipids of the present application may involve a small molecule intermediate IM-N containing a reactive group and an aliphatic tail chain, and the structure is represented by F₃-B₁-L₁-R₁ or F₄-B₂-L₂-R₂; wherein, F₃ and F₄ are reactive groups that can react with F₁ and F₂ in the aforementioned IM-C', preferably -OH, -COOH, -NH₂, -COCl, -Cl or -Br. The definitions of the remaining symbols are the same as those described in general formula (1). IM-N can be obtained by purchase or synthesis, which is preferably obtained by simple single-step or step-by-step reactions such as esterification, amidation, alkylation, addition or substitution. For example, in Example 5, the small molecule intermediate IM-N 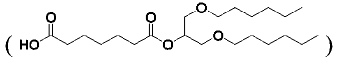 was obtained via the condensation reaction of S5-1 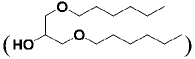 and S5-2 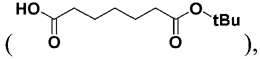 followed by removing the tBu protecting group.

In the present application, the structure of IM-N is selected from the group consisting of F₃-B₁-L₁-R₁, F₃-B₁-R₁, F₃-R₁, F₄-B₂-L₂-R₂, F₄-B₂-R₂, and F₄-R₂; wherein, L₁, L₂, B₃, and B₄ are not linking bonds; specifically, IM-N is selected from the group consisting of the following structures: 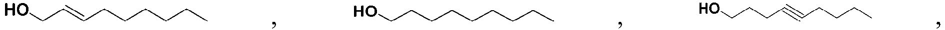 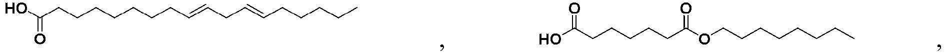 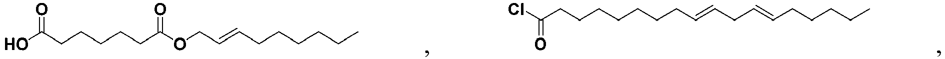 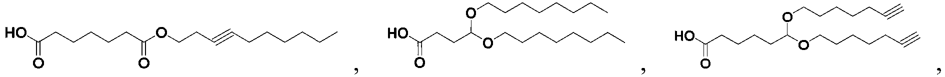 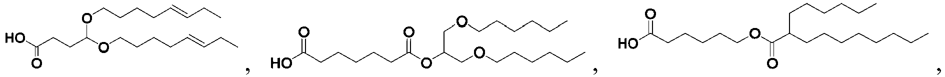 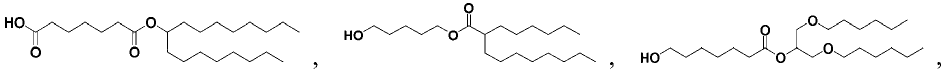 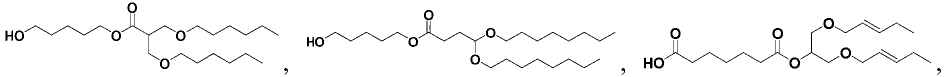 and

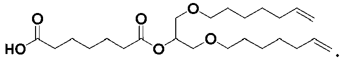

The preparation process of cationic lipids of the present application may involve an intermediate LYS-C containing a lysine core and two reactive groups with a structure represented by 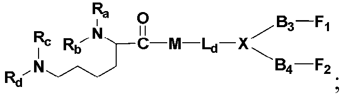 wherein, the functional groups F₁ and F₂ are selected from the group consisting of -OH, -NH₂, and -COOH. The definitions of the remaining symbols are the same as those described in general formula (1). LYS-C can be obtained by the single-step or step-by-step reaction between LYS₀ and IM-C'. For example, in the method 2 of Example 25, the LYS-C intermediate 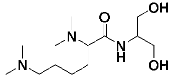 can be obtained by the condensation reaction between S25-5 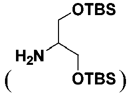 and S1-3 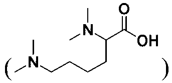 and then removing the TBS protecting group.

In the present application, LYS-C is selected from the group consisting of the following structures:

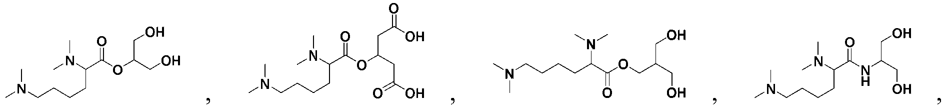

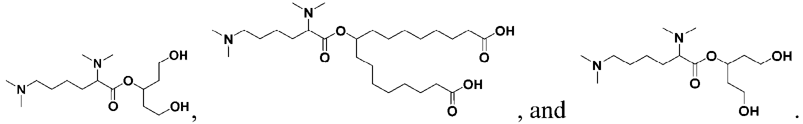

The preparation process of cationic lipids of the present application may involve an intermediate IM-C containing one reactive group and two aliphatic tail chains, and its structure can be represented by 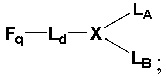 wherein, F_{q} is a protected or unprotected group that can react with F₀ in LYS₀, and it is selected from the group consisting of -OH, -NH₂, and -SH. The definitions of the remaining symbols are the same as those described in general formula (1). IM-C can be obtained by purchase or synthesis. Specifically, IM-C can be obtained by a single-step or step-by-step reaction between IM-N and IM-C'; for example, in Example 2, the IM-C intermediate 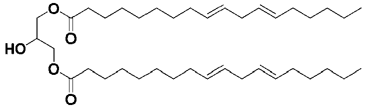 can be obtained by the condensation reaction between S2-1 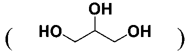 and S2-2 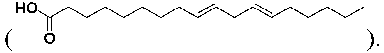 The IM-C intermediate can also be obtained by the addition reaction of the aforementioned intermediate IM-N under the action of a Grignard reagent; for example, in Example 1, S1-2 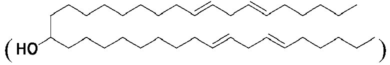 can be obtained by the reaction of S1-1 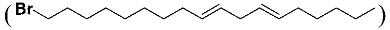 under the action of metallic magnesium.

In the present application, IM-C is selected from the group consisting of the following structures: 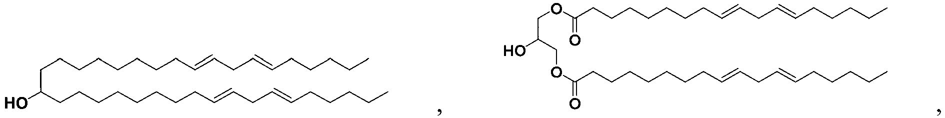 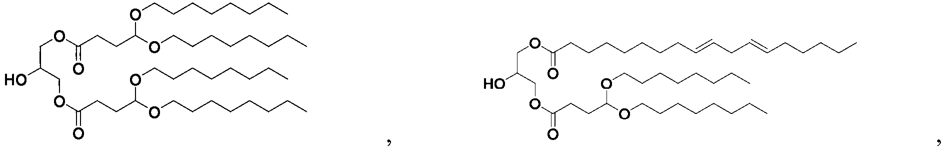 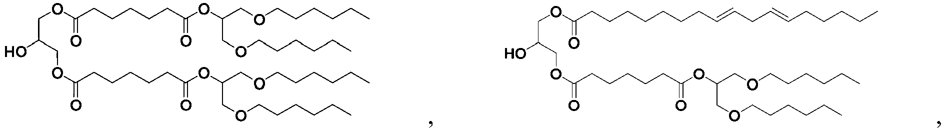 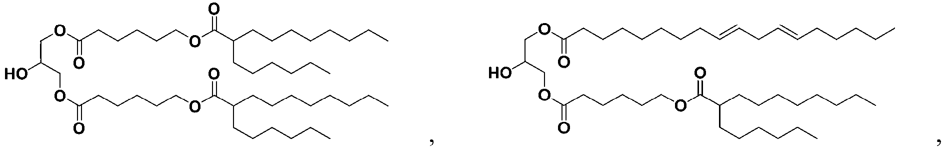 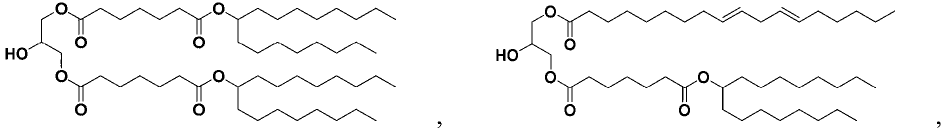 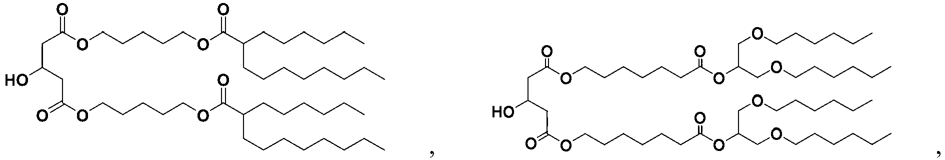 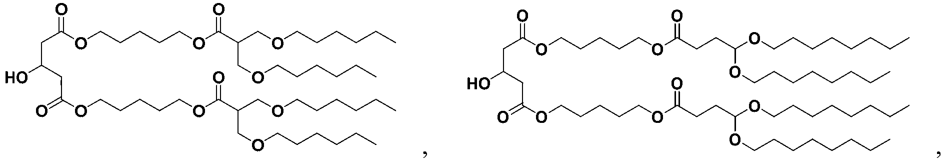 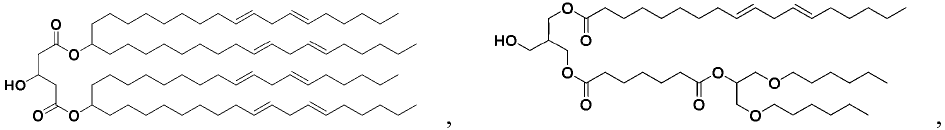 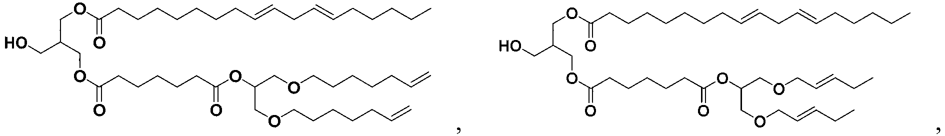 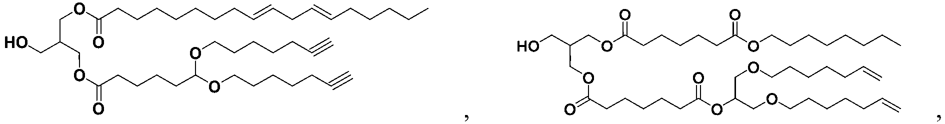 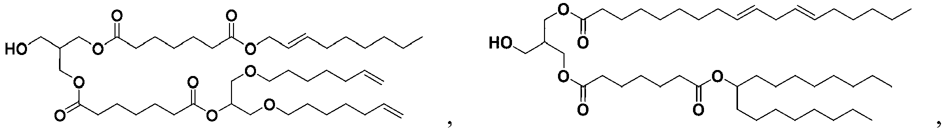 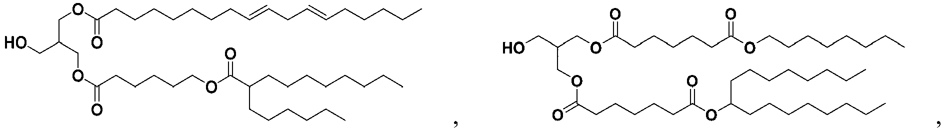 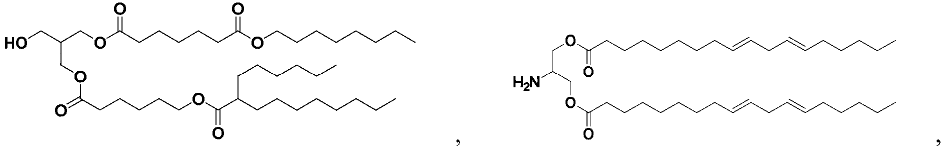 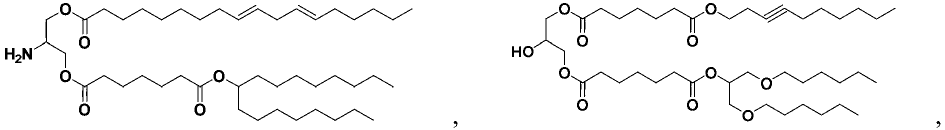 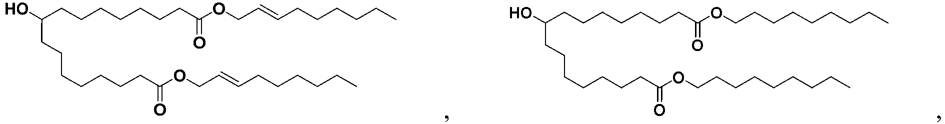 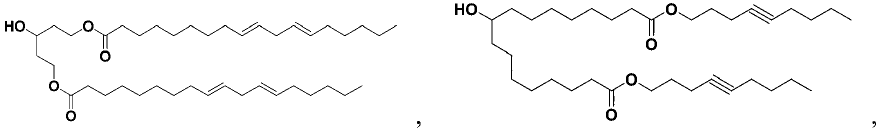 and

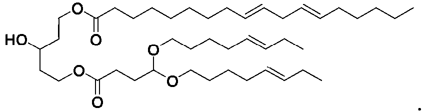

In the present application, after obtaining LYS-C by the reaction between any one of the aforementioned LYS₀ and any one of IM-C', the cationic lipid is obtained after a single-step or step-by-step reaction between LYS-C and two identical or different IM-N; the cationic lipid can also be obtained by reacting any of the aforementioned IM-C' with two identical or different IM-N to obtain IM-C, followed by reaction with LYS₀. Or, the cationic lipid can be obtained by a reaction between two IM-N to obtain IM-C, followed by reaction with LYS₀.

### 2.2. Description of relevant starting materials and/or steps in the preparation process

### 2.2.1. "Protection" and "deprotection" of relevant groups involved in the reaction process

In the present application, the reaction process also involves the "protection" and "deprotection" processes of relevant groups. To prevent a functional group from affecting the reaction, it is usually protected. In addition, when there are two or more functional groups and only the target functional group needs to react, the other functional groups should therefore be protected. The protecting group not only protects the functional group stably but also needs to be removed easily as needed. Therefore, in organic synthesis, it is important to remove only the protecting group bonded to the specified functional group under appropriate conditions.

In the present application, the definitions of "carboxyl protection group" and "amino protection group" are consistent with those in the " Description of Terms" section, and will not be repeated herein.

In the present application, the hydroxyl groups protected by hydroxyl protecting groups are not particularly limited, e.g., alcoholic hydroxyl groups, phenolic hydroxyl groups, and the like. The amino groups protected by amino protecting groups are not particularly limited, such as those from primary amines, secondary amines, hydrazines, and amides, etc. The amino groups in the present application are not particularly limited, including but not limited to primary amino groups, secondary amino groups, tertiary amino groups, and quaternary ammonium ions.

In the present application, the deprotection of protected hydroxyl groups is related to the types of hydroxyl protecting groups. The types of hydroxyl protecting groups are not particularly limited; for example, silyl ethers, and tert-butyl groups can be used to protect terminal hydroxyl groups, and the corresponding deprotection methods include the following:
A: Deprotection of silyl ether protecting groups
   Compounds used for this type of hydroxyl protection include trimethylsilyl ether, triethylsilyl ether, tert-butyldimethylsilyl ether, tert-butyldiphenylsilyl ether, and the like. The deprotection of such silyl ethers uses compounds containing fluoride ions, wherein the compound is preferably tetrabutylammonium fluoride, tetraethylammonium fluoride, hydrofluoric acid, or potassium fluoride, and more preferably tetrabutylammonium fluoride or potassium fluoride. The amount of the fluoride-containing compound is 5 to 20 folds of that of the protected hydroxyl group, and preferably 8 to 15 folds of that of the initiator. When the amount of the fluoride-containing compound used is less than 5 molar equivalents per molar equivalent of protected hydroxyl groups, the deprotonation might not be complete. When the amount of deprotection reagent used exceeds 20 molar equivalents per molar equivalent of the initiator, the excess reagent or compound tends to cause difficulty in the purification process and may blend into the subsequent steps to result in side reactions. The reaction solvent is not particularly limited as long as it can dissolve the reagents and the products, preferably an aprotic solvent, and more preferably tetrahydrofuran or dichloromethane. The reaction temperature is preferably 0 to 30 °C; when it is lower than 0 °C, the reaction rate is relatively slow, and the protecting group cannot be completely removed.
B: Deprotection of t-butyl protecting groups

The deprotection of tert-butyl groups is carried out under an acidic condition, and the pH of the solution is preferably 0 to 4. The acid is not particularly limited but is preferably acetic acid, phosphoric acid, sulfuric acid, hydrochloric acid, or nitric acid, and more preferably hydrochloric acid. The reaction solvent is not particularly limited as long as it can dissolve the reagents and the products, preferably water. The reaction temperature is preferably 0 to 30 °C.

In the present application, the starting materials in each preparation method can be synthesized or purchased.

The intermediates and end-products prepared in the present application can be purified by the purification method including but not limited to extraction, recrystallization, adsorption treatment, precipitation, reverse precipitation, thin-film dialysis, supercritical extraction, and the like. The characterization of the structure and the molecular weight of end-products can use methods including but not limited to NMR, electrophoresis, UV-visible spectrophotometer, FTIR, AFM, GPC, HPLC, MALDI-TOF, circular dichroism spectroscopy, mass spectrometry, and the like.

### 3. Lipid compositions, preparation of lipid compositions, lipid pharmaceutical compositions, formulation of the lipid pharmaceutical composition

### 3.1. Lipid compositions

In the present application, provided herein is a lipid composition containing any cationic lipid whose structure is represented by the general formula (1).

In a specific embodiment of the present application, the lipid composition preferably contains, in addition to a cationic lipid with a structure represented by the general formula (1), one or more types of lipids selected from the group consisting of phospholipid, steroid lipid, and PEGylated lipid, and is selected from any one of the following cases:
Case (1): the lipid composition additionally contains a phospholipid;
Case (2): the lipid composition additionally contains a steroid lipid;
Case (3): the lipid composition additionally contains a PEGylated lipid;
Case (4): the lipid composition additionally contains a phospholipid and a steroid lipid;
Case (5): the lipid composition additionally contains a phospholipid and a PEGylated lipid;
Case (6): the lipid composition additionally contains a steroid lipid and a PEGylated lipid;
Case (7): the lipid composition additionally contains a phospholipid, a steroid lipid, and a PEGylated lipid;
more preferably, the lipid composition additionally contains a phospholipid lipid, a steroid lipid, and a PEGylated lipid, simultaneously.

In a specific embodiment of the present application, the phospholipid in the lipid composition is preferably selected from the group consisting of 1,2-dilinoleoyl-sn-glycero-3-phosphocholine (DLPC), 1,2-dimyristoleoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-plamitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine , 1,2-didecosahexaenoyl-sn-glycero-3-phosphocholine, 1,2-dioleoyl-sn-glycero-3-phosphoethanolamine (DOPE), 1,2-diphytanyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didecosahexaenoyl-sn-glycero-3-phosphoethanolamine, 1,2-dioleoyl-sn-glycero-3-phospho-rac-(1-glycerol) sodium salt (DOPG), dioleoyl phosphatidylserine (DOPS), dipalmitoyl phosphatidylglycerol (DPPG), palmitoyloleoyl phosphatidylethanolamine (POPE), distearoyl phosphatidylethanolamine (DSPE), dipalmitoyl phosphatidylethanolamine (DPPE), dimyristoleoyl phosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethnolamine, phosphatidylserine, phosphatidylinositol, phosphatidic acid, palmitoyloleoyl phosphatidylcholine, lysophosphatidylcholine, lysophosphatidylethanolamine (LPE), and combinations thereof.

In a specific embodiment of the present application, the steroid lipid in the lipid composition is preferably selected from the group consisting of cholesterol, coprostanol, sitosterol, ergosterol, campesterol, stigmasterol, brassicasterol, tomatine, ursolic acid, α-tocopherol, and combinations thereof.

In a specific embodiment of the present application, the PEGylated lipid in the lipid composition is preferably selected from the group consisting of polyethylene glycol-1,2-dimyristoylglycerol (PEG-DMG), polyethylene glycol-distearoylphosphatidylethanolamine (PEG-DSPE), PEG-cholesterol, polyethylene glycol-diacylglycerol (PEG-DAG), and polyethylene glycol-dialkyloxypropyl (PEG-DAA), specifically including polyethylene glycol 500-dipalmitoylphosphatidylcholine, polyethylene glycol 2000-dipalmitoylphosphatidylcholine, polyethylene glycol 500-distearylphosphatidylethanolamine polyethylene glycol 2000-distearylphosphatidylethanolamine, polyethylene glycol 500-1,2-oleoylphosphatidylethanolamine, polyethylene glycol 2000-1,2-oleoylphosphatidylethanolamine, polyethylene glycol 2000-2,3-dimyristoylglycerol (PEG-DMG), and combinations thereof.

In a specific embodiment of the present application, the PEGylated lipid in the lipid composition is selected from the group consisting of the following structures and combinations thereof: 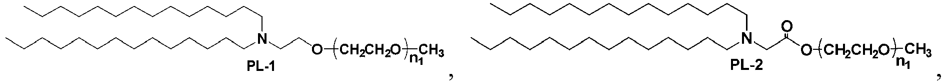 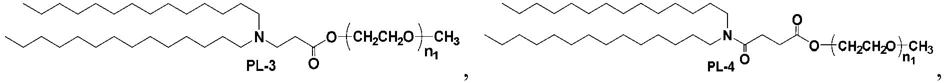 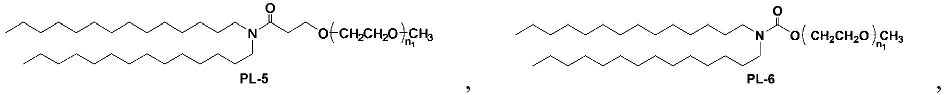 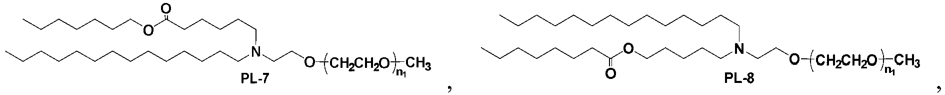 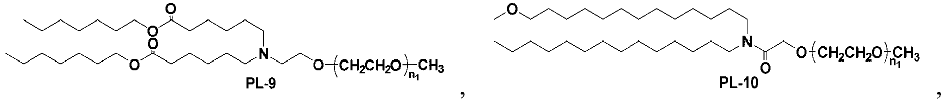 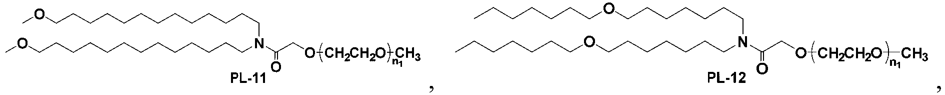 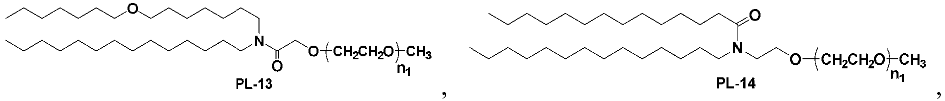 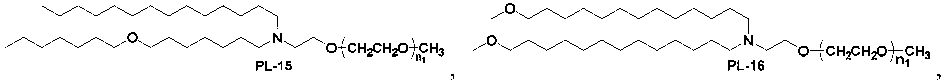 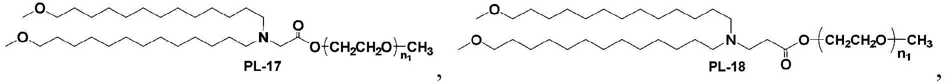 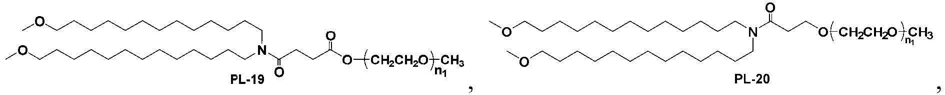 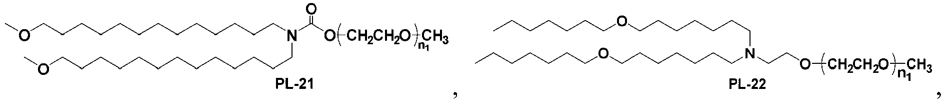 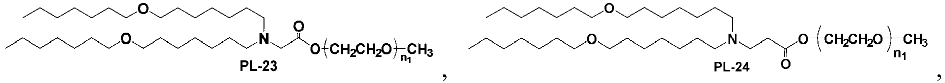 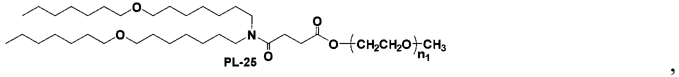 and 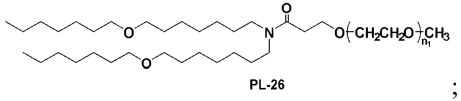 wherein, n₁ is an integer from 25 to 300, and more preferably, n₁ is selected from the group consisting of 44, 45, 46, 47 and 48.

In one specific embodiment of the present application, any of the aforementioned lipid composition preferably contains 20-80% cationic lipid containing two tertiary amine groups represented by the formula (1), 5-15% phospholipid, 25-55% steroid lipid, and 0.5-10% PEGylated lipid, wherein the percentage is the molar percentage of each lipid in the total lipids.

In a specific embodiment of the present application, in any of the aforementioned lipid composition, the molar percentage of cationic lipid in the total lipids is preferably 30-65%, more preferably about 35%, 40%, 45%, 46%, 47%, 48%, 49%, 50%, or 55%.

In a specific embodiment of the present application, in any of the aforementioned lipid composition, the molar percentage of phospholipid in the total lipids is about 7.5-13%, more preferably about 8%, 9%, 10%, 11%, or 12%.

In a specific embodiment of the present application, in any of the aforementioned lipid composition, the molar percentage of steroid lipid in the total lipids is 35-50%, more preferably about 40%, 41%, 42%, 43%, 44%, 45%, 46%, 47%, 48%, 49%, or 50%.

In a specific embodiment of the present application, in any of the aforementioned lipid composition, the molar percentage of PEGylated lipid in the total is 0.5-5%, preferably 1-3%, and more preferably about 1.5%, 1.6%, 1.7%, 1.8%, or 1.9%.

### 3.2. Preparation of lipid compositions

In the present application, the lipid composition can be prepared by the following methods, including but not limited to ethanol injection, microfluidic method, T-tube mixing method, periplasmic extrusion method, preferably ethanol injection and microfluidic method.

### 4. Lipid pharmaceutical compositions and formulations thereof

### 4.1. Lipid pharmaceutical compositions

In one embodiment of the present application, provided herein is a lipid pharmaceutical composition, which contains any aforementioned lipid composition and a drug; wherein, the lipid composition contains any aforementioned cationic lipid containing two tertiary amine groups with the structure represented by formula (1), and the drug is selected from the group consisting of a nucleic acid drug, a gene vaccine, an antitumor drug, a small molecule drug, a peptide drug and a protein drug.

In a specific embodiment of the present application, in a lipid pharmaceutical composition, the nucleic acid drug is selected from the group consisting of RNA, DNA, antisense nucleic acid, plasmid, interfering nucleic acid, aptamer, antagomir, and ribozyme; wherein, the RNA is selected from the group consisting of mRNA, saRNA, circRNA, miRNA, and siRNA; preferably, the nucleic acid drug is selected from the group consisting of DNA, mRNA, miRNA, and siRNA.

In a specific embodiment of the present application, the lipid pharmaceutical composition is preferably used as a drug, and is selected from the group consisting of the following drugs: an antineoplastic agent, an antiviral agent, an antifungal agent, and a vaccine.

In a specific embodiment of the present application, the N/P ratio of the lipid composition to the nucleic acid is preferably (0.1~100): 1, more preferably (0.2~30): 1, and most preferably (0.5~20): 1.

### 4.2. Formulations of Lipid Pharmaceutical Compositions

In a specific embodiment of the present application, the drug in the lipid pharmaceutical composition is a nucleic acid drug; the working solution of the formulation of the lipid pharmaceutical composition is deionized water, ultrapure water, phosphate buffer or normal saline, more preferably phosphate buffer or normal saline, and most preferably normal saline; the ratio of the lipid composition to the working solution is preferably (0.05~20) g: 100 mL, more preferably (0.1~10) g: 100 mL, and most preferably (0.2~5) g: 100 mL.

In a specific embodiment of the application, provided herein is a formulation of lipid pharmaceutical composition, which contains the aforementioned lipid pharmaceutical composition and a pharmaceutically acceptable diluent or excipient; the diluent or excipient is preferably deionized water, ultrapure water, phosphate buffer, or normal saline, more preferably phosphate buffer or normal saline, and most preferably normal saline.

In the present application, the preparation of the formulation of lipid pharmaceutical composition includes the following steps:
(1) equilibrate the lipid composition in the diluent or excipient;
(2) add the nucleic acid drug to the equilibrated mixture of the lipid composition and the diluent or excipient, for complexing;
wherein, the equilibrium time is preferably 0.1~12 h, preferably 0.2~6 h, and more preferably 0.5~3 h; the time for complexing is preferably 0.1~12 h, preferably 0.2~5 h, and more preferably 0.5~2 h.

### 5. Liposomes or lipid nanoparticles and preparation thereof

### 5.1. Liposomes or lipid nanoparticles

In a specific embodiment of the present application, provided herein is a liposome or lipid nanoparticle, which contains any lipid composition described above.

In a specific embodiment of the present application, the aforementioned lipid nanoparticle is preferably an LNP pharmaceutical composition, an LPP pharmaceutical composition or a PNP pharmaceutical composition, preferably an LNP pharmaceutical composition, more preferably an LNP-nucleic acid pharmaceutical composition, more preferably an LNP-mRNA pharmaceutical composition.

### 5.2. Preparation of liposomes or lipid nanoparticles

In a specific embodiment of the present application, liposomes containing a cationic lipid represented by formula (1) can be prepared by the following methods, including but not limited to thin-film dispersion method, ultrasonic dispersion method, reverse-phase evaporation method, freeze-drying method, freeze-thaw method, double emulsion method and injection method, preferably by thin-film dispersion method, ultrasonic dispersion method and/or reverse-phase evaporation method.

In a specific embodiment of the present application, lipid nanoparticles can be prepared by the following methods, including but not limited to microemulsion method, double emulsion method, high-shear ultrasonic homogenization method, thin-film hydration extrusion method, and microfluidic method.

In a specific embodiment of the present application, liposomes are prepared by the thin-film dispersion method that includes the following steps:
Step (1): cationic lipid, steroid lipid, phospholipid, and PEGylated lipid are weighed and fully dissolved in an organic solvent, shaken well; the organic solvent is removed by vacuum rotary evaporation under reduced pressure to form an oil film and then by drying with a vacuum pump;
Step (2): phosphate buffer with dissolved cryoprotectant is added and sonicated in a water bath to form a translucent emulsion;
Step (3): the emulsion is processed through a high-pressure homogenizer for overpressure and added to the liposome extruder for film-passing to obtain the liposome;
Step (4): optionally, the liposome is dried in a freeze dryer to obtain a liposome powder;

wherein, the organic solvent is preferably dichloromethane, chloroform, and/or methanol, more preferably chloroform or methanol; wherein, the rotational speed of vacuum rotary evaporation is preferably 30 to 300 rpm, more preferably 50 to 200 rpm, and most preferably 100 to 170 rpm; wherein, the temperature of vacuum rotary evaporation is preferably 10 to 200 °C, more preferably 20 to 200 °C, and most preferably 40 to 80 °C;
preferably, the time of the drying with a vacuum pump is 1 to 72 h, more preferably 5 to 48 h, and most preferably 15 to 36 h;
preferably, the mass concentration of the cryoprotectant dissolved in phosphate buffer is 0.1 to 80%, preferably 1 to 50%, and more preferably 5 to 20%;
preferably, the frequency of the water-bath ultrasonication is 10 to 300 kHz, more preferably 30 to 200 kHz, and most preferably 60 to 150 kHz;
preferably, the time of the water-bath ultrasonication is 0.1 to 5 h, more preferably 0.2 to 2 h, and most preferably 0.25 to 1 h;
preferably, the pressure of the high-pressure homogenizer is 50 to 240 MPa, more preferably 80 to 200 MPa, and most preferably 100 to 150 MPa;
preferably, the times of the overpressure of the high-pressure homogenizer is any integer from 1 to 50, more preferably any integer from 3 to 20, and most preferably any integer from 5 to 10;
preferably, the pressure of the liposome extruder is 50 to 300 MPa, more preferably 80 to 250 MPa, and most preferably 120 to 200 MPa;
preferably, the times of the film-passing of liposome extruder is any integer from 1 to 50, more preferably any integer from 3 to 30, and most preferably any integer from 5 to 20;
preferably, the time of the drying in a freeze dryer is 1 to 120 h, more preferably 5 to 72 h, and most preferably 10 to 36 h.

In the preparation methods for cationic liposomes in the present application, the ratio of cationic liposome to phosphate buffer with dissolved cryoprotectant could be 1 mg : (0.1~100) mL, preferably 1 mg : (0.3~50) mL, and more preferably 1 mg : (0.5~5) mL.

In a specific embodiment of the present application, lipid nanoparticles are preferably prepared by the microfluidic method, and the steps are as follows:
Step (1): each lipid components are dissolved in the organic solvent to obtain a lipid composition dissolved in the organic phase; the organic phase is preferably ethanol;
Step (2): the nucleic acid drug is added to the buffer to obtain an aqueous solution; the aqueous phase is preferably a citrate buffer or sodium acetate buffer;
Step (3): the organic phase solution and aqueous phase solution are mixed by a microfluidic device to form the lipid nanoparticle composition, followed by purification such as ultrafiltration and the like to remove the organic solvent and free nucleic acid molecules.

The following specific examples are further descriptions of the preparation methods of cationic lipids, lipid compositions, and formulations of lipid pharmaceutical compositions, and the biological activity assays for lipid pharmaceutical compositions. The specific examples are disclosed to further illustrate the application, but should not be regarded as a limitation of the scope of the present application. Wherein, in the embodiments of preparing cationic lipids, the structures of end products were characterized by nuclear magnetic resonance (NMR), and the molecular weight was confirmed by mass spectrometry.

### Example 1: Cationic Lipid (E1-1)

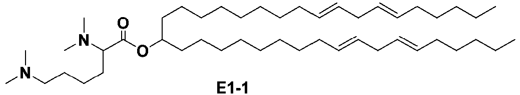

The preparation process is as follows:
Step a: the newly activated magnesium granules (0.33 g, 13.8 mmol) and 2 mL of anhydrous ether were added to a clean round-bottom flask. The bromide 18-bromooctadecane-6,9-diene **(S1-1,** 3.64 g, 11.1 mmol) was dissolved in anhydrous ether (10 mL) and added to a drop funnel. Under ice bath conditions, the bromide ether solution was added dropwise to the magnesium granules. After the addition was completed, the reaction mixture was reacted at 35 °C for 1 h, and then the solution was cooled in an ice bath. **Ethyl formate** (0.37 g, 5.0 mmol) was dissolved in anhydrous ether (8 mL) and added to the drop funnel, and then the solution was added to the reaction mixture with stirring. An exothermic reaction was observed and the reaction mixture began to reflux. After initiation of the reaction, the remaining ether solution containing formate was quickly added to the reaction in the form of a liquid stream, and the reaction mixture was stirred for an additional 1 h at room temperature. Then 5 mL of acetone was added dropwise, followed by ice water (10 mL) for quenching. The reaction mixture was treated with aqueous H₂SO₄ (10% vol., 50 mL) until the solution became homogeneous and the solution was stood for stratification. The aqueous phase was extracted with ether (20 mL*2), and the merged ether layer was dried with anhydrous magnesium sulfate, concentrated to obtain a crude product, which was purified by column chromatography to obtain compound **S1-2** (2.05 g).
Step b: under a nitrogen atmosphere, dicyclohexylcarbodiimide **(DCC,** 0.91 g, 4.4 mmol) was added to a round-bottom flask containing **S1-2** (1.27 g, 2.4 mmol), lysine with both amino groups tertiary-aminated **(S1-3,** 0.40 g, 2.0 mmol), and 4-(dimethylamino)pyridine **(DMAP,** 61.00 mg, 0.5 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E1-1** (1.22 g). ¹H NMR (400 MHz, CDCl₃) δ: 5.41-5.29 (m, 8H, *-*C*H*=C*H*-), 4.87 (t, 1H, -C(=O)OC*H*<), 3.14 (t, 1H, -C*H*N(CH₃)₂), 2.81-2.66 (m, 6H; -C*H*₂N(CH₃)₂, 2H; -CH=CHC*H*₂CH=CH-, 4H), 2.56 (s, 6H, >CHN(C*H*₃)₂), 2.25 (s, 6H, -CH₂N(C*H*₃)₂), 2.09-1.98 (m, 8H, -CH₂C*H*₂CH=CH-), 1.65-1.55 (m, 4H, -C*H*₂C*H*₂CH₂CH₂N(CH₃)₂), 1.47-1.19 (m, 42H; -CH₂C*H*₂CH₂-, -C*H*₂CH₃), 0.87 (t, 6H, -CH₂C*H*₃). MS (ESI): m/z=713.23 ([M+H]⁺).

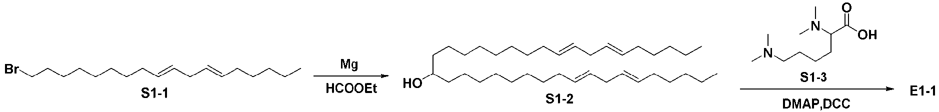

### Example 2: Cationic Lipid (E2-1)

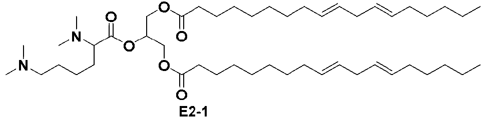

The preparation process is as follows:
Step a: glycerol **(S2-1,** 0.37 g, 4.0 mmol) was adsorbed on the same weight of silica gel by vigorous stirring for 30 min, and then linoleic acid **(S2-2,** 2.24 g, 8.0 mmol), immobilized **lipase R. miehei** (0.15 g) and **molecular sieve** (0.44 g) were added to the formulation successively. The mixture was added to diethyl ether (30 mL) and the reaction was stirred for 48 h at room temperature, and then the reaction was continuously monitored by TLC analysis. After completion of the reaction, the lipase and silica gel were separated by filtration, the filtrate was concentrated, and then the crude product was recrystallized in methanol to obtain the target compound **S2-3** (2.00 g).
Step b: under a nitrogen atmosphere, dicyclohexylcarbodiimide **(DCC,** 0.91 g, 4.4 mmol) was added to a round-bottom flask containing **S2-3** (1.48 g, 2.4 mmol), **S1-3** (0.40 g, 2.0 mmol), and **DMAP** (61.00 mg, 0.5 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E2-1** (1.37 g). ¹H NMR (400 MHz, CDCl₃) δ: 5.41-5.26 (m, 9H; -C*H*=C*H-,* 8H; -C(=O)OC*H*<, 1H), 4.36-4.28 (dd, 2H, >CH(C*H*₂OC(=O))₂-), 4.17-4.10 (dd, 2H, >CH(C*H*₂OC(=O))₂-), 3.15 (t, 1H, -C*H*N(CH₃)₂), 2.80-2.66 (m, 6H; -C*H*₂N(CH₃)₂, 2H; -CH=CHC*H*₂CH=CH-, 4H), 2.56 (s, 6H, >CHN(C*H*₃)₂), 2.34-2.25 (m, 10H; -OC(=O)C*H*₂-, 4H; -CH₂N(C*H*₃)₂, 6H), 2.08-1.97 (m, 8H, -CH₂C*H*₂CH=CH-), 1.66-1.55 (m, 4H, -C*H*₂C*H*₂CH₂CH₂N(CH₃)₂), 1.49-1.19 (m, 34H; -CH₂C*H*₂CH₂-, -C*H*₂CH₃), 0.86 (t, 6H, -CH₂C*H*₃). MS (ESI): m/z=801.67 ([M+H]⁺).

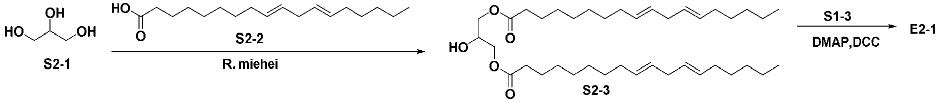

### Example 3: Cationic Lipid (E3-1)

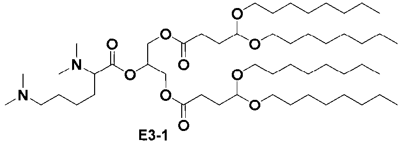

The preparation process is as follows:
Step a: pyridinium tosylate **(PPTS,** 0.20 g, 0.8 mmol) was dissolved in toluene at room temperature, then 4,4-diethoxybutyronitrile **(S3-2,** 2.36 g, 15.0 mmol) and n-octanol **(S3-1,** 5.85 g, 45.0 mmol) were added to the mixture successively, and the mixture was reacted at 135 °C for 20 h. After completion of the reaction, the reaction solution was cooled to room temperature, extracted, dried, concentrated, and then the crude product was purified by column chromatography to obtain compound **S3-3** (2.05 g).
Step b: **S3-3** (1.63 g, 5.0 mmol) was dissolved in ethanol (20 mL), then potassium hydroxide **(KOH,** 0.70 g, 12.5 mmol) was added dropwise to the mixture and the reaction mixture was reacted at 110 °C for 24 h. After completion of the reaction, the reaction solution was cooled to room temperature. The pH of the solution was adjusted to 5 by adding 1 M HCl. The solution was extracted twice (10 mL*2) with hexane, and the organic layers were merged. The combined organic layers were washed with water and saline, dried with anhydrous magnesium sulfate, filtered and concentrated, and then the crude product was purified by column chromatography to obtain the target compound **S3-4** (1.67 g, 95%).
Step c: **S2-1** (0.37 g, 4.0 mmol) was adsorbed on the same weight of silica gel by vigorous stirring for 30 min, and then **S3-4** (2.75 g, 8.0 mmol), immobilized **lipase R. miehei** (0.19 g) and **molecular sieve** (0.53 g) were added to the formulation successively. The mixture was added to diethyl ether (30 mL) and the reaction was stirred for 48 h at room temperature, and then the reaction was continuously monitored by TLC analysis. After completion of the reaction, the lipase and silica gel were separated by filtration, the filtrate was concentrated, and then the crude product was recrystallized in methanol to obtain the target compound **S3-5** (2.35 g).
Step d: under a nitrogen atmosphere, **DCC** (0.91 g, 4.4 mmol) was added to a round-bottom flask containing **S3-5** (1.79 g, 2.4 mmol), **S1-3** (0.40 g, 2.0 mmol), and **DMAP** (61.00 mg, 0.5 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E3-1** (1.57 g). ¹H NMR (400 MHz, CDCl₃) δ: 5.29-5.26 (m, 1H, -C(=O)OC*H*<), 4.63 (t, 2H, >C*H*(OCH₂)₂-), 4.36-4.28 (dd, 2H, >CH(CH₂OC(=O))₂-), 4.17-4.11 (dd, 2H, >CH(CH₂OC(=O))₂-), 3.53-3.14 (m, 9H; >CH(OC*H*₂)₂-, 8H; -C*H*N(CH₃)₂, 1H), 2.80-2.66 (m, 2H, -CH₂N(C*H*₃)₂), 2.58 (s, 6H, >CHN(C*H*₃)₂), 2.35-2.25 (m, 10H; -OC(=O)C*H*₂-, 4H; -CH₂N(C*H*₃)₂, 6H), 2.02-1.82 (m, 4H, -C*H*₂CH(OCH₂)₂-), 1.65-1.55 (m, 4H, -C*H*₂C*H*₂CH₂CH₂N(CH₃)₂), 1.48-1.19 (m, 50H; -CH₂C*H*₂CH₂-, -C*H*₂CH₃), 0.88 (t, 12H, -CH₂C*H*₃). MS (ESI): m/z=928.77 ([M+H]⁺).

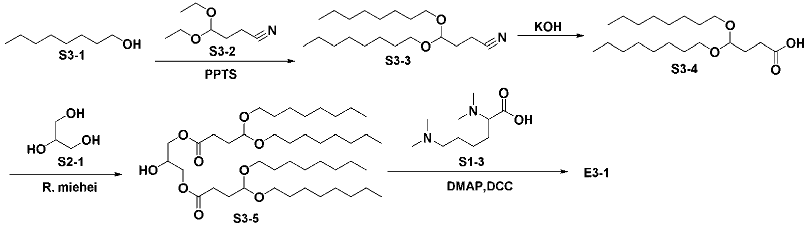

### Example 4: Cationic Lipid (E4-1)

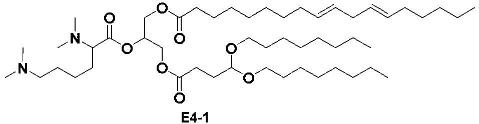

The preparation process is as follows:
Step a: glycerol **(S4-1,** 1.65 g, 8.0 mmol) with a TBS-protected hydroxyl group was dissolved in DMF (120 mL) at room temperature, then **S2-2** (4.61 g, 16.5 mmol), **DMAP** (0.20 g, 1.6 mmol) and *N,N*-diisopropyelethylamine **(DIPEA,** 2.48 g, 19.2 mmol) were added to the solution successively. 1-ethyl-(3-dimethylaminopropyl) carbodiimide hydrochloride **(EDCI,** 3.69 g, 19.2 mmol) was added to the solution in three batches within 15 min and DMF (1 mL) was added at the same time, and then the mixture was stirred for 16 h at room temperature. After completion of the reaction, the mixture was washed with water, 1 M HCI and 5% sodium bicarbonate, dried with anhydrous magnesium sulfate, filtered, concentrated, and then the residue was purified by column chromatography to obtain the target compound monoglyceride **S4-2** (2.36 g).
Step b: under a nitrogen atmosphere, **DCC** (1.81 g, 8.8 mmol) was added to a round-bottom flask containing **S4-2** (2.25 g, 4.8 mmol), **S3-4** (1.38 g, 4.0 mmol), and **DMAP** (0.12 g, 1.0 mmol) dissolved in dichloromethane (50 mL), and the reaction was conducted for 16 h at room temperature. After completion of the reaction, the precipitate was removed by filtration, the filtrate was concentrated, and then the residue was purified by column chromatography to obtain compound **S4-3** (2.69 g).
Step c: the above product **S4-3** (2.39 g, 3.0 mmol) was dissolved in THF (30 mL), and **TBAF** (30 mL, 1 M) was added to the solution under nitrogen protection. The reaction was carried out overnight to remove TBS protection. After completion of the reaction, the solution was concentrated, extracted, and the organic layers were combined, dried with anhydrous magnesium sulfate, filtered and concentrated, and then the crude product was purified by column chromatography to obtain double-substituted diglyceride **S4-4** (1.80 g, 87.9%).
Step d: under a nitrogen atmosphere, **DCC** (0.91 g, 4.4 mmol) was added to a round-bottom flask containing **S4-4** (1.63 g, 2.4 mmol), **S1-3** (0.40 g, 2.0 mmol), and **DMAP** (61.00 mg, 0.5 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E4-1** (1.46 g). ¹H NMR (400 MHz, CDCl₃) δ: 5.42-5.26 (m, 5H; -C*H*=C*H-,* 4H; -C(=O)OCH<, 1H), 4.62 (t, 1H, >C*H*(OCH₂)₂-), 4.36-4.28 (dd, 2H, >CH(C*H*₂OC(=O))₂-), 4.17-4.10 (dd, 2H, >CH(C*H*₂OC(=O))₂-), 3.52-3.13 (m, 5H; >CH(OC*H*₂)₂-, 4H; -C*H*N(CH₃)₂, 1H), 2.80-2.66 (m, 4H; -C*H*₂N(CH₃)₂, 2H; -CH=CHC*H*₂CH=CH-, 2H), 2.56 (s, 6H, >CHN(C*H*₃)₂), 2.34-2.25 (m, 10H; -OC(=O)C*H*₂-, 4H; -CH₂N(C*H*₃)₂, 6H), 2.11-1.82 (m, 6H; -CH₂C*H*₂CH=CH-, 4H; -C*H*₂CH(OCH₂)₂-, 2H), 1.65-1.55 (m, 4H, -C*H*₂C*H*₂CH₂CH₂N(CH₃)₂), 1.48-1.20 (m, 42H; -CH₂C*H*₂CH₂-, -C*H*₂CH₃), 0.88 (t, 9H, -CH₂C*H*₃). MS (ESI): m/z=864.72 ([M+H]⁺).

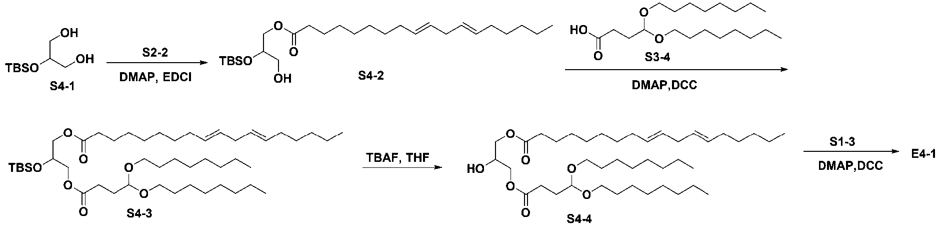

### Example 5: Cationic Lipid (E5-1)

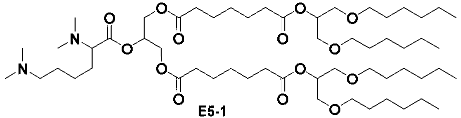

The preparation process is as follows:
Step a: under a nitrogen atmosphere, **DCC** (2.72 g, 13.2 mmol) was added to a round-bottom flask containing 7-(tert-butoxy)-7-oxoheptanoic acid **(S5-2,** 1.30 g, 6.0 mmol), **S5-1** (1.87 g, 7.2 mmol, **S5-1** was obtained by the reaction between a glycerol with a TBS-protected hydroxyl group and bromohexane, followed by deprotection), and **DMAP** (0.18 g, 1.5 mmol) dissolved in dichloromethane (50 mL), and the reaction was conducted for 16 h at room temperature. After completion of the reaction, the precipitate was removed by filtration and the filtrate was concentrated to obtain a crude product. In a dry and clean round-bottom flask, a solution of trifluoroacetic acid/dichloromethane (1:2, v/v) was prepared, and the dichloromethane solution of the above crude product was added dropwise under ice bath condition, followed by reacting at room temperature for 2 h. After completion of the reaction, the reaction solution was concentrated, and purified water was added, followed by extraction with dichloromethane. The extract was dried with anhydrous magnesium sulfate, filtered, the filtrate was concentrated, and then the crude product was recrystallized to obtain compound **S5-3** (1.93 g).
Step b: **S2-1** (0.18 g, 2.0 mmol) was adsorbed on the same weight of silica gel by vigorous stirring for 30 min, and then **S5-3** (1.61 g, 4.0 mmol), immobilized **lipase R. miehei** (0.11 g) and **molecular sieve** (0.30 g) were added to the formulation successively. The mixture was added to diethyl ether (30 mL) and the reaction was stirred for 48 h at room temperature, and then the reaction was continuously monitored by TLC analysis. After completion of the reaction, the lipase and silica gel were separated by filtration, the filtrate was concentrated, and then the crude product was recrystallized in methanol to obtain the target compound **S5-4** (1.38 g).
Step c: under a nitrogen atmosphere, **DCC** (0.45 g, 2.2 mmol) was added to a round-bottom flask containing **S5-4** (1.03 g, 1.2 mmol), **S1-3** (0.20 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E5-1** (0.87 g). ¹H NMR (400 MHz, CDCl₃) δ: 5.28-5.27 (m, 1H, >CHC(=O)OC*H*<), 5.17-5.01 (m, 2H, >C*H*(CH₂OCH₂)₂-), 4.36-4.28 (dd, 2H, >CH(C*H*₂OC(=O))₂-), 4.17-4.10 (dd, 2H, >CH(C*H*₂OC(=O))₂-), 3.59-3.52 (m, 8H, >C*H*(CH₂OCH₂)₂-), 3.48-3.37 (m, 8H; >C*H*(CH₂OCH₂)₂-), 3.14 (t, 1H, -C*H*N(CH₃)₂), 2.80-2.66 (m, 2H, -CH₂N(C*H*₃)₂), 2.56 (s, 6H, >CHN(C*H*₃)₂), 2.34-2.26 (m, 14H; -OC(=O)C*H*₂-, 8H; -CH₂N(C*H*₃)₂, 6H), 1.66-1.55 (m, 4H, -C*H*₂C*H*₂CH₂CH₂N(CH₃)₂), 1.48-1.21 (m, 46H; -CH₂C*H*₂CH₂-, -C*H*₂CH₃), 0.90 (t, 12H, -CH₂C*H*₃). MS (ESI): m/z=1044.78 ([M+H]⁺).

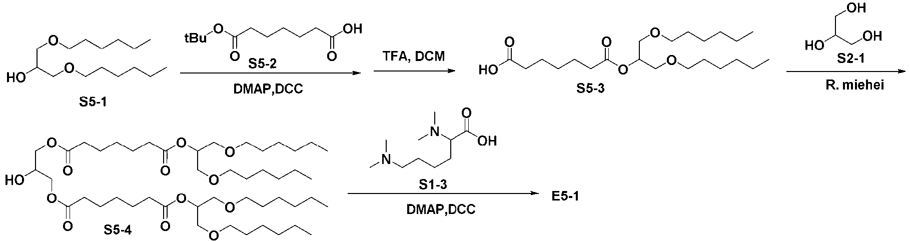

### Example 6: Cationic Lipid (E6-1)

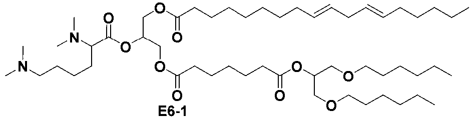

The preparation process is as follows:
Step a: under a nitrogen atmosphere, **DCC** (1.81 g, 8.8 mmol) was added to a round-bottom flask containing **S4-2** (2.25 g, 4.8 mmol), **S5-3** (1.61 g, 4.0 mmol), and **DMAP** (0.12 g, 1.0 mmol) dissolved in dichloromethane (50 mL), and the reaction was conducted for 16 h at room temperature. After completion of the reaction, the precipitate was removed by filtration, the filtrate was concentrated, and then the residue was purified by column chromatography to obtain compound **S6-1** (2.86 g).
Step b: the above product **S6-1** (2.56 g, 3.0 mmol) was dissolved in THF (30 mL), and **TBAF** (30 mL, 1 M) was added to the solution under nitrogen protection. The reaction was conducted overnight to remove TBS protection. After completion of the reaction, the solution was concentrated, extracted, and the organic layers were combined, dried with anhydrous magnesium sulfate, filtered and concentrated, and then the crude product was purified by column chromatography to obtain double-substituted diglyceride **S6-2** (1.96g).
Step c: under a nitrogen atmosphere, **DCC** (0.91 g, 4.4 mmol) was added to a round-bottom flask containing **S6-2** (1.77 g, 2.4 mmol), **S1-3** (0.40 g, 2.0 mmol), and **DMAP** (61.00 mg, 0.5 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E6-1** (1.58 g). ¹H NMR (400 MHz, CDCl₃) δ: 5.42-5.27 (m, 5H; -C*H*=C*H-,* 4H; >CHC(=O)OC*H*<, 1H), 5.17-5.03 (m, 1H, >C*H*(CH₂OCH₂)₂-), 4.36-4.29 (dd, 2H, >CH(CH₂OC(=O))₂-), 4.17-4.10 (dd, 2H, >CH(CH₂OC(=O))₂-), 3.58-3.52 (m, 4H, >C*H*(CH₂OCH₂)₂-), 3.48-3.38 (m, 4H, >C*H*(CH₂OCH₂)₂-), 3.14 (t, 1H, -C*H*N(CH₃)₂), 2.80-2.67 (m, 4H; -C*H*₂N(CH₃)₂, 2H; -CH=CHC*H*₂CH=CH-, 2H), 2.56 (s, 6H, >CHN(C*H*₃)₂), 2.34-2.25 (m, 12H; -OC(=O)C*H*₂-, 6H; -CH₂N(C*H*₃)₂, 6H), 2.08-1.98 (m, 4H, -CH₂C*H*₂CH=CH-), 1.65-1.56 (m, 4H, -C*H*₂C*H*₂CH₂CH₂N(CH₃)₂), 1.49-1.20 (m, 40H; -CH₂C*H*₂CH₂-, -C*H*₂CH₃), 0.86 (t, 9H, -CH₂C*H*₃). MS (ESI): m/z=922.72 ([M+H]⁺).

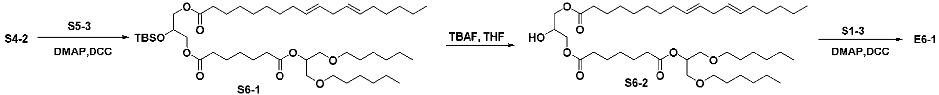

### Example 7: Cationic Lipid (E7-1)

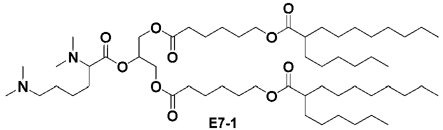

The preparation process is as follows:
Step a: under a nitrogen atmosphere, **DCC** (3.63 g, 17.6 mmol) was added to a round-bottom flask containing **S7-2** (2.05 g, 8.0 mmol), **S7-1** (1.80 g, 9.6 mmol), and **DMAP** (0.24 g, 2.0 mmol) dissolved in dichloromethane (50 mL), and the reaction was conducted for 16 h at room temperature. After completion of the reaction, the precipitate was removed by filtration, the filtrate was concentrated, and then the residue was purified by column chromatography to obtain compound S7-3 (2.92 g).
Step b: Removal of the tBu protecting group. In a dry and clean round-bottom flask, a solution of trifluoroacetic acid/dichloromethane (1:2, v/v) was prepared, and a dichloromethane solution of **S7-3** (2.14 g, 5.0 mmol) was slowly added dropwise under ice bath conditions for 2 h at room temperature. After completion of the reaction, the reaction solution was concentrated, and purified water was added, followed by extraction with methylene chloride. The extract was dried with anhydrous magnesium sulfate, filtered, and then the filtrate was concentrated, recrystallized to obtain compound **S7-4** (1.71 g, 92.1%).
Step c: **S2-1** (0.18 g, 2.0 mmol) was adsorbed on the same weight of silica gel by vigorous stirring for 30 min, and then **S7-4** (1.48 g, 4.0 mmol), immobilized **lipase R. miehei** (0.10 g) and **molecular sieve** (0.28 g) were added to the formulation successively. The mixture was added to diethyl ether (30 mL) and the reaction was stirred for 48 h at room temperature, and then the reaction was continuously monitored by TLC analysis. After completion of the reaction, the lipase and silica gel were separated by filtration, the filtrate was concentrated, and then the crude product was recrystallized in methanol to obtain the target compound **S7-5** (1.28 g).
Step d: under a nitrogen atmosphere, **DCC** (0.45 g, 2.2 mmol) was added to a round-bottom flask containing **S7-5** (0.96 g, 1.2 mmol), **S1-3** (1.03 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E7-1** (0.82 g). ¹H NMR (400 MHz, CDCl₃) δ: 5.29-5.25 (m, 1H, -C(=O)OCH<), 4.37-4.28 (dd, 2H, >CH(C*H*₂OC(=O))₂-), 4.18-4.10 (dd, 2H, >CH(C*H*₂OC(=O))₂-), 4.02 (t, 4H, -C*H*₂OC(=O)CH<), 3.14 (t, 1H, -C*H*N(CH₃)₂), 2.80-2.66 (m, 2H, -CH₂N(C*H*₃)₂), 2.56 (s, 6H, >CHN(C*H*₃)₂), 2.35-2.25 (m, 12H; -OC(=O)C*H*₂-, 4H; -CH₂OC(=O)C*H*<, 2H; -CH₂N(C*H*₃)₂, 6H), 1.65-1.57 (m, 4H, -C*H*₂C*H*₂CH₂CH₂N(CH₃)₂), 1.48-1.19 (m, 62H; -CH₂C*H*₂CH₂-, -C*H*₂CH₃), 0.88 (t, 12H, -CH₂C*H*₃). MS (ESI): m/z=980.80 ([M+H]⁺).

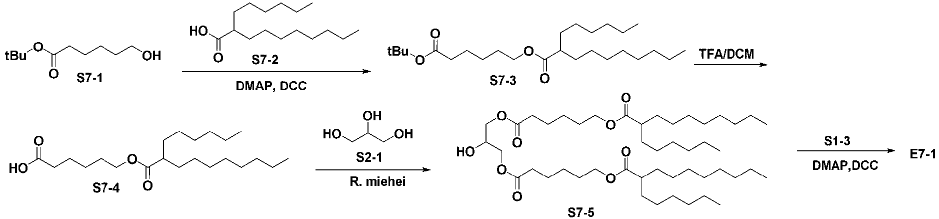

### Example 8: Cationic Lipid (E8-1)

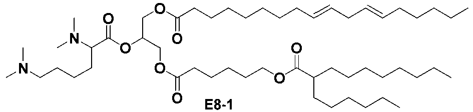

The preparation process is as follows:
Step a: under a nitrogen atmosphere, **DCC** (1.81 g, 8.8 mmol) was added to a round-bottom flask containing **S4-2** (2.25 g, 4.8 mmol), **S7-4** (1.48 g, 4.0 mmol), and **DMAP** (0.12 g, 1.0 mmol) dissolved in dichloromethane (50 mL), and the reaction was conducted for 16 h at room temperature. After completion of the reaction, the precipitate was removed by filtration, the filtrate was concentrated, and then the residue was purified by column chromatography to obtain compound **S8-1** (2.76 g).
Step b: the above product **S8-1** (2.56 g, 3.0 mmol) was dissolved in THF (30 mL), and **TBAF** (30 mL, 1 M) was added to the solution under nitrogen protection. The reaction was conducted overnight to remove TBS protection. After completion of the reaction, the solution was concentrated, extracted, and the organic layers were combined, dried with anhydrous magnesium sulfate, filtered and concentrated, and then the crude product was purified by column chromatography to obtain double-substituted diglyceride **S8-2** (1.86 g, 87.8%).
Step c: under a nitrogen atmosphere, **DCC** (0.91 g, 4.4 mmol) was added to a round-bottom flask containing **S8-2** (1.70 g, 2.4 mmol), **S1-3** (0.40 g, 2.0 mmol), and **DMAP** (61.00 mg, 0.5 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E8-1** (1.50 g). ¹H NMR (400 MHz, CDCl₃) δ: 5.42-5.29 (m, 5H, -C*H*=C*H-,* 4H; >CHC(=O)OC*H*<, 1H), 4.36-4.28 (dd, 2H, >CH(C*H*₂OC(=O))₂-), 4.17-4.10 (dd, 2H, >CH(C*H*₂OC(=O))₂-), 4.01 (t, 2H, -C*H*₂OC(=O)CH<), 3.15 (t, 1H, -C*H*N(CH₃)₂), 2.80-2.66 (m, 4H; -C*H*₂N(CH₃)₂, 2H; -CH=CHC*H*₂CH=CH-, 2H), 2.58 (s, 6H, >CHN(C*H*₃)₂), 2.34-2.25 (m, 11H; -OC(=O)C*H*₂-, 4H; -CH₂OC(=O)C*H*<, 1H; -CH₂N(C*H*₃)₂, 6H), 2.08-1.96 (m, 4H, -CH₂C*H*₂CH=CH-), 1.66-1.55 (m, 4H, -C*H*₂C*H*₂CH₂CH₂N(CH₃)₂), 1.41-1.21 (m, 48H; -CH₂C*H*₂CH₂-, -C*H*₂CH₃), 0.86 (t, 9H, -CH₂C*H*₃). MS (ESI): m/z=890.73 ([M+H]⁺).

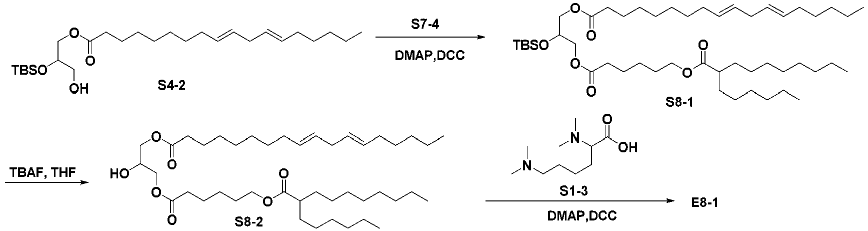

### Example 9: Cationic Lipid (E9-1)

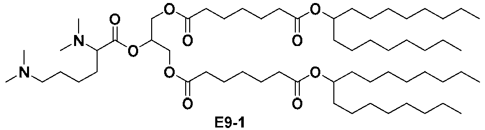

The preparation process is as follows:
Step a: under a nitrogen atmosphere, **DCC** (2.72 g, 13.2 mmol) was added to a round-bottom flask containing **S5-2** (1.30 g, 6.0 mmol), **S9-1** (1.85 g, 7.2 mmol), and **DMAP** (0.18 g, 1.5 mmol) dissolved in dichloromethane (50 mL), and the reaction was conducted for 16 h at room temperature. After completion of the reaction, the precipitate was removed by filtration and the filtrate was concentrated to obtain a crude product. In a dry and clean round-bottom flask, a solution of trifluoroacetic acid/dichloromethane (1:2, v/v) was prepared, and the dichloromethane solution of the above crude product was added dropwise under ice bath condition. The reaction was conducted at room temperature for 2 h. After completion of the reaction, the reaction solution was concentrated, and purified water was added, followed by extraction with dichloromethane. The extract was dried with anhydrous magnesium sulfate, filtered, the filtrate was concentrated, and then the crude product was recrystallized to obtain compound **S9-2** (1.95 g).
Step b: **S2-1** (0.18 g, 2.0 mmol) was adsorbed on the same weight of silica gel by vigorous stirring for 30 min, and then **S9-2** (1.60 g, 4.0 mmol), immobilized **lipase R. miehei** (0.11 g) and **molecular sieve** (0.30 g) were added to the formulation successively. The mixture was added to diethyl ether (30 mL) and the reaction was stirred for 48 h at room temperature, and then the reaction was continuously monitored by TLC analysis. After completion of the reaction, the lipase and silica gel were separated by filtration, the filtrate was concentrated, and then the crude product was recrystallized in methanol to obtain the target compound **S9-3** (1.36 g).
Step c: under a nitrogen atmosphere, **DCC** (0.45 g, 2.2 mmol) was added to a round-bottom flask containing **S9-3** (1.02 g, 1.2 mmol), **S1-3** (0.20 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E9-1** (0.87 g). ¹H NMR (400 MHz, CDCl₃) δ: 5.37-5.31 (m, 1H, >CHC(=O)OC*H*<), 4.91-4.83 (m, 2H, -C(=O)OC*H*(CH₂)₂-), 4.38-4.29 (m, 2H, >CH(C*H*₂OC(=O))₂-), 4.19-4.13 (m, 2H, >CH(C*H*₂OC(=O))₂-), 3.17 (t, 1H, -C*H*N(CH₃)₂), 2.41-2.27 (m, 22H, -C*H*₂N(CH₃)₂, 2H; >CHN(C*H*₃)₂, 6H; -CH₂N(C*H*₃)₂, 6H; -OC(=O)C*H*₂-, 8H), 1.72-1.58 (m, 10H, -C*H*₂CH₂CH₂CH₂N(CH₃)₂), 2H; -C(=O)OCH(C*H*₂)₂-, 8H), 1.56-1.47 (m, 10H, -CH₂C*H*₂CH₂CH₂N(CH₃)₂), 2H; -OC(=O)CH₂C*H*₂-, 8H), 1.33-1.20 (m, 54H; -CH₂C*H*₂CH₂-, -C*H*₂CH₃), 0.89 (t, 12H, -CH₂C*H*₃). MS (ESI): m/z=1037.87 ([M+H]⁺).

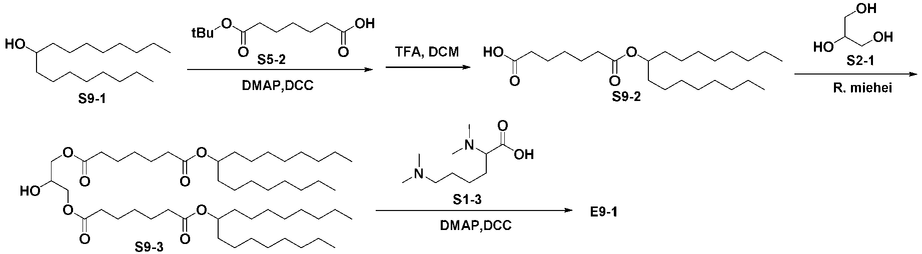

### Example 10: Cationic Lipid (E10-1)

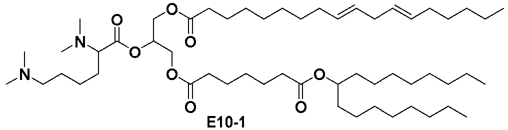

The preparation process is as follows:
Step a: under a nitrogen atmosphere, **DCC** (1.81 g, 8.8 mmol) was added to a round-bottom flask containing **S4-2** (2.25 g, 4.8 mmol), **S9-2** (1.60 g, 4.0 mmol), and **DMAP** (0.12 g, 1.0 mmol) dissolved in dichloromethane (50 mL), and the reaction was conducted for 16 h at room temperature. After completion of the reaction, the precipitate was removed by filtration, the filtrate was concentrated, and then the residue was purified by column chromatography to obtain compound **S10-1** (2.84 g).
Step b: the above product **S10-1** (2.55 g, 3.0 mmol) was dissolved in THF (30 mL), and **TBAF** (30 mL, 1 M) was added to the solution under nitrogen protection. The reaction was conducted overnight to remove TBS protection. After completion of the reaction, the solution was concentrated, extracted, and the organic layers were combined, dried with anhydrous magnesium sulfate, filtered and concentrated, and then the crude product was purified by column chromatography to obtain double-substituted diglyceride **S10-2** (1.94 g, 88.2%).
Step c: under a nitrogen atmosphere, **DCC** (0.91 g, 4.4 mmol) was added to a round-bottom flask containing **S10-2** (1.76 g, 2.4 mmol), **S1-3** (0.40 g, 2.0 mmol), and **DMAP** (61.00 mg, 0.5 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E10-1** (1.55 g). ¹H NMR (400 MHz, CDCl₃) δ: 5.37-5.27 (m, 5H; -C*H*=C*H-,* 4H; >CHC(=O)OC*H*<, 1H), 4.91-4.82 (m, 1H, >C*H*(CH₂CH₂)₂-), 4.38-4.29 (dd, 2H, >CH(C*H*₂OC(=O))₂-), 4.19-4.13 (dd, 2H, >CH(C*H*₂OC(=O))₂-), 3.17 (t, 1H, -C*H*N(CH₃)₂), 2.80-2.66 (m, 4H; -C*H*₂N(CH₃)₂, 2H; -CH=CHC*H*₂CH=CH-, 2H), 2.58 (s, 6H, >CHN(C*H*₃)₂), 2.34-2.25 (m, 12H; -OC(=O)C*H*₂-, 6H; -CH₂N(C*H*₃)₂, 6H), 2.08-1.98 (m, 4H, -CH₂C*H*₂CH=CH-), 1.66-1.55 (m, 4H, -C*H*₂C*H*₂CH₂CH₂N(CH₃)₂), 1.41-1.20 (m, 52H; -CH₂C*H*₂CH₂-, -C*H*₂CH₃), 0.87 (t, 9H, -CH₂C*H*₃). MS (ESI): m/z=918.76 ([M+H]⁺).

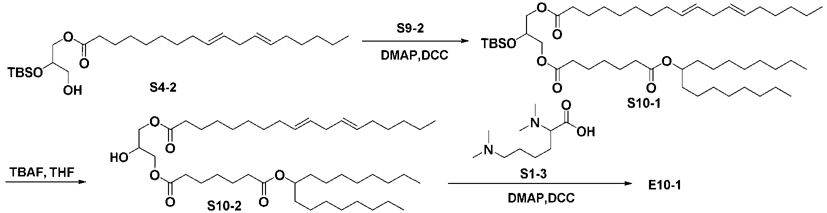

### Example 11: Cationic Lipid (E11-1)

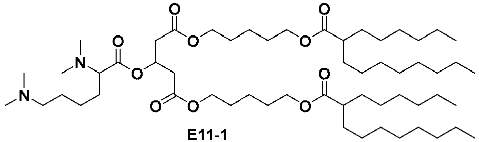

The preparation process is as follows:
Step a: under a nitrogen atmosphere, **DCC** (7.25 g, 35.2 mmol) was added to a round-bottom flask containing **S7-2** (4.10 g, 16.0 mmol), 5-(tert-butyldimethylsilane)-1-pentanol **(S11-1,** 2.00 g, 19.2 mmol), and **DMAP** (0.49 g, 4.0 mmol) dissolved in dichloromethane (200 mL), and the reaction was conducted for 16 h at room temperature. After completion of the reaction, the precipitate was removed by filtration, and the filtrate was concentrated. The residue was dissolved in tetrahydrofuran (30 mL), and then 30 mL of TBAF tetrahydrofuran solution (1 M) was added. The reaction was conducted overnight to remove TBS protection. After completion of the reaction, the solution was concentrated, extracted, and the organic phases were combined, dried with anhydrous magnesium sulfate, and filtered. The filtrate was concentrated to obtain crude S11-2, which was then purified by column chromatography to obtain the compound **S11-2** (4.42 g).
Step b: 3-hydroxyglutaric acid **(S11-3,** 0.79 g, 3.0 mmol) with a TBS-protected hydroxyl group was dissolved in acetonitrile (50 mL) and N,N'-diisopropylcarbodiimide **(DIC,** 1.86 mL, 12.0 mmol) was added dropwise to the solution, then **DMAP** (0.55 g, 4.5 mmol) and **S11-2** (4.12 g, 12.0 mmol) were added to the solution successively. The mixture was reacted with stirring overnight at room temperature. After completion of the reaction, the precipitate was removed by filtration, and the filtrate was concentrated. The residue was dissolved in DCM (30 mL), the resulting solution was washed with water (20 mL*2) and brine (30 mL*1) in turn, the organic phase was collected and dried with anhydrous magnesium sulfate, filtered and concentrated, and then purified by column chromatography to obtain **S11-4** (2.58 g, 94.3%).
Step c: the above product **S11-4** (1.82 g, 2.0 mmol) was dissolved in THF (20 mL), and **TBAF** (20 mL, 1 M) was added to the solution under nitrogen protection. The reaction was conducted overnight to remove TBS protection. After completion of the reaction, the solution was concentrated, extracted, the organic layer was combined, dried with anhydrous magnesium sulfate, filtered and concentrated, and then the crude product was purified by column chromatography to obtain double-substituted diglyceride **S11-5** (1.39 g, 87.3%).
Step d: under a nitrogen atmosphere, **DCC** (0.45 g, 2.2 mmol) was added to a round-bottom flask containing **S11-5** (0.96 g, 1.2 mmol), **S1-3** (0.20 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E11-1** (0.83 g). ¹H NMR (400 MHz, CDCl₃) δ: 5.61-5.51 (m, 1H, -C(=O)OC*H*<), 4.02 (t, 8H, -C(=O)OC*H*₂-), 3.17 (t, 1H, -C*H*N(CH₃)₂), 2.80-2.66 (m, 6H; -C*H*₂N(CH₃)₂, 2H; >CH(C*H*₂C(=O)O)₂-, 4H), 2.56 (s, 6H, >CHN(C*H*₃)₂), 2.33-2.25 (m, 8H, -CH₂OC(=O)C*H*<, 2H; -CH₂N(C*H*₃)₂, 6H), 1.66-1.55 (m, 4H, -C*H*₂C*H*₂CH₂CH₂N(CH₃)₂), 1.42-1.21 (m, 62H; -CH₂C*H*₂CH₂-, -C*H*₂CH₃), 0.88 (t, 12H, -CH₂C*H*₃). MS (ESI): m/z=980.80 ([M+H]⁺).

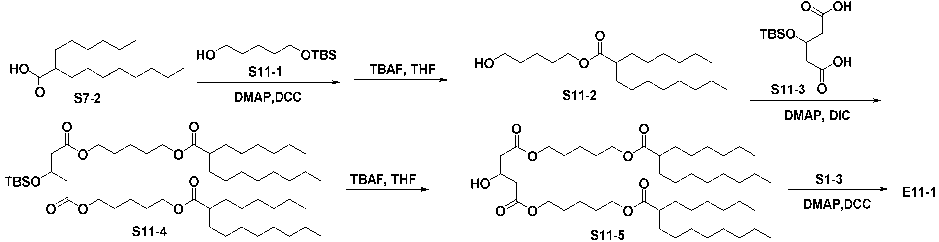

### Example 12: Cationic Lipid (E12-1)

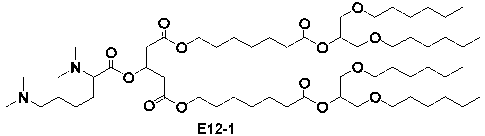

The preparation process is as follows:
Step a: under a nitrogen atmosphere, **DCC** (9.06 g, 44.0 mmol) was added to a round-bottom flask containing **S5-1** (6.24 g, 24.0 mmol), **S12-1** (5.20 g, 20.0 mmol), and **DMAP** (0.61 g, 5.0 mmol) dissolved in dichloromethane (200 mL), and the reaction was conducted for 16 h at room temperature. After completion of the reaction, the precipitate was removed by filtration, the filtrate was concentrated, and then the residue was purified by column chromatography to obtain **S12-2** (8.56 g).
Step b: the above product **S12-2** (8.05 g, 16.0 mmol) was dissolved in THF (100 mL), and **TBAF** (100 mL, 1 M) was added to the solution under nitrogen protection. The reaction was conducted overnight to remove TBS protection. After completion of the reaction, the solution was concentrated, extracted, and the organic layers were combined, dried with anhydrous magnesium sulfate, filtered and concentrated, and then the crude product was purified by column chromatography to obtain double-substituted diglyceride **S12-3** (5.43 g, 87.3%).
Step c: **S11-3** (0.79 g, 3.0 mmol) was dissolved in acetonitrile (50 mL), and DIC (2 mL, 12.0 mmol) was added dropwise to the solution, then **DMAP** (0.55 g, 4.5 mmol) and **S12-3** (4.67 g, 12.0 mmol) were added to the solution successively. The mixture was reacted with stirring overnight at room temperature. After completion of the reaction, the precipitate was removed by filtration, and the filtrate was concentrated. The residue was dissolved in DCM (50 mL), the resulting solution was washed with water (20 mL*2) and brine (30 mL*1) in turn, the organic phase was collected and dried with anhydrous magnesium sulfate, filtered and concentrated, and then purified by column chromatography to obtain **S12-4** (2.83 g, 93.8%).
Step d: the above product **S12-4** (2.00 g, 2.0 mmol) was dissolved in THF (20 mL), and **TBAF** (20 mL, 1 M) was added to the solution under nitrogen protection. The reaction was conducted overnight to remove TBS protection. After completion of the reaction, the solution was concentrated, extracted, and the organic layers were combined, dried with anhydrous magnesium sulfate, filtered and concentrated, and then the crude product was purified by column chromatography to obtain double-substituted diglyceride **S12-5** (1.54 g, 86.8%).
Step e: under a nitrogen atmosphere, **DCC** (0.45 g, 2.2 mmol) was added to a round-bottom flask containing **S12-5** (1.07 g, 1.2 mmol), **S1-3** (0.20 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E12-1** (0.90 g). ¹H NMR (400 MHz, CDCl₃) δ: 5.62-5.51 (m, 1H, >CHC(=O)OC*H*<), 5.16-5.02 (m, 2H, >C*H*(CH₂OCH₂)₂-), 4.04 (t, 4H, -C(=O)OC*H*₂-), 3.58-3.52 (m, 8H, >CH(C*H*₂OCH₂)₂-), 3.48-3.41 (m, 8H, >CH(CH₂OC*H*₂)₂-), 3.16 (t, 1H, -C*H*N(CH₃)₂), 2.80-2.67 (m, 6H; -C*H*₂N(CH₃)₂, 2H; >CH(C*H*₂C(=O)O)₂-, 4H), 2.57 (s, 6H, >CHN(C*H*₃)₂), 2.33-2.25 (m, 10H; -C*H*₂C(=O)OCH<, 4H; -CH₂N(C*H*₃)₂, 6H), 1.65-1.55 (m, 4H, -C*H*₂C*H*₂C*H*₂CH₂N(CH₃)₂), 1.43-1.20 (m, 50H; -CH₂C*H*₂CH₂-, -C*H*₂CH₃), 0.89 (t, 12H, -CH₂C*H*₃). MS (ESI): m/z=1072.81 ([M+H]⁺).

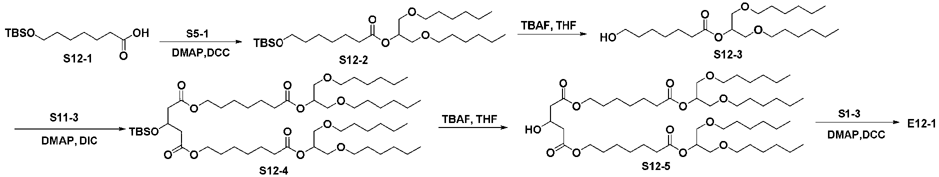

### Example 13: Cationic Lipid (E13-1)

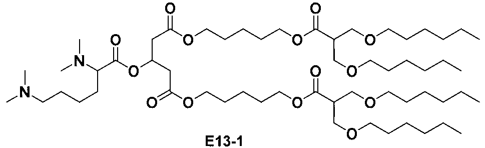

The preparation process is as follows:
Step a: bromohexane **(S13-2,** 9.90 g, 60.0 mmol), **KOH** (5.38 g, 96.0 mmol) and triethylenediamine **(DABCO,** 1.88 mg, 0.016 mmol) were added to a round-bottom flask containing 2,2-bis(hydroxymethyl)-acetic acid **(S13-1,** 1.44 g, 12.0 mmol) dissolved in toluene, and the mixture was refluxed at 135°C for 48 h. After completion of the reaction, the reactants were cooled to room temperature, and the pH of the solution was adjusted to 5 by adding dilute HCl. Then the solution was filtered, and the filtrate was concentrated, and then purified by column chromatography to obtain compound **S13-3** (2.38 g).
Step b: under a nitrogen atmosphere, **DCC** (7.25 g, 35.2 mmol) was added to a round-bottom flask containing **S13-3** (4.61 g, 16.0 mmol), **S11-1** (2.00 g, 19.2 mmol), and **DMAP** (0.49 g, 4.0 mmol) dissolved in dichloromethane (200 mL), and the reaction was conducted for 16 h at room temperature. After completion of the reaction, the precipitate was removed by filtration and the filtrate was concentrated. The residue was dissolved in tetrahydrofuran (30 mL), and 30 mL of **TBAF** tetrahydrofuran solution (1 M) was added to the solution. The reaction was conducted overnight to remove TBS protection. After completion of the reaction, the solution was concentrated, extracted, and the organic layers were combined, dried with anhydrous magnesium sulfate, filtered and concentrated, and then the crude product of S13-4 was purified by column chromatography to obtain compound **S13-4** (4.83 g).
Step c: **S11-3** (0.79 g, 3.0 mmol) was dissolved in acetonitrile (50 mL), and **DIC** (2 mL, 12.0 mmol) was added dropwise to the solution, then **DMAP** (0.55 g, 4.5 mmol) and **S13-4** (4.50 g, 12.0 mmol) were added to the solution successively. The mixture was reacted with stirring overnight at room temperature. After completion of the reaction, the precipitate was removed by filtration, and the filtrate was concentrated. The residue was dissolved in DCM (50 mL), the resulting solution was washed with water (20 mL*2) and brine (30 mL*1) in turn, the organic phase was collected and dried with anhydrous magnesium sulfate, filtered and concentrated, and then purified by column chromatography to obtain **S13-5** (2.75 g, 93.9%).
Step d: the above product **S13-5** (1.95 g, 2.0 mmol) was dissolved in THF (20 mL), and **TBAF** (20 mL, 1 M) was added to the solution under nitrogen protection. The reaction was conducted overnight to remove TBS protection. After completion of the reaction, the solution was concentrated, extracted, and the organic layers were combined, dried with anhydrous magnesium sulfate, filtered and concentrated, and then the crude product was purified by column chromatography to obtain double-substituted diglyceride **S13-6** (1.50 g, 87%).
Step e: under a nitrogen atmosphere, **DCC** (0.45 g, 2.2 mmol) was added to a round-bottom flask containing **S13-6** (1.03 g, 1.2 mmol), **S1-3** (0.20 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E13-1** (0.89 g). ¹H NMR (400 MHz, CDCl₃) δ: 5.62-5.50 (m, 1H, -C(=O)OC*H*<), 4.04 (t, 8H, -C(=O)OC*H*₂-), 3.64-3.57 (m, 8H, >C*H*(CH₂OCH₂)₂-), 3.48-3.40 (m, 8H, >C*H*(CH₂OCH₂)₂-), 3.17 (t, 1H, -C*H*N(CH₃)₂), 2.95-2.90 (m, 2H, >C*H*(CH₂OCH₂)₂-), 2.80-2.66 (m, 6H; -C*H*₂N(CH₃)₂, 2H; >CH(C*H*₂C(=O)O)₂-, 4H), 2.56 (s, 6H, >CHN(C*H*₃)₂), 2.27 (s, 6H, -CH₂N(C*H*₃)₂), 1.68-1.55 (m, 4H, -C*H*₂C*H*₂CH₂CH₂N(CH₃)₂), 1.41-1.21 (m, 46H; -CH₂C*H*₂CH₂-, -C*H*₂CH₃), 0.91 (t, 12H, -CH₂C*H*₃). MS (ESI): m/z=1044.78 ([M+H]⁺).

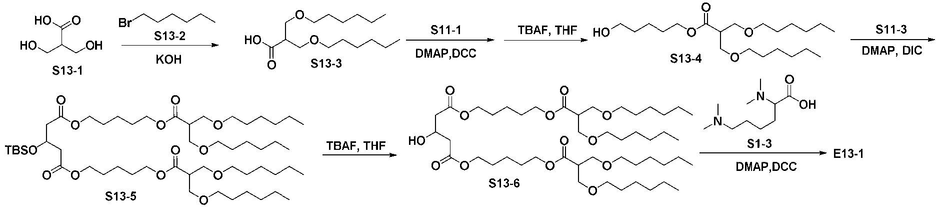

### Example 14: Cationic Lipid (E14-1)

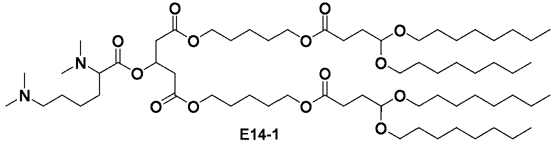

The preparation process is as follows:
Step a: **S11-3** (0.79 g, 3.0 mmol) was dissolved in acetonitrile (50 mL), and **DIC** (2 mL, 12.0 mmol) was added dropwise to the solution, then **DMAP** (0.55 g, 4.5 mmol) and **S14-1** (5.17 g, 12.0 mmol, **S14-1** was obtained by the reaction between **S3-5** and **S11-1,** and the specific experimental procedure could refer to **Step a of Example 11)** were added to the solution successively. The mixture was reacted with stirring overnight at room temperature. After completion of the reaction, the precipitate was removed by filtration, and the filtrate was concentrated. The residue was dissolved in DCM (50 mL), the resulting solution was washed with water (20 mL*2) and brine (30 mL*1) in turn, the organic phase was collected and dried with anhydrous magnesium sulfate, filtered and concentrated, and then purified by column chromatography to obtain **S14-2** (3.07 g, 94.1%).
Step b: under nitrogen protection, the above product **S14-2** (2.18 g, 2.0 mmol) was dissolved in THF (20 mL), and **TBAF** (20 mL, 1 M) was added to the solution. The reaction was conducted overnight to remove TBS protection. After completion of the reaction, the solution was concentrated, extracted, and the organic layers were combined, dried with anhydrous magnesium sulfate, filtered and concentrated, and then the crude product was purified by column chromatography to obtain double-substituted diglyceride **S14-3** (1.67 g, 85.9%).
Step c: under a nitrogen atmosphere, **DCC** (0.45 g, 2.2 mmol) was added to a round-bottom flask containing **S14-3** (1.17 g, 1.2 mmol), **S1-3** (0.20 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E14-1** (0.98 g). ¹H NMR (400 MHz, CDCl₃) δ: 5.62-5.52 (m, 1H, -C(=O)OC*H*<), 4.63 (t, 2H, >C*H*(OCH₂)₂-), 4.04 (t, 8H, -C(=O)OC*H*₂-), 3.52-3.36 (m, 8H, >CH(OC*H*₂)₂-), 3.17 (t, 1H, -C*H*N(CH₃)₂), 2.80-2.66 (m, 6H; -C*H*₂N(CH₃)₂, 2H; >CH(C*H*₂C(=O)O)₂-, 4H), 2.56 (s, 6H, >CHN(C*H*₃)₂), 2.32-2.30 (m, 4H, -OC(=O)C*H*₂CH₂-), 2.23 (s, 6H, -CH₂N(C*H*₃)₂), 2.00-1.82 (m, 4H, -C*H*₂CH(OCH₂)₂-), 1.66-1.55 (m, 4H, -C*H*₂C*H*₂CH₂CH₂N(CH₃)₂), 1.41-1.21 (m, 62H; -CH₂C*H*₂CH₂-, -C*H*₂CH₃), 0.89 (t, 12H, -CH₂C*H*₃). MS (ESI): m/z=1156.91 ([M+H]⁺).

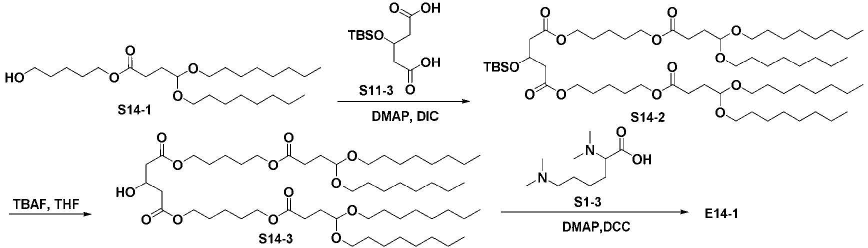

### Example 15: Cationic Lipid (E15-1)

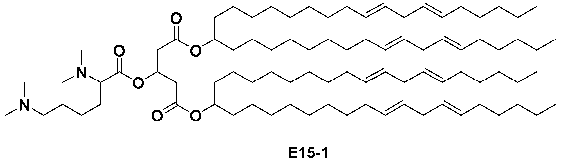

The preparation process is as follows:
Step a: **S11-3** (0.79 g, 3.0 mmol) was dissolved in acetonitrile (50 mL), and **DIC** (2 mL, 12.0 mmol) was added dropwise to the solution, then **DMAP** (0.55 g, 4.5 mmol) and **S1-2** (6.35 g, 12.0 mmol) were added to the solution successively. The mixture was reacted with stirring overnight at room temperature. After completion of the reaction, the precipitate was removed by filtration, and the filtrate was concentrated. The residue was dissolved in DCM (50 mL), the resulting solution was washed with water (20 mL*2) and brine (30 mL*1) in turn, the organic phase was collected and dried with anhydrous magnesium sulfate, filtered and concentrated, and then purified by column chromatography to obtain **S15-1** (3.62 g, 94.1%).
Step b: under nitrogen protection, the above product **S15-1** (2.57 g, 2.0 mmol) was dissolved in THF (30 mL), and **TBAF** (30 mL, 1 M) was added to the solution. The reaction was conducted overnight to remove TBS protection. After completion of the reaction, the solution was concentrated, extracted, and the organic layers were combined, dried with anhydrous magnesium sulfate, filtered and concentrated, and then the crude product was purified by column chromatography to obtain double-substituted diglyceride **S15-2** (2.02 g, 86.4%).
Step c: under a nitrogen atmosphere, **DCC** (0.45 g, 2.2 mmol) was added to a round-bottom flask containing **S15-1** (1.40 g, 1.2 mmol), **S1-3** (0.20 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E15-1** (1.15 g). ¹H NMR (400 MHz, CDCl₃) δ: 5.62-5.51 (m, 1H, >CHC(=O)OC*H*<), 5.45-5.30 (m, 16H, -C*H*=C*H*-), 4.87 (t, 2H, >C*H*(CH₂)₂-), 3.16 (t, 1H, -C*H*N(CH₃)₂), 2.80-2.66 (m, 14H; -C*H*₂N(CH₃)₂, 2H; >CH(C*H*₂C(=O)O)₂-, 4H; -CH=CHC*H*₂CH=CH-, 8H), 2.57 (s, 6H, >CHN(C*H*₃)₂), 2.23 (s, 6H, -CH₂N(C*H*₃)₂), 2.09-1.99 (m, 16H, -CH₂C*H*₂CH=CH-), 1.66-1.55 (m, 4H, -C*H*₂C*H*₂CH₂CH₂N(CH₃)₂), 1.41-1.21 (m, 82H; -CH₂C*H*₂CH₂-, -C*H*₂CH₃), 0.89 (t, 12H, -CH₂C*H*₃). MS (ESI): m/z=1353.23 ([M+H]⁺).

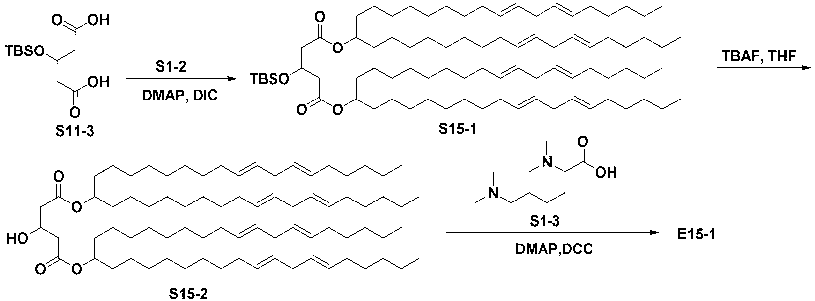

### Example 16: Cationic Lipid (E16-1)

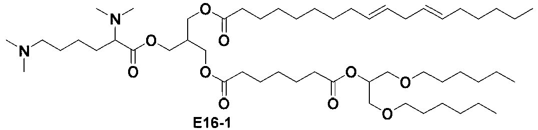

The preparation process is as follows:
Step a: 2-(hydroxymethyl)-1,3-diol **(S16-1,** 0.53 g, 5.0 mmol), **S2-2** (1.40 g, 5.0 mmol) and **DMAP** (0.10 g, 0.8 mmol) were dissolved in anhydrous THF (20 mL) successively under a nitrogen atmosphere, and then **EDCI** (0.10 g, 0.5 mmol) was quickly added to the above mixture under ice bath conditions. When the EDCI was completely dissolved, the reaction solution was warmed to room temperature and the reaction continued for 15 h. After completion of the reaction, the solvent was concentrated, and the residue was dissolved in ethyl acetate, washed with water (10 mL*3), the organic phase was collected and dried with anhydrous magnesium sulfate, filtered and concentrated, and then purified by column chromatography to obtain **S16-2** (1.33 g).
Step b: **S16-2** (0.74 g, 2.0 mmol), **S5-3** (0.80 g, 2.0 mmol) and **DMAP** (0.04 g, 0.3 mmol) were dissolved in anhydrous THF (20 mL) successively under a nitrogen atmosphere, and then **EDCI** (0.04 g, 0.2 mmol) was quickly added to the above mixture under ice bath conditions. When the EDCI was completely dissolved, the reaction solution was warmed to room temperature and the reaction continued for 15 h. After completion of the reaction, the solvent was concentrated, and the residue was dissolved in ethyl acetate, washed with water (10 mL*3), the organic phase was collected and dried with anhydrous magnesium sulfate, filtered and concentrated, and then purified by column chromatography to obtain **S16-3** (1.23 g).
Step c: under a nitrogen atmosphere, **DCC** (0.45 g, 2.2 mmol) was added to a round-bottom flask containing **S16-3** (0.90 g, 1.2 mmol), **S1-3** (0.20 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E16-1** (0.80 g). ¹H NMR (400 MHz, CDCl₃) δ: 5.44-5.29 (m, 4H, -C*H*=C*H*-), 5.16-5.02 (m, 1H, >C*H*(CH₂OCH₂)₂-), 4.15 (d, 6H, >CH(C*H*₂OC(=O))₃-), 3.58-3.52 (m, 4H, >C*H*(CH₂OCH₂)₂-), 3.48-3.40 (m, 4H, >C*H*(CH₂OCH₂)₂-), 3.17 (t, 1H, -C*H*N(CH₃)₂), 2.80-2.66 (m, 4H; -C*H*₂N(CH₃)₂, 2H; -CH=CHC*H*₂CH=CH-, 2H), 2.56 (s, 6H, >CHN(C*H*₃)₂), 2.43-2.37 (m, 1H, >C*H*(CH₂OC(=O))₃-), 2.34-2.25 (m, 12H; -C*H*₂C(=O)O-, 6H; -CH₂N(C*H*₃)₂, 6H), 2.10-1.98 (m, 4H, -CH₂C*H*₂CH=CH-), 1.66-1.55 (m, 4H, -C*H*₂C*H*₂CH₂CH₂N(CH₃)₂), 1.40-1.20 (m, 40H; -CH₂C*H*₂CH₂-, -C*H*₂CH₃), 0.88 (t, 9H, -CH₂C*H*₃). MS (ESI): m/z=936.74 ([M+H]⁺).

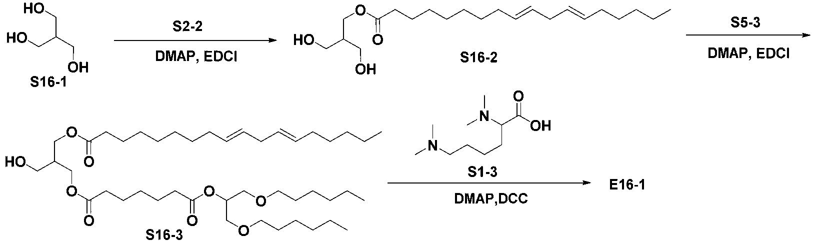

### Example 17.1: Cationic Lipid (E17-1)

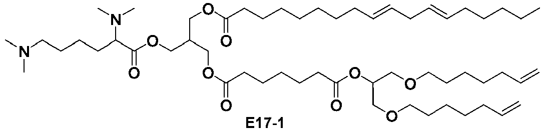

The preparation process is as follows:
Step a: under a nitrogen atmosphere, **DCC** (2.72 g, 13.2 mmol) was added to a round-bottom flask containing **S5-2** (1.30 g, 6.0 mmol), **S17-1** (2.04 g, 7.2 mmol, ), and **DMAP** (0.18 g, 1.5 mmol) dissolved in dichloromethane (50 mL), and the reaction was conducted for 16 h at room temperature. After completion of the reaction, the precipitate was removed by filtration and the filtrate was concentrated to obtain a crude product. In a dry and clean round-bottom flask, a solution of trifluoroacetic acid/dichloromethane (1:2, v/v) was prepared, and the dichloromethane solution of the above crude product was added dropwise under ice bath condition. The reaction was conducted at room temperature for 2 h. After completion of the reaction, the reaction solution was concentrated, and purified water was added, followed by extraction with dichloromethane. The extract was dried with anhydrous magnesium sulfate, filtered, the filtrate was concentrated, and then the crude product was recrystallized to obtain compound **S17-2** (2.08 g).
Step b: **S16-2** (0.74 g, 2.0 mmol), **S17-2** (0.85 g, 2.0 mmol) and **DMAP** (0.04 g, 0.3 mmol) were dissolved in anhydrous THF (150 mL) successively under a nitrogen atmosphere, and then **EDCI** (0.04 g, 0.2 mmol) was quickly added to the above mixture under ice bath conditions. When the EDCI was completely dissolved, the reaction solution was warmed to room temperature and the reaction continued for 15 h. After completion of the reaction, the solvent was concentrated, the residue was dissolved in ethyl acetate, washed with water (10 mL*3), the organic phase was collected and dried with anhydrous magnesium sulfate, filtered and concentrated, and then purified by column chromatography to obtain **S17-3** (1.25 g).
Step c: under a nitrogen atmosphere, **DCC** (0.45 g, 2.2 mmol) was added to a round-bottom flask containing **S17-3** (0.93 g, 1.2 mmol), **S1-3** (0.20 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E17-1** (0.81 g). ¹H NMR (400 MHz, CDCl₃) δ: 5.85-5.75 (m, 2H, -CH₂C*H*=CH₂), 5.45-5.29 (m, 4H, -C*H*=C*H*-), 5.17-4.91 (m, 5H, >C*H*(CH₂OCH₂)₂-, 1H; -CH₂CH=C*H*₂, 4H), 4.14 (d, 6H, >CH(C*H*₂OC(=O))₃-), 3.58-3.52 (m, 4H, >C*H*(CH₂OCH₂)₂-), 3.48-3.40 (m, 4H, >C*H*(CH₂OCH₂)₂-), 3.17 (t, 1H, -C*H*N(CH₃)₂), 2.80-2.66 (m, 4H; -C*H*₂N(CH₃)₂, 2H; -CH=CHC*H*₂CH=CH-, 2H), 2.56 (s, 6H, >CHN(C*H*₃)₂), 2.43-2.37 (m, 1H, >C*H*(CH₂OC(=O))₃-), 2.34-2.25 (m, 12H; -C*H*₂C(=O)O-, 6H; -CH₂N(C*H*₃)₂, 6H), 2.10-1.98 (m, 4H, -CH₂C*H*₂CH=CH-), 1.66-1.55 (m, 4H, -C*H*₂C*H*₂CH₂CH₂N(CH₃)₂), 1.40-1.21 (m, 36H; -CH₂C*H*₂CH₂-, -C*H*₂CH₃), 0.89 (t, 3H, -CH₂C*H*₃). MS (ESI): m/z=960.74 ([M+H]⁺).

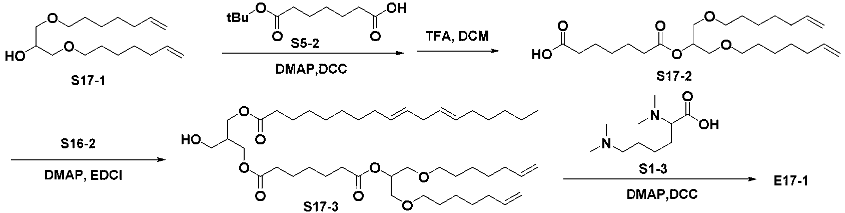

### Example 17.2: Cationic Lipid (E17-2)

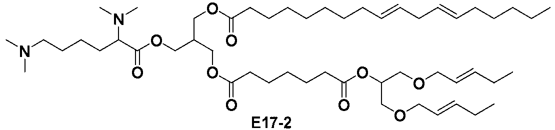

The preparation process is as follows:
Step a: under a nitrogen atmosphere, **DCC** (2.72 g, 13.2 mmol) was added to a round-bottom flask containing **S5-2** (1.30 g, 6.0 mmol), **S17-4** (1.64 g, 7.2 mmol, **S17-4** was obtained by the reaction between a glycerol with a TBS-protected hydroxyl group and 1-bromo-2-pentene, followed by deprotection), and **DMAP** (0.18 g, 1.5 mmol) dissolved in dichloromethane (50 mL), and the reaction was conducted for 16 h at room temperature. After completion of the reaction, the precipitate was removed by filtration and the filtrate was concentrated to obtain a crude product. In a dry and clean round-bottom flask, a solution of trifluoroacetic acid/dichloromethane (1:2, v/v) was prepared, and the dichloromethane solution of the above crude product was added dropwise under ice bath condition, reacted at room temperature for 2 h. After completion of the reaction, the reaction solution was concentrated, and purified water was added, followed by extraction with dichloromethane. The extract was dried with anhydrous magnesium sulfate, filtered, the filtrate was concentrated, and then the crude product was recrystallized to obtain compound **S17-5** (1.80 g).
Step b: **S16-2** (0.74 g, 2.0 mmol), **S17-5** (0.74 g, 2.0 mmol) and **DMAP** (0.04 g, 0.3 mmol) were dissolved in anhydrous THF (20 mL) successively under a nitrogen atmosphere, and then **EDCI** (0.04 g, 0.2 mmol) was quickly added to the above mixture under ice bath conditions. When the EDCI was completely dissolved, the reaction solution was warmed to room temperature and the reaction continued for 15 h. After completion of the reaction, the solvent was concentrated, the residue was dissolved in ethyl acetate, washed with water (10 mL*3), and the organic phase was collected and dried with anhydrous magnesium sulfate, filtered and concentrated, and then purified by column chromatography to obtain **S17-6** (1.17 g).
Step c: under a nitrogen atmosphere, **DCC** (0.45 g, 2.2 mmol) was added to a round-bottom flask containing **S17-6** (0.87 g, 1.2 mmol), **S1-3** (0.20 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E17-2** (0.76 g). ¹H NMR (400 MHz, CDCl₃) δ: 5.68-5.47 (m, 4H, -OCH₂C*H*=C*H*-), 5.45-5.29 (m, 4H, -CH₂CH₂C*H*=C*H*-), 5.16-5.02 (m, 1H, >C*H*(CH₂OCH₂)₂-), 4.14 (d, 6H, >CH(C*H*₂OC(=O))₃-), 3.84 (d, 4H, -OC*H*₂CH=CH-), 3.58-3.52 (m, 4H, >CH(C*H*₂OCH₂)₂-), 3.17 (t, 1H, -C*H*N(CH₃)₂), 2.80-2.66 (m, 4H; -C*H*₂N(CH₃)₂, 2H; -CH=CHC*H*₂CH=CH-, 2H), 2.56 (s, 6H, >CHN(C*H*₃)₂), 2.43-2.37 (m, 1H, >C*H*(CH₂OC(=O))₃-), 2.35-2.25 (m, 12H; -C*H*₂C(=O)O-, 6H; -CH₂N(C*H*₃)₂, 6H), 2.11-1.82 (m, 8H; -CH=CHC*H*₂CH₃, 4H; -CH₂C*H*₂CH=CH-, 4H), 1.66-1.55 (m, 4H, -C*H*₂CH₂C*H*₂CH₂N(CH₃)₂), 1.39-1.21 (m, 24H; -CH₂C*H*₂CH₂-, -C*H*₂CH₃), 0.85 (t, 9H, -CH₂C*H*₃). MS (ESI): m/z=904.68 ([M+H]⁺).

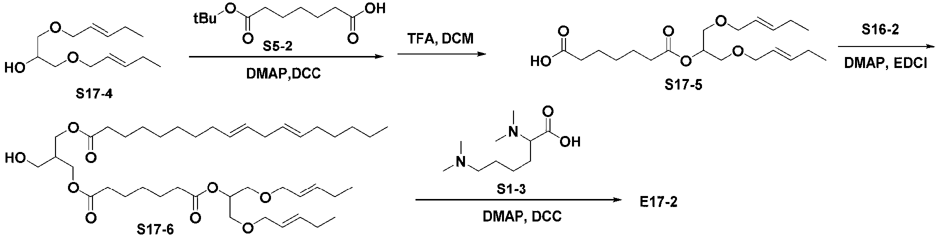

### Example 18.1: Cationic Lipid (E18-1)

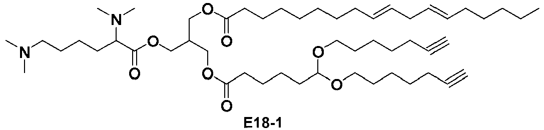

The preparation process is as follows:
Step a: tert-butyl 6-hydroxyhexanoate (**S18-1,** 9.40 g, 50 mmol) was dissolved in 250 mL of dichloromethane solution, and pyridine chlorochromate salt (**PCC**, 16.13 g, 75.0 mmol) was added to the solution. After stirring at 15 °C for at least 2 h, the solution was filtered, concentrated under reduced pressure, and then purified by column chromatography to obtain **S18-2** (5.95 g).
Step b: the above compounds **S18-2** (5.58 g, 30.0 mmol) and 6-heptanylol (**S18-3,** 8.40 g, 75.0 mmol) were dissolved in 250 mL of dichloromethane solution, then p-toluene sulfonic acid monohydrate (**TsOH·H₂O,** 1.14 g, 6.0 mmol) and **anhydrous sodium sulfate** (10.65 g, 75.0 mmol) were added to the solution. After stirring at 15°C for at least 24 h, the solution was filtered, the crude product was concentrated under reduced pressure, and then purified by column chromatography to obtain compound **S18-4** (2.59 g).
Step c: Removal of the tBu protecting group. In a dry and clean round-bottom flask, a solution of trifluoroacetic acid/dichloromethane (1:2, v/v) was prepared, and a dichloromethane solution of **S18-4** (1.97 g, 5.0 mmol) was slowly added dropwise under ice bath conditions. The reaction was conducted for 2 h at room temperature. After completion of the reaction, the reaction solution was concentrated, and purified water was added, followed by extraction with dichloromethane. The extract was dried with anhydrous magnesium sulfate, filtered, and then the filtrate was concentrated, recrystallized to obtain compound **S18-5** (1.58 g, 93.8%).
Step d: under a nitrogen atmosphere, **S16-2** (0.74 g, 2.0 mmol), **S18-5** (0.67 g, 2.0 mmol) and **DMAP** (0.04 g, 0.3 mmol) were dissolved in anhydrous THF (20 mL) successively, and **EDCI** (0.04 g, 0.2 mmol) was quickly added to the above mixture under ice bath conditions. When the EDCI was completely dissolved, the reaction solution was warmed to room temperature and the reaction continued for 15 h. After completion of the reaction, the solvent was concentrated, the residue was dissolved in ethyl acetate, washed with water (10 mL*3), the organic phase was collected and dried with anhydrous magnesium sulfate, filtered and concentrated, and then purified by column chromatography to obtain **S18-6** (1.09 g).
Step e: under a nitrogen atmosphere, **DCC** (0.45 g, 2.2 mmol) was added to a round-bottom flask containing **S18-6** (0.82 g, 1.2 mmol), **S1-3** (0.20 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E18-1** (0.73 g). ¹H NMR (400 MHz, CDCl₃) δ: 5.47-5.28 (m, 4H, *-CH=CH-),* 4.64 (t, 1H, >C*H*(OCH₂)₂-), 4.14 (d, 6H, >CH(C*H*₂OC(=O))₃-), 3.52-3.36 (m, 4H, >CH(OC*H*₂)₂-), 3.17 (t, 1H, -C*H*N(CH₃)₂), 2.80-2.66 (m, 4H; -C*H*₂N(CH₃)₂, 2H; -CH=CHC*H*₂CH=CH-, 2H), 2.57 (s, 6H, >CHN(C*H*₃)₂), 2.43-2.37 (m, 1H, >C*H*(CH₂OC(=O))₃-), 2.34-2.25 (m, 10H; -C*H*₂C(=O)O-, 4H; -CH₂N(C*H*₃)₂, 6H), 2.21 (t, 4H, -C*H*₂C ≡ CH), 2.10-1.82 (m, 8H; -CH₂C ≡ C*H*, 2H; -CH₂C*H*₂CH=CH-, 4H; -C*H*₂CH(OCH₂)₂-, 2H), 1.66-1.55 (m, 4H, -C*H*₂CH₂C*H*₂CH₂N(CH₃)₂), 1.39-1.21 (m, 34H; -CH₂C*H*₂CH₂-, -C*H*₂CH₃), 0.88 (t, 3H, -CH₂C*H*₃). MS (ESI): m/z=870.67 ([M+H]⁺).

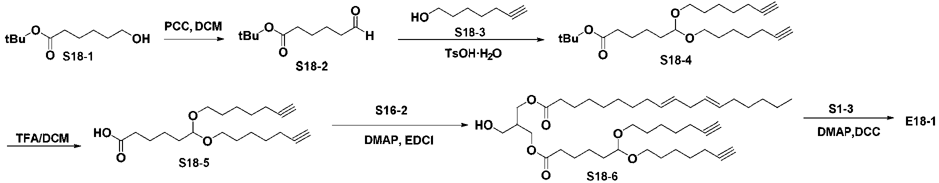

### Example 19: Cationic Lipid (E19-1)

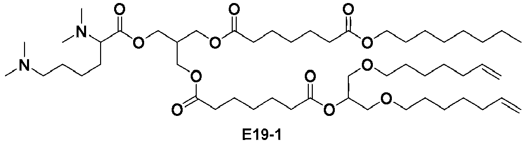

The preparation process is as follows:
Step a: under a nitrogen atmosphere, **DCC** (3.63 g, 17.6 mmol) was added to a round-bottom flask containing **S5-2** (1.73 g, 8.0 mmol), **S3-1** (1.25 g, 9.6 mmol), and **DMAP** (0.24 g, 2.0 mmol) dissolved in dichloromethane (60 mL), and the reaction was conducted for 16 h at room temperature. After completion of the reaction, the precipitate was removed by filtration and the filtrate was concentrated to obtain a crude product. In a dry and clean round-bottom flask, a solution of trifluoroacetic acid/dichloromethane (1:2, v/v) was prepared, and the dichloromethane solution of the above crude product was added dropwise under ice bath condition. The reaction was conducted at room temperature for 2 h. After completion of the reaction, the reaction solution was concentrated, and purified water was added, followed by extraction with dichloromethane. The extract was dried with anhydrous magnesium sulfate, filtered, the filtrate was concentrated, and then the crude product was recrystallized to obtain compound **S19-1** (1.77 g).
Step b: **S16-1** (0.53 g, 5.0 mmol), **S19-1** (1.36 g, 5.0 mmol) and **DMAP** (0.10 g, 0.8 mmol) were dissolved in anhydrous THF (30 mL) successively under a nitrogen atmosphere, and then **EDCI** (0.10 g, 0.5 mmol) was quickly added to the above mixture under ice bath conditions. When the EDCI was completely dissolved, the reaction solution was warmed to room temperature and the reaction continued for 15 h. After completion of the reaction, the solvent was concentrated, the residue was dissolved in ethyl acetate, washed with water (10 mL*3), the organic phase was collected and dried with anhydrous magnesium sulfate, filtered and concentrated, and then purified by column chromatography to obtain **S19-2** (1.29 g).
Step c: under a nitrogen atmosphere, **S19-2** (0.72 g, 2.0 mmol), **S17-2** (0.85 g, 2.0 mmol) and **DMAP** (0.04 g, 0.3 mmol) were dissolved in anhydrous THF (20 mL) successively, and then **EDCI** (0.04 g, 0.2 mmol) was quickly added to the above mixture under ice bath conditions. When the EDCI was completely dissolved, the reaction solution was warmed to room temperature and the reaction continued for 15 h. After completion of the reaction, the solvent was concentrated, the residue was dissolved in ethyl acetate, washed with water (10 mL*3), the organic phase was collected and dried with anhydrous magnesium sulfate, filtered and concentrated, and then purified by column chromatography to obtain **S19-3** (1.26 g).
Step d: under a nitrogen atmosphere, **DCC** (0.45 g, 2.2 mmol) was added to a round-bottom flask containing **S19-3** (0.92 g, 1.2 mmol), **S1-3** (0.20 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E19-1** (0.80 g). ¹H NMR (400 MHz, CDCl₃) δ: 5.86-5.75 (m, 2H, -CH₂C*H*=CH₂), 5.17-4.92 (m, 5H, >C*H*(CH₂OCH₂)₂-, 1H; -CH₂CH=C*H*₂, 4H), 4.15 (d, 6H, >CH(C*H*₂OC(=O))₃-), 4.04 (t, 2H, -C(=O)OC*H*₂-), 3.58-3.51 (m, 4H, >CH(C*H*₂OCH₂)₂-), 3.48-3.40 (m, 4H, >CH(CH₂OC*H*₂)₂-), 3.15 (t, 1H, -C*H*N(CH₃)₂), 2.81-2.66 (m, 2H, -C*H*₂N(CH₃)₂), 2.55 (s, 6H, >CHN(C*H*₃)₂), 2.43-2.37 (m, 1H, >C*H*(CH₂OC(=O))₃-), 2.34-2.25 (m, 14H; -C*H*₂C(=O)O-, 8H; -CH₂N(C*H*₃)₂, 6H), 2.10-1.81 (m, 4H, -CH₂C*H*₂CH=CH-), 1.66-1.54 (m, 4H, -C*H*₂CH₂C*H*₂CH₂N(CH₃)₂), 1.41-1.22 (m, 38H; -CH₂C*H*₂CH₂-, -C*H*₂CH₃), 0.86 (t, 3H, -CH₂C*H*₃). MS (ESI): m/z=952.70 ([M+H]⁺).

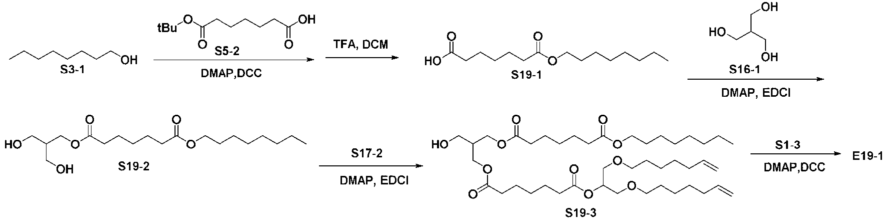

### Example 20: Cationic Lipid (E20-1)

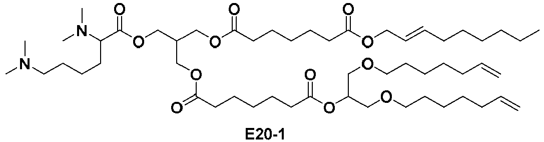

The preparation process is as follows:
Step a: under a nitrogen atmosphere, **DCC** (3.63 g, 17.6 mmol) was added to a round-bottom flask containing **S5-2** (1.73 g, 8.0 mmol), 2-nonen-1-ol (**S20-1,** 1.36 g, 9.6 mmol), and **DMAP** (0.24 g, 2.0 mmol) dissolved in dichloromethane (60 mL), and the reaction was conducted for 16 h at room temperature. After completion of the reaction, the precipitate was removed by filtration and the filtrate was concentrated to obtain a crude product. In a dry and clean round-bottom flask, a solution of trifluoroacetic acid/dichloromethane (1:2, v/v) was prepared, and the dichloromethane solution of the above crude product was added dropwise under ice bath condition. The reaction was conducted at room temperature for 2 h. After completion of the reaction, the reaction solution was concentrated, and purified water was added, followed by extraction with dichloromethane. The extract was dried with anhydrous magnesium sulfate, filtered, the filtrate was concentrated, and then the crude product was recrystallized to obtain compound **S20-2** (1.85 g).
Step b: under a nitrogen atmosphere, **S16-1** (0.53 g, 5.0 mmol), **S20-2** (1.42 g, 5.0 mmol) and **DMAP** (0.10 g, 0.8 mmol) were dissolved in anhydrous THF (30 mL) successively, and then **EDCI** (0.10 g, 0.5 mmol) was quickly added to the above mixture under ice bath conditions. When the EDCI was completely dissolved, the reaction solution was warmed to room temperature and the reaction continued for 15 h. After completion of the reaction, the solvent was concentrated, the residue was dissolved in ethyl acetate, washed with water (10 mL*3), the organic phase was collected and dried with anhydrous magnesium sulfate, filtered and concentrated, and then purified by column chromatography to obtain **S20-3** (1.35 g).
Step c: under a nitrogen atmosphere, **S20-3** (0.75 g, 2.0 mmol), **S17-2** (0.85 g, 2.0 mmol) and **DMAP** (0.04 g, 0.3 mmol) were dissolved in anhydrous THF (30 mL) successively, and then **EDCI** (0.04 g, 0.2 mmol) was quickly added to the above mixture under ice bath conditions. When the EDCI was completely dissolved, the reaction solution was warmed to room temperature and the reaction continued for 15 h. After completion of the reaction, the solvent was concentrated, the residue was dissolved in ethyl acetate, washed with water (10 mL*3), the organic phase was collected and dried with anhydrous magnesium sulfate, filtered and concentrated, and then purified by column chromatography to obtain **S20-4** (1.28 g).
Step d: under a nitrogen atmosphere, **DCC** (0.45 g, 2.2 mmol) was added to a round-bottom flask containing **S20-4** (0.94 g, 1.2 mmol), **S1-3** (0.20 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, and the filtrate was concentrated, and then the residue was purified by column chromatography to obtain the cationic lipid **E20-1** (0.82 g). ¹H NMR (400 MHz, CDCl₃) δ: 5.85-5.68 (m, 4H; -CH₂C*H*=C*H*₂, 2H; -C*H*=C*H*-, 2H), 5.17-4.91 (m, 5H, >C*H*(CH₂OCH₂)₂-, 1H; -CH₂CH=C*H*₂, 4H), 4.57 (dd, 2H, -C(=O)OC*H*₂CH=CH-), 4.15 (d, 6H, >CH(C*H*₂OC(=O))₃-), 3.58-3.51 (m, 4H, >CH(C*H*₂OC*H*₂)₂-), 3.47-3.41 (m, 4H, >CH(CH₂OC*H*₂)₂-), 3.15 (t, 1H, -C*H*N(CH₃)₂), 2.81-2.66 (m, 2H, -C*H*₂N(CH₃)₂), 2.55 (s, 6H, >CHN(C*H*₃)₂), 2.43-2.37 (m, 1H, >C*H*(CH₂OC(=O))₃-), 2.33-2.25 (m, 14H; -C*H*₂C(=O)O-, 8H; -CH₂N(C*H*₃)₂, 6H), 2.10-1.82 (m, 6H; -CH₂C*H*₂CH=CH-, 4H, -C*H*₂CH=CH-, 2H), 1.66-1.54 (m, 4H, -C*H*₂CH₂C*H*₂CH₂N(CH₃)₂), 1.41-1.22 (m, 34H; -CH₂C*H*₂CH₂-, -C*H*₂CH₃), 0.87 (t, 3H, -CH₂C*H*₃). MS (ESI): m/z=964.70 ([M+H]⁺).

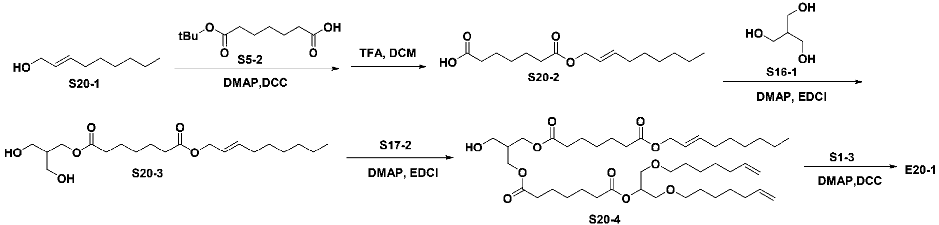

### Example 21: Cationic Lipid (E21-1)

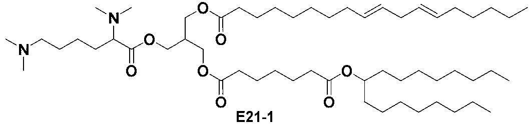

The preparation process is as follows:
Step a: under a nitrogen atmosphere, **S16-2** (0.74 g, 2.0 mmol), S9-2 (0.80 g, 2.0 mmol) and **DMAP** (0.04 g, 0.3 mmol) were dissolved in anhydrous THF (30 mL) successively, and then **EDCI** (0.04 g, 0.2 mmol) was quickly added to the above mixture under ice bath conditions. When the EDCI was completely dissolved, the reaction solution was warmed to room temperature and the reaction continued for 15 h. After completion of the reaction, the solvent was concentrated, and the residue was dissolved in ethyl acetate, washed with water (10 mL*3), and then the organic phase was collected and dried with anhydrous magnesium sulfate, filtered and concentrated, and purified by column chromatography to obtain **S21-1** (1.23 g).
Step b: under a nitrogen atmosphere, **DCC** (0.45 g, 2.2 mmol) was added to a round-bottom flask containing **S21-1** (0.90 g, 1.2 mmol), **S1-3** (0.20 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E21-1** (0.79 g). ¹H NMR (400 MHz, CDCl₃) δ: 5.45-5.29 (m, 4H, *-CH=CH-),* 4.85 (t, 1H, >C*H*(CH₂CH₂)₂-), 4.14 (d, 6H, >CH(C*H*₂OC(=O))₃-), 3.17 (t, 1H, -C*H*N(CH₃)₂), 2.80-2.66 (m, 4H; -C*H*₂N(CH₃)₂, 2H; -CH=CHC*H*₂CH=CH-, 2H), 2.56 (s, 6H, >CHN(C*H*₃)₂), 2.43-2.37 (m, 1H, >C*H*(CH₂OC(=O))₃-), 2.34-2.25 (m, 12H; -C*H*₂C(=O)O-, 6H; -CH₂N(C*H*₃)₂, 6H), 2.10-1.98 (m, 4H, -CH₂C*H*₂CH=CH-), 1.66-1.55 (m, 4H, -C*H*₂CH₂C*H*₂CH₂N(CH₃)₂), 1.40-1.21 (m, 52H; -CH₂C*H*₂CH₂-, -C*H*₂CH₃), 0.89 (t, 9H, -CH₂C*H*₃). MS (ESI): m/z=932.78 ([M+H]⁺).

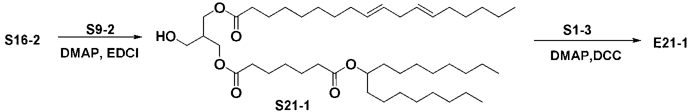

### Example 22: Cationic Lipid (E22-1)

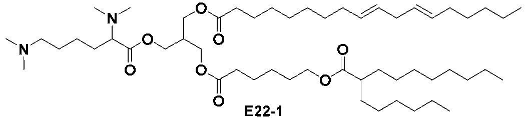

The preparation process is as follows:
Step a: under a nitrogen atmosphere, **S16-2** (0.74 g, 2.0 mmol), **S7-4** (0.74 g, 2.0 mmol) and **DMAP** (0.04 g, 0.3 mmol) were dissolved in anhydrous THF (30 mL) successively, and then **EDCI** (0.04 g, 0.2 mmol) was quickly added to the above mixture under ice bath conditions. When the EDCI was completely dissolved, the reaction solution was warmed to room temperature and the reaction continued for 15 h. After completion of the reaction, the solvent was concentrated, and the residue was dissolved in ethyl acetate, washed with water (10 mL*3), and the organic phase was collected and dried with anhydrous magnesium sulfate, filtered and concentrated, and then purified by column chromatography to obtain **S22-1** (1.18 g).
Step b: under a nitrogen atmosphere, **DCC** (0.45 g, 2.2 mmol) was added to a round-bottom flask containing **S22-1** (0.87 g, 1.2 mmol), **S1-3** (0.20 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E22-1** (0.76 g). ¹H NMR (400 MHz, CDCl₃) δ: 5.44-5.29 (m, 4H, *-CH=CH-),* 4.14 (d, 6H, >CH(C*H*₂OC(=O))₃-), 4.04 (t, 2H, >CHC(=O)OC*H*₂-), 3.17 (t, 1H, -C*H*N(CH₃)₂), 2.80-2.66 (m, 4H; -C*H*₂N(CH₃)₂, 2H; -CH=CHC*H*₂CH=CH-, 2H), 2.56 (s, 6H, >CHN(C*H*₃)₂), 2.43-2.37 (m, 1H, >C*H*(CH₂OC(=O))₃-), 2.34-2.25 (m, 11H; -C*H*₂C(=O)O-, 4H; -CH₂N(C*H*₃)₂, 6H; -OC(=O)C*H*(CH₂)₂, 1H), 2.10-1.98 (m, 4H, -CH₂C*H*₂CH=CH-), 1.66-1.55 (m, 4H, -C*H*₂CH₂C*H*₂CH₂N(CH₃)₂), 1.40-1.21 (m, 48H; -CH₂C*H*₂CH₂-, -C*H*₂CH₃), 0.88 (t, 9H, -CH₂C*H*₃). MS (ESI): m/z=904.75 ([M+H]⁺).

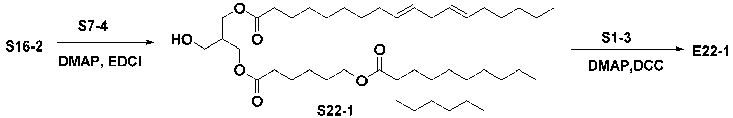

### Example 23: Cationic Lipid (E23-1)

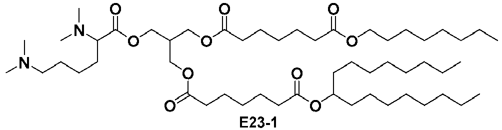

The preparation process is as follows:
Step a: under a nitrogen atmosphere, **S19-2** (0.72 g, 2.0 mmol), **S9-2** (0.80 g, 2.0 mmol) and **DMAP** (0.04 g, 0.3 mmol) were dissolved in anhydrous THF (30 mL) successively, and then **EDCI** (0.04 g, 0.2 mmol) was quickly added to the above mixture under ice bath conditions. When the EDCI was completely dissolved, the reaction solution was warmed to room temperature and the reaction continued for 15 h. After completion of the reaction, the solvent was concentrated, and the residue was dissolved in ethyl acetate, washed with water (10 mL*3), and the organic phase was collected and dried with anhydrous magnesium sulfate, filtered and concentrated, and then purified by column chromatography to obtain **S23-1** (1.22 g).
Step b: under a nitrogen atmosphere, **DCC** (0.45 g, 2.2 mmol) was added to a round-bottom flask containing **S22-1** (0.87 g, 1.2 mmol), **S1-3** (0.20 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E23-1** (0.79 g). ¹H NMR (400 MHz, CDCl₃) δ: 4.87 (t, 1H, >C*H*(CH₂CH₂)₂-), 4.15 (d, 6H, >CH(C*H*₂OC(=O))₃-), 4.04 (t, 2H, -C(=O)OC*H*₂-), 3.16 (t, 1H, -C*H*N(CH₃)₂), 2.80-2.66 (m, 2H, -C*H*₂N(CH₃)₂), 2.55 (s, 6H, >CHN(C*H*₃)₂), 2.43-2.37 (m, 1H, >C*H*(CH₂OC(=O))₃-), 2.34-2.25 (m, 14H; -C*H*₂C(=O)O-, 8H; -CH₂N(C*H*₃)₂, 6H), 1.66-1.55 (m, 4H, -C*H*₂CH₂C*H*₂CH₂N(CH₃)₂), 1.42-1.21 (m, 54H; -CH₂C*H*₂CH₂-, -C*H*₂CH₃), 0.89 (t, 9H, -CH₂C*H*₃). MS (ESI): m/z=924.74 ([M+H]⁺).

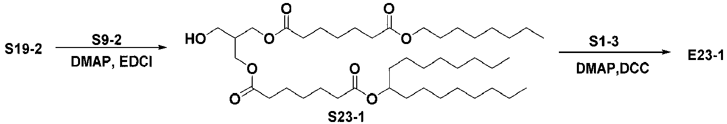

### Example 24: Cationic Lipid (E24-1)

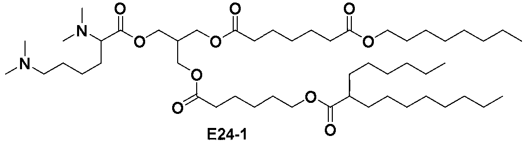

The preparation process is as follows:
Step a: under a nitrogen atmosphere, **S19-2** (0.72 g, 2.0 mmol), **S7-4** (0.74 g, 2.0 mmol) and **DMAP** (0.04 g, 0.3 mmol) were dissolved in anhydrous THF (30 mL) successively, and then **EDCI** (0.04 g, 0.2 mmol) was quickly added to the above mixture under ice bath conditions, and. When the EDCI was completely dissolved, the reaction solution was warmed to room temperature and the reaction continued for 15 h. After completion of the reaction, the solvent was concentrated, and the residue was dissolved in ethyl acetate, washed with water (10 mL*3), and the organic phase was collected and dried with anhydrous magnesium sulfate, filtered and concentrated, and then purified by column chromatography to obtain **S24-1** (1.16 g).
Step b: under a nitrogen atmosphere, **DCC** (0.45 g, 2.2 mmol) was added to a round-bottom flask containing **S24-1** (0.86 g, 1.2 mmol), **S1-3** (0.20 g, 1.0 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E24-1** (0.75 g). ¹H NMR (400 MHz, CDCl₃) δ: 4.15 (d, 6H, >CH(C*H*₂OC(=O))₃-), 4.04 (t, 4H, -C(=O)OC*H*₂-), 3.16 (t, 1H, -C*H*N(CH₃)₂), 2.80-2.66 (m, 2H, -C*H*₂N(CH₃)₂), 2.55 (s, 6H, >CHN(C*H*₃)₂), 2.43-2.37 (m, 1H, >C*H*(CH₂OC(=O))₃-), 2.34-2.25 (m, 13H; -C*H*₂C(=O)O-, 6H; -CH₂N(C*H*₃)₂, 6H; -OC(=O)C*H*(CH₂)₂, 1H), 1.66-1.55 (m, 4H, -C*H*₂CH₂C*H*₂CH₂N(CH₃)₂), 1.42-1.21 (m, 50H; -CH₂C*H*₂CH₂-, -C*H*₂CH₃), 0.88 (t, 9H, -CH₂C*H*₃). MS (ESI): m/z=896.71 ([M+H]⁺).

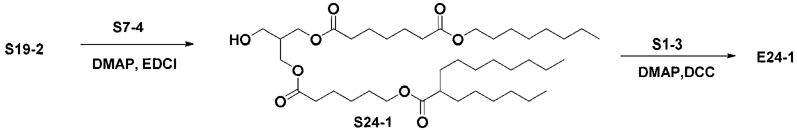

### Example 25: Cationic Lipid (E25-1)

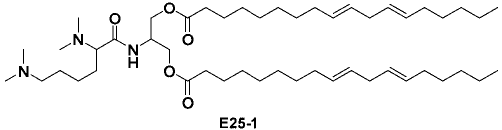

### Method 1:

The preparation process is as follows:
Step a: N-Boc-serine (**S25-1,** 0.96 g, 5.0 mmol) was dissolved in CHCl₃ (30 mL). Then pyridine (1.21 mL, 15.0 mmol) and oleoyl chloride (**S25-2,** 3.28 mL, 10.0 mmol) were added to the solution successively, and the mixture was stirred overnight at room temperature. After completion of the reaction, the mixture was washed sequentially with NH₄Cl sat, water, and saline. The organic phase was dried with anhydrous magnesium sulfate, filtered and concentrated, and then the crude product was purified by column chromatography to obtain compound **S25-3** (3.19 g, 89%).
Step b: Removal of the Boc protecting group. In a dry and clean round-bottom flask, a solution of trifluoroacetic acid/dichloromethane (1:2, v/v) was prepared, and a dichloromethane solution of **S25-3** (2.15 g, 3.0 mmol) was slowly added dropwise under ice bath conditions. The reaction was conducted for 2 h at room temperature. After completion of the reaction, the reaction solution was concentrated, and purified water was added to dilute, followed by extraction with dichloromethane. The extract was dried with anhydrous magnesium sulfate, filtered, the filtrate was concentrated, and recrystallized to obtain compound **S25-4** (1.66 g, 90.1%).
Step c: **S1-3** (0.40 g, 2.0 mmol) was dissolved in 20 mL of dichloromethane, and **EDCI** (0.42 g, 2.2 mmol) was added the solution in batches at 0°C. After stirring for 30 min, **S25-4** (1.23 g, 2.0 mmol) was added dropwise to the reaction solution. After the dropwise addition, the mixture was stirred overnight at room temperature. After completion of the reaction, the organic phase was dried with anhydrous magnesium sulfate, filtered and concentrated, and then the crude product was purified by column chromatography to obtain cationic lipid **E25-1** (1.31 g).

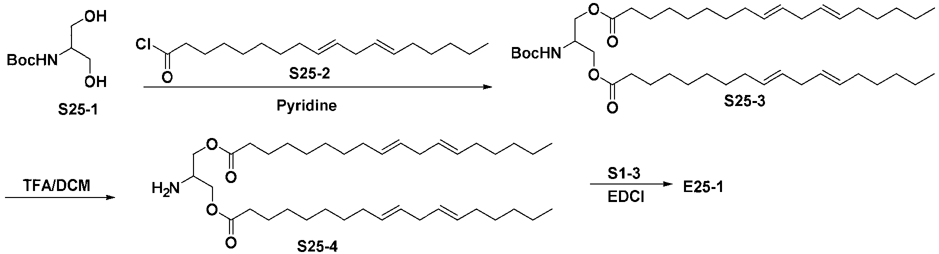

### Method 2:

The preparation process is as follows:
Step a: **S1-3** (1.33 g, 6.6 mmol) was dissolved in DCM (20 mL), then **DCC** (1.36 g, 6.6 mmol) was slowly to the solution added. After stirring for 30 min, **DMAP** (0.07 g, 0.6 mmol) and **S25-5** (1.91 g, 6.0 mmol) were added successively, and the reaction continued for 2 h with stirring. After completion of the reaction, the mixture was filtered, the solvent was concentrated, the crude product was dissolved in DCM, washed once with H₂O, then extracted twice (10 mL*2) with DCM, and the organic layers were combined and dried with anhydrous magnesium sulfate, filtered and concentrated, and then the crude product was purified by column chromatography to obtain compound **S25-6** (2.66 g, 88%).
Step b: the above product **S25-6** (1.51 g, 3.0 mmol) was dissolved in THF (15 mL), and **TBAF** (15 mL, 1 M) was added to the solution under nitrogen protection. The reaction was conducted overnight to remove TBS protection. After completion of the reaction, the solution was concentrated and extracted, the organic layers were combined, dried with anhydrous magnesium sulfate, filtered and concentrated, and then the crude product was purified by column chromatography to obtain **S25-7** (0.74 g, 89.1%).
Step c: **S2-2** (1.23 g, 4.4 mmol) was dissolved in DCM (20 mL), DMF (5 mL) and **DCC** (0.91 g, 4.4 mol) were added to the solution, and then the mixture was stirred for 30 min at room temperature. Subsequently, **S25-7** (0.55 g, 2.0 mmol) was slowly added to the above mixture and stirred for 10 min. Then **DMAP** (0.02 g, 0.2 mmol) was added to the mixture, and the mixture was stirred overnight at room temperature. After completion of the reaction, the solution was filtered and concentrated, and then the crude product was purified by column chromatography to obtain the cationic lipid **E25-1** (1.18 g). ¹H NMR (400 MHz, CDCl₃) δ: 5.44-5.26 (m, 8H, *-CH=CH-),* 4.48-4.42 (m, 1H, -C(=O)NHCH<), 4.15-4.06 (m, 4H, >CH(C*H*₂OC(=O))₂-), 3.18-2.94 (m, 3H; -C*H*₂N(CH₃)₂, 2H; -C*H*N(CH₃)₂, 1H), 2.76-2.68 (m, 4H, -CH=CHC*H*₂CH=CH-), 2.58 (s, 6H, >CHN(C*H*₃)₂), 2.34-2.25 (m, 10H; -OC(=O)C*H*₂-, 4H; -CH₂N(C*H*₃)₂, 6H), 2.08-1.98 (m, 8H, -CH₂C*H*₂CH=CH-), 1.65-1.55 (m, 4H, -C*H*₂CH₂C*H*₂CH₂N(CH₃)₂), 1.47-1.21 (m, 34H; -CH₂C*H*₂CH₂-, -C*H*₂CH₃), 0.87 (t, 6H, -CH₂C*H*₃). MS (ESI): m/z=799.68 ([M+H]⁺).

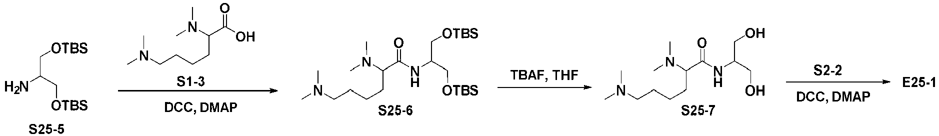

### Example 26: Cationic Lipid (E26-1)

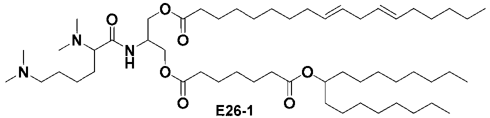

The preparation process is as follows:
Step a: **S25-7** (2.20 g, 8.0 mmol) was dissolved in DMF (120 mL) at room temperature, then **S2-2** (4.61 g, 16.5 mmol), **DMAP** (0.20 g, 1.6 mmol) and **DIPEA** (2.48 g, 19.2 mmol) were added to the solution successively. **EDCI** (3.69 g, 19.2 mmol) was added to the solution in three batches within 15 min and DMF (1 mL) was added at the same time, and the mixture was stirred for 16 h at room temperature. After completion of the reaction, the mixture was washed with water, 1 M HCI and 5% sodium bicarbonate, dried with anhydrous magnesium sulfate, filtered, concentrated, and then the residue was purified by column chromatography to obtain the target compound **S26-1** (2.71 g).
Step b: under a nitrogen atmosphere, **DCC** (0.91 g, 4.4 mmol) was added to a round-bottom flask containing **S26-1** (2.29 g, 2.4 mmol), **S9-2** (0.80 g, 2.0 mmol), and **DMAP** (61.00 mg, 0.5 mmol) dissolved in dichloromethane (50 mL), and the reaction was conducted for 16 h at room temperature. After completion of the reaction, the precipitate was removed by filtration, the filtrate was concentrated, and then the residue was purified by column chromatography to obtain the cationic lipid **E26-1** (1.54 g). ¹H NMR (400 MHz, CDCl₃) δ: 5.44-5.26 (m, 4H, *-CH=CH-),* 4.86 (t, 1H, >C*H*(CH₂CH₂)₂-), 4.48-4.43 (m, 1H, -C(=O)NHCH<), 4.15-4.05 (m, 4H, >CH(C*H*₂OC(=O))₂-), 3.18-2.95 (m, 3H; -C*H*₂N(CH₃)₂, 2H; -C*H*N(CH₃)₂, 1H), 2.76-2.68 (m, 2H, -CH=CHC*H*₂CH=CH-), 2.58 (s, 6H, >CHN(C*H*₃)₂), 2.34-2.25 (m, 12H; -OC(=O)C*H*₂-, 6H; -CH₂N(C*H*₃)₂, 6H), 2.08-1.98 (m, 4H, -CH₂C*H*₂CH=CH-), 1.65-1.55 (m, 4H, -C*H*₂CH₂C*H*₂CH₂N(CH₃)₂), 1.47-1.23 (m, 52H; -CH₂C*H*₂CH₂-, -C*H*₂CH₃), 0.89 (t, 9H, -CH₂C*H*₃). MS (ESI): m/z=917.78 ([M+H]⁺).

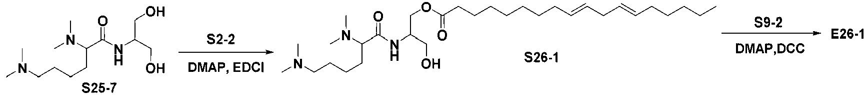

### Example 27: Cationic Lipid (E27-1)

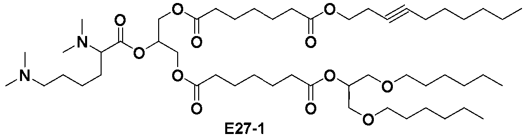

The preparation process is as follows:
Step a: under a nitrogen atmosphere, **DCC** (9.97 g, 48.4 mmol) was added to a round-bottom flask containing **S5-2** (4.75 g, 22.0 mmol), **S27-1** (4.07 g, 26.4 mmol), and **DMAP** (0.67 g, 5.5 mmol) dissolved in dichloromethane (100 mL), and the reaction was conducted for 16 h at room temperature. After completion of the reaction, the precipitate was removed by filtration and the filtrate was concentrated to obtain a crude product. In a dry and clean round-bottom flask, a solution of trifluoroacetic acid/dichloromethane (1:2, v/v) was prepared, and the dichloromethane solution of the above crude product was added dropwise under ice bath condition, reacted at room temperature for 2 h. After completion of the reaction, the reaction solution was concentrated, and purified water was added, followed by extraction with dichloromethane. The extract was dried with anhydrous magnesium sulfate, filtered, the filtrate was concentrated, and then the crude product was recrystallized to obtain compound **S27-2** (5.29 g).
Step b: **S4-1** (1.65 g, 8.0 mmol) was dissolved in DMF (120 mL) at room temperature, then **S27-2** (4.88 g, 16.5 mmol), **DMAP** (0.20 g, 1.6 mmol) and **DIPEA** (2.48 g, 19.2 mmol) were added to the solution successively. **EDCI** (3.69 g, 19.2 mmol) was added to the solution in three batches within 15 min and DMF (1 mL) was added at the same time, and then the mixture was stirred for 16 h at room temperature. After completion of the reaction, the mixture was washed with water, 1 M HCI and 5% sodium bicarbonate, dried with anhydrous magnesium sulfate, filtered, concentrated, and then the residue was purified by column chromatography to obtain the target compound **S27-3** (2.41 g).
Step c: under a nitrogen atmosphere, **DCC** (1.81 g, 8.8 mmol) was added to a round-bottom flask containing **S27-3** (2.33 g, 4.8 mmol), **S5-3** (1.61 g, 4.0 mmol), and **DMAP** (0.12 g, 1.0 mmol) dissolved in dichloromethane (50 mL), and the reaction was conducted for 16 h at room temperature. After completion of the reaction, the precipitate was removed by filtration, the filtrate was concentrated, and then the residue was purified by column chromatography to obtain compound **S27-4** (2.94 g).
Step d: the above product **S27-4** (2.61 g, 3.0 mmol) was dissolved in THF (30 mL), and **TBAF** (30 mL, 1 M) was added to the solution under nitrogen protection. The reaction was conducted overnight to remove TBS protection. After completion of the reaction, the solution was concentrated, extracted, the organic layers were combined, dried with anhydrous magnesium sulfate, filtered and concentrated, and then the crude product was purified by column chromatography to obtain double-substituted diglyceride **S27-5** (1.98 g, 87.6%).
Step e: under a nitrogen atmosphere, **DCC** (0.91 g, 4.4 mmol) was added to a round-bottom flask containing **S27-5** (1.81 g, 2.4 mmol), **S1-3** (0.40 g, 2.0 mmol), and **DMAP** (61.00 mg, 0.5 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E27-1** (1.59 g). ¹H NMR (400 MHz, CDCl₃) δ: 5.29-5.27 (m, 1H, >CHC(=O)OCH<), 5.17-5.03 (m, 1H, >C*H*(CH₂OC*H*₂)₂-), 4.36-4.28 (dd, 2H, >CH(C*H*₂OC(=O))₂-), 4.17-4.10 (m, 4H; >CH(C*H*₂OC(=O))₂-, 2H; -C*H*₂CH₂C≡C-, 2H), 3.58-3.53 (m, 4H, >CH(C*H*₂OC*H*₂)₂-), 3.48-3.38 (m, 4H, >CH(CH₂OC*H*₂)₂-), 3.14 (t, 1H, -C*H*N(CH₃)₂), 2.80-2.66 (m, 2H, -C*H*₂N(CH₃)₂), 2.56 (s, 6H, >CHN(C*H*₃)₂), 2.52-2.46 (m, 2H, -C≡CC*H*₂(CH₂)₄-), 2.34-2.24 (m, 14H; -OC(=O)C*H*₂-, 8H; -CH₂N(C*H*₃)₂, 6H), 2.15-2.10 (m, 2H, -C*H*₂C≡CCH₂(CH₂)₄-), 1.65-1.55 (m, 4H, -C*H*₂CH₂C*H*₂CH₂N(CH₃)₂), 1.49-1.21 (m, 38H; -CH₂C*H*₂CH₂-, -C*H*₂CH₃), 0.89 (t, 9H, -CH₂C*H*₃). MS (ESI): m/z=938.68 ([M+H]⁺).

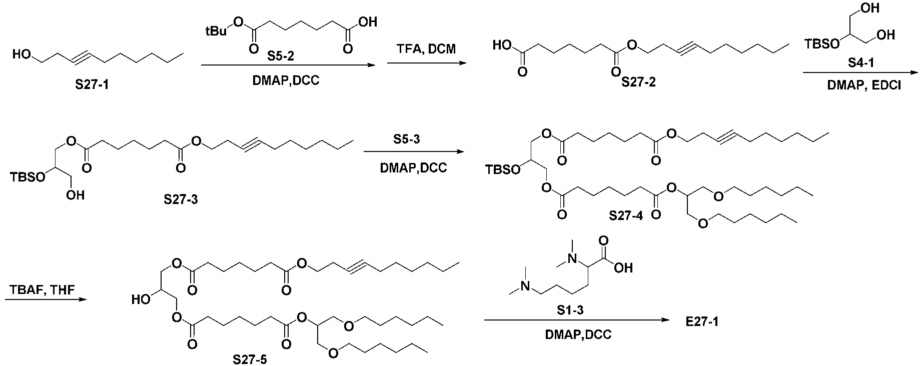

### Example 28.1: Cationic Lipid (E28-1)

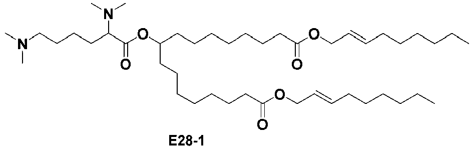

The preparation process is as follows:
Step a: the freshly activated **magnesium granules** (0.79 g, 33.1 mmol), and 2 mL of anhydrous ether were added to a clean round-bottom flask. Bromide 9-bromo-1-nonene **(S28-1,** 5.46 g, 26.6 mmol) was dissolved in anhydrous ether (80 mL) and added to a drop funnel. Under ice bath conditions, the bromide ether solution was added dropwise to the magnesium granules. After the addition was completed, the reaction mixture was reacted at 35°C for 1 h, and then cooled in an ice bath. **Ethyl formate** (0.89 g, 12.0 mmol) was dissolved in anhydrous ether (8 mL) and added to the drop funnel, then added to the reaction mixture with stirring. An exothermic reaction was observed and the reaction mixture began to reflux. After initiation of the reaction, the remaining ether solution of formate was quickly added in the form of a liquid stream and the reaction mixture was stirred for an additional 1 h at room temperature. Then 5 mL of acetone was added dropwise and ice water (15 mL) was added to quench the reaction. The reaction mixture was treated with aqueous H₂SO₄ (10% vol., 100 mL) until the solution became homogeneous and allowed to stratify. The aqueous phase was extracted with ether (50 mL*2), and the merged ether layer was dried with anhydrous magnesium sulfate, and concentrated to obtain a crude product, which was purified by column chromatography to obtain compound **S28-2** (3.02 g).
Step b: **S28-2** (2.80 g, 10.0 mmol) was dissolved in a mixed solution of dichloromethane (30 mL) and acetonitrile (30 mL), then ruthenium chloride **(RuCl₃,** 0.37 g, 1.8 mmol) was added to the mixture, which was cooled to 10°C, and then the aqueous solution of sodium periodic acid (0.22 g, 1.0 mmol) was added dropwise to the mixture. The reaction was stirred at 10°C for 20 h. After completion of the reaction, the reaction mixture was diluted with water, the organic phase and the aqueous phase were separated, saturated salt water was added to the organic layer under stirring, and then 3% sodium sulfide solution was added drop by drop for decolorization. The organic phase and the aqueous phase were separated, the organic layer was dried with sodium sulfate, filtered, and then concentrated to obtain compound **S28-3** (2.72 g).
Step c: under nitrogen atmosphere, **tert-butyldimethylchlorosilane** (1.16 g, 7.7 mmol) was added to a round-bottom flask containing **S28-3** (2.21 g, 7.0 mmol) and **imidazole** (1.19 g, 17.5 mmol) dissolved in DMF (50 mL), and the reaction solution was stirred overnight at 50°C. After completion of the reaction, the solution was cooled to room temperature, and the reaction mixture was diluted with water. The mixture was extracted three times with ethyl acetate, the organic layers were combined and washed once with saturated salt water, then dried with sodium sulfate, filtered, concentrated, and the crude product was purified by column chromatography to obtain a diacid with a TBS-protected hydroxyl group (**S28-4,** 2.72 g, 90%).
Step d: under a nitrogen atmosphere, **DCC** (1.85 g, 9.0 mmol) was added to a round-bottom flask containing **S20-1** (0.71 g, 5.0 mmol), **S28-4** (0.86 g, 2.0 mmol), and **DMAP** (0.12 g, 1.0 mmol) dissolved in dichloromethane (20 mL), and the reaction was conducted for 16 h at room temperature. After completion of the reaction, the precipitate was removed by filtration and the filtrate was concentrated. The residue was dissolved in tetrahydrofuran (20 mL), then 20 mL of **TBAF** tetrahydrofuran solution (1 M) was added to the solution. The reaction was conducted overnight to remove TBS protection. After completion of the reaction, the solution was concentrated, extracted, the organic layers were combined, dried with anhydrous magnesium sulfate, filtered and concentrated, and then the crude product of S28-5 was purified by column chromatography to obtain compound **S28-5** (0.86 g).
Step e: under a nitrogen atmosphere, **DCC** (0.45 g, 2.2 mmol) was added to a round-bottom flask containing **S1-3** (0.24 g, 1.0 mmol), **S28-5** (0.57 g, 1.2 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E28-1** (0.62 g). ¹H NMR (400 MHz, CDCl₃) δ: 5.82-5.71 (m, 2H, -C(=O)OCH₂C*H*=CH-), 5.56-5.54 (m, 2H, -C(=O)OCH₂CH=C*H*-), 4.91-4.83 (m, 1H, -C(=O)OC*H*<), 4.55 (t, 4H, -C(=O)OC*H*₂CH=CH-), 314 (t, 1H, -C*H*N(CH₃)₂), 2.80-2.66 (m, 2H, -C*H*₂N(CH₃)₂), 2.56 (s, 6H, >CHN(C*H*₃)₂), 2.25 (s, 6H, -CH₂N(C*H*₃)₂), 2.23 (t, 4H, -C*H*₂C(=O)O-), 2.06-1.99 (m, 4H, -CH=CHC*H*₂CH₂-), 1.65-1.55 (m, 4H, -C*H*₂CH₂C*H*₂CH₂N(CH₃)₂), 1.47-1.19 (m, 42H; -CH₂C*H*₂CH₂-, -C*H*₂CH₃), 0.87 (t, 6H, -CH₂C*H*₃). MS (ESI): m/z=748.73 ([M+H]⁺).

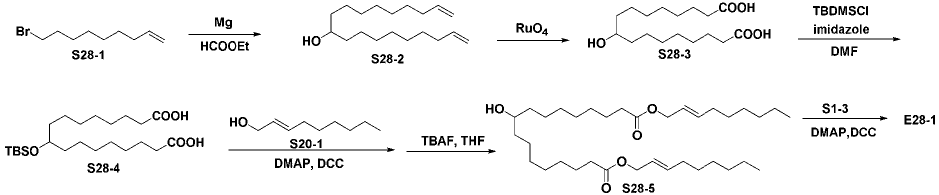

### Example 28.2: Cationic Lipid (E28-2)

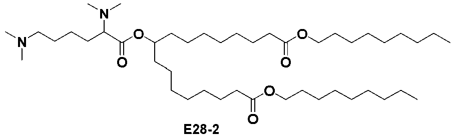

The preparation process is as follows:
Step a: under a nitrogen atmosphere, **DCC** (1.85 g, 9.0 mmol) was added to a round-bottom flask containing 1-nonanol (**S28-6,** 0.72 g, 5.0 mmol), **S28-4** (0.86 g, 2.0 mmol), and **DMAP** (0.12 g, 1.0 mmol) dissolved in dichloromethane (30 mL), and the reaction was conducted for 16 h at room temperature. After completion of the reaction, the precipitate was removed by filtration and the filtrate was concentrated. The residue was dissolved in tetrahydrofuran (20 mL), and 20 mL of **TBAF** tetrahydrofuran solution (1 M) was added to the solution. The reaction was conducted overnight to remove TBS protection. After completion of the reaction, the solution was concentrated, extracted, the organic layers were combined, dried with anhydrous magnesium sulfate, filtered and concentrated, and then the crude product of S28-7 was purified by column chromatography to obtain compound **S28-7** (0.88 g).
Step b: under a nitrogen atmosphere, **DCC** (0.45 g, 2.2 mmol) was added to a round-bottom flask containing **S28-7** (0.57 g, 1.0 mmol), **S1-3** (0.24 g, 1.2 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E28-2** (0.63 g). ¹H NMR (400 MHz, CDCl₃) δ: 4.92-4.83 (m, 1H, -C(=O)OCH<), 4.14-4.04 (t, 4H, -C(=O)OC*H*₂-), 3.14 (t, 1H, -C*H*N(CH₃)₂), 2.80-2.66 (m, 2H, -C*H*₂N(CH₃)₂), 2.55 (s, 6H, >CHN(C*H*₃)₂), 2.25 (s, 6H, -CH₂N(C*H*₃)₂), 2.23 (t, 4H, -C*H*₂C(=O)O-), 1.64-1.55 (m, 4H, -C*H*₂CH₂C*H*₂CH₂N(CH₃)₂), 1.47-1.19 (m, 54H; -CH₂C*H*₂CH₂-, -C*H*₂CH₃), 0.86 (t, 6H, -CH₂C*H*₃). MS (ESI): m/z=752.66 ([M+H]⁺).

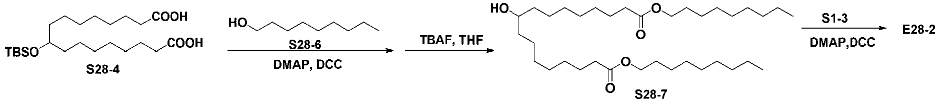

### Example 29: Cationic Lipid (E29-1)

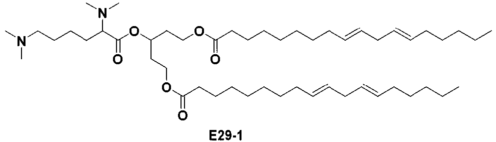

The preparation process is as follows:
Step a: under ice bath conditions, tert-butyldimethylchlorosilane (**TBDMSCl**, 1.81 g, 12.0 mmol) and **imidazole** (1.36 g, 20.0 mmol) were added to a round-bottom flask containing diethyl 3-hydroxyglutarate (**S29-1**, 1.76 g, 10.0 mmol) dissolved in DCM (30 mL). The reaction mixture was warmed to room temperature and the reaction was continued with stirring for 17 h. After completion of the reaction, the reaction mixture was filtered and concentrated, and then the crude product was purified by column chromatography to obtain compound **S29-2** (1.97 g).
Step b: under ice bath conditions, THF (1M, 4.8 mL, 4.8 mmol) solution of diisobutylaluminum hydride (**DIBAL-H**) was added to a round-bottom flask containing **S29-2** (1.74 g, 6.0 mmol) dissolved in DCM (20 mL). The reaction mixture was warmed to room temperature and the reaction was continued with stirring for 17 h. After completion of the reaction, the reaction was quenched with MeOH, filtered and concentrated, and then the crude product was purified by column chromatography to obtain compound **S29-3** (0.78 g).
Step c: under a nitrogen atmosphere, **DCC** (1.85 g, 9.0 mmol) was added to a round-bottom flask containing **S2-2** (1.40 g, 5.0 mmol), **S29-3** (0.47 g, 2.0 mmol), and **DMAP** (0.12 g, 1.0 mmol) dissolved in dichloromethane (30 mL), and the reaction was conducted for 16 h at room temperature. After completion of the reaction, the precipitate was removed by filtration and the filtrate was concentrated. The residue was dissolved in tetrahydrofuran (20 mL), then 20 mL of **TBAF** tetrahydrofuran solution (1 M) was added to the solution. The reaction was conducted overnight to remove TBS protection. After completion of the reaction, the solution was concentrated, extracted, the organic layers were combined, dried with anhydrous magnesium sulfate, filtered and concentrated, and then the crude product of S29-4 was purified by column chromatography to obtain compound **S29-4** (1.00 g).
Step d: under a nitrogen atmosphere, **DCC** (0.45 g, 2.2 mmol) was added to a round-bottom flask containing **S29-4** (0.65 g, 1.0 mmol), **S1-3** (0.24 g, 1.2 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E29-1** (0.70 g). ¹H NMR (400 MHz, CDCl₃) δ: 5.35-5.24 (m, 8H, *-CH=CH-),* 4.91-4.82 (m, 1H, -C(=O)OCH<), 4.14-4.04 (t, 4H, -C(=O)OC*H*₂-), 3.14 (t, 1H, -C*H*N(CH₃)₂), 2.80-2.66 (m, 6H; -C*H*₂N(CH₃)₂, 2H; CH=CHC*H*₂CH=CH-, 4H), 2.55 (s, 6H, >CHN(C*H*₃)₂), 2.25 (s, 6H, -CH₂N(C*H*₃)₂), 2.23 (t, 4H, -C*H*₂C(=O)O-), 2.03-1.97 (m, 8H, -CH=CHC*H*₂-), 1.79-1.77 (m, 4H, -C(=O)OCH₂C*H*₂-), 1.64-1.55 (m, 4H, -C*H*₂CH₂C*H*₂CH₂N(CH₃)₂), 1.47-1.19 (m, 34H; -CH₂C*H*₂CH₂-, -C*H*₂CH₃), 0.86 (t, 6H, -CH₂C*H*₃). MS (ESI): m/z=828.70 ([M+H]⁺).

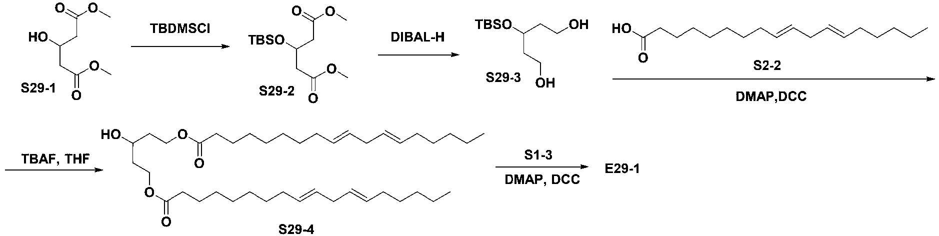

### Example 30: Cationic Lipid (E30-1)

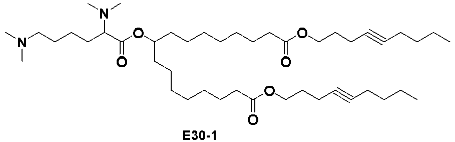

The preparation process is as follows:
Step a: under a nitrogen atmosphere, **DCC** (1.85 g, 9.0 mmol) was added to a round-bottom flask containing 4-nonyn-1-ol (**S30-1,** 0.70 g, 5.0 mmol), **S28-4** (0.86 g, 2.0 mmol), and **DMAP** (0.12 g, 1.0 mmol) dissolved in dichloromethane (30 mL), and the reaction was conducted for 16 h at room temperature. After completion of the reaction, the precipitate was removed by filtration and the filtrate was concentrated. The residue was dissolved in tetrahydrofuran (20 mL), and 20 mL of **TBAF** tetrahydrofuran solution (1 M) was added to the solution. The reaction was conducted overnight to remove TBS protection. After completion of the reaction, the solution was concentrated, extracted, the organic layers were combined, dried with anhydrous magnesium sulfate, filtered and concentrated, and then the crude product of S30-2 was purified by column chromatography to obtain compound **S30-2** (0.86 g).
Step b: under a nitrogen atmosphere, **DCC** (0.45 g, 2.2 mmol) was added to a round-bottom flask containing **S30-2** (0.56 g, 1.0 mmol), **S1-3** (0.24 g, 1.2 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E30-1** (0.61 g). ¹H NMR (400 MHz, CDCl₃) δ: 4.91-4.83 (m, 1H, -C(=O)OCH<), 4.14-4.04 (t, 4H, -C(=O)OC*H*₂-), 3.14 (t, 1H, -C*H*N(CH₃)₂), 2.80-2.66 (m, 2H, -C*H*₂N(CH₃)₂), 2.55 (s, 6H, >CHN(C*H*₃)₂), 2.27-2.25 (m, 10H; -CH₂N(C*H*₃)₂, 6H; -C(=O)O(CH₂)₂C*H*₂-, 4H), 2.23 (t, 4H, -C*H*₂C(=O)O-), 2.14 (tt, 4H, -C≡CC*H*₂(CH₂)₂CH₃), 1.80 (q, 4H, -C(=O)OCH₂C*H*₂CH₂-), 1.64-1.55 (m, 4H, -C*H*₂CH₂C*H*₂CH₂N(CH₃)₂), 1.47-1.19 (m, 34H; -CH₂C*H*₂CH₂-, -C*H*₂CH₃), 0.86 (t, 6H, -CH₂C*H*₃). MS (ESI): m/z=744.60 ([M+H]⁺).

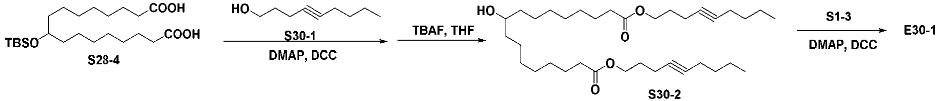

### Example 31: Cationic Lipid (E31-1)

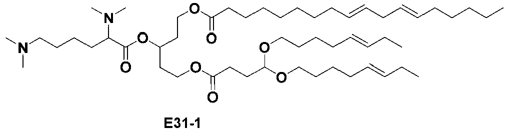

The preparation process is as follows:
Step a: pyridinium tosylate (**PPTS,** 0.20 g, 0.8 mmol) was dissolved in toluene at room temperature, then **S3-2** (2.51 g, 16.0 mmol) and 2-octen-1-ol (**S31-1,** 6.14 g, 48.0 mmol) were added to the mixture successively, and then the mixture was reacted at 135°C for 20 h. After completion of the reaction, the reaction solution was cooled to room temperature, extracted, dried, concentrated, and then the crude product was purified by column chromatography to obtain compound **S31-2** (2.16 g).
Step b: **S31-2** (1.93 g, 60.0 mmol) was dissolved in ethanol (30 mL), then **KOH** (0.84 g, 15.0 mmol) was added dropwise to the mixture. The reaction mixture was reacted at 110°C for 24 h. After completion of the reaction, the reactants were cooled to room temperature, and the pH of the solution was adjusted to 5 by adding dilute HCl. Then the solution was extracted twice with hexane (10 mL*2), and the organic layers were combined, washed with water and saline, dried with anhydrous magnesium sulfate, filtered and concentrated, and then the crude product was purified by column chromatography to obtain compound **S31-3** (1.94 g, 95%)
Step c: **S29-3** (0.94 g, 4.0 mmol) was dissolved in DMF (50 mL) at room temperature, then **S31-3** (2.81 g, 8.2 mmol), **DMAP** (0.10 g, 0.8 mmol) and **DIPEA** (1.24 g, 9.6 mmol) were added to the solution successively. **EDCI** (1.84 g, 9.6 mmol) was added to the solution in three batches within 15 min and DMF (1 mL) was added at the same time, then the mixture was stirred for 16 h at room temperature. After completion of the reaction, the mixture was washed with water, 1 M HCI and 5% sodium bicarbonate, dried with anhydrous magnesium sulfate, filtered, concentrated, and then the residue was purified by column chromatography to obtain the target compound **S31-4** (1.40 g).
Step d: under a nitrogen atmosphere, **DCC** (0.91 g, 4.4 mmol) was added to a round-bottom flask containing **S31-4** (1.11 g, 2.0 mmol), **S2-2** (0.48 g, 2.4 mmol), and **DMAP** (0.06 g, 0.5 mmol) dissolved in dichloromethane (20 mL), and the reaction was conducted for 16 h at room temperature. After completion of the reaction, the precipitate was removed by filtration and the filtrate was concentrated. The residue was dissolved in tetrahydrofuran (20 mL), and 20 mL of **TBAF** tetrahydrofuran solution (1 M) was added to the solution. The reaction was conducted overnight to remove TBS protection. After completion of the reaction, the solution was concentrated, extracted, the organic layers were combined, dried with anhydrous magnesium sulfate, filtered and concentrated, and then the crude product of S31-5 was purified by column chromatography to obtain compound **S31-5** (1.37 g).
Step e: under a nitrogen atmosphere, **DCC** (0.45 g, 2.2 mmol) was added to a round-bottom flask containing **S31-4** (0.71 g, 1.0 mmol), **S1-3** (0.24 g, 1.2 mmol), and **DMAP** (31.00 mg, 0.3 mmol) dissolved in dichloromethane (20 mL), and the reaction was conducted for 16 h at room temperature. After completion of the reaction, the precipitate was removed by filtration, the filtrate was concentrated, and then the residue was purified by column chromatography to obtain the cationic lipid **E31-1** (0.74 g). ¹H NMR (400 MHz, CDCl₃) δ: 5.41-5.26 (m, 8H, *-CH=CH-),* 4.91-4.83 (m, 1H, -C(=O)OC*H*<), 4.62 (t, 1H, >C*H*(OCH₂)₂-), 4.02 (t, 4H, -C(=O)OC*H*₂-), 3.52-3.13 (m, 5H; >CH(OC*H*₂)₂-, 4H; -C*H*N(CH₃)₂, 1H), 2.80-2.66 (m, 4H; -C*H*₂N(CH₃)₂, 2H; -CH=CHC*H*₂CH=CH-, 2H), 2.56 (s, 6H, >CHN(C*H*₃)₂), 2.34-2.25 (m, 10H; -OC(=O)C*H*₂-, 2H; -CH₂N(C*H*₃)₂, 6H), 2.11-1.82 (m, 14H; -CH₂C*H*₂CH=CH-, 12H; -C*H*₂CH(OCH₂)₂-, 2H), 1.79-1.77 (m, 4H, -C(=O)OCH₂C*H*₂-), 1.65-1.55 (m, 4H, -C*H*₂CH₂C*H*₂CH₂N(CH₃)₂), 1.48-1.20 (m, 26H; -CH₂C*H*₂CH₂-, -C*H*₂CH₃), 0.88 (t, 9H, -CH₂C*H*₃). MS (ESI): m/z=888.72 ([M+H]⁺).

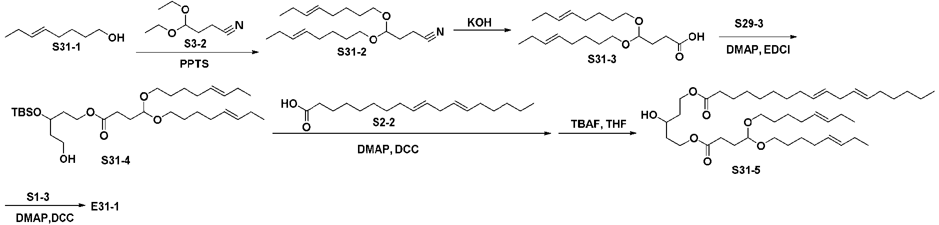

### Example 32: Preparation of LNP-mRNA Pharmaceutical Composition and Assays of

### Physicochemical Properties Thereof

### Example 32.1: Preparation of LNP-mRNA Pharmaceutical Compositions

In this embodiment, multiple groups of lipid compositions containing a cationic lipid represented by formula (1) of the present application and lipid nanoparticles (LNPs) containing the lipid compositions were prepared, specifically LNP-mRNA pharmaceutical compositions containing Fluc-mRNA. In each group, the phospholipid contained was DSPC, and the steroid lipid contained was cholesterol. The differences among the groups lied in two components, the cationic lipid and the PEGylated lipid. Wherein, in the control group (L-CT), the PEGylated lipid was PEG2k-DMG (abbreviated as DMG), and the cationic lipid was Compound 29 (C29); in the experimental group series (L-1~L-35), the PEGylated lipid was DMG or PL-14, and the cationic lipid was selected from those prepared in the embodiments of the present application. Specifically, the formulation of each lipid composition is shown in Table 1.

The Compound 29 (C29) was obtained with reference to the preparation method disclosed by CN101674853A, and the structure is as follows:

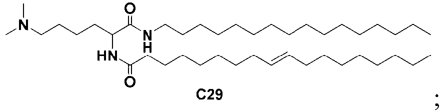

The PL-1 was obtained with reference to the preparation method disclosed by CN115353616A, and the structure is as follows:

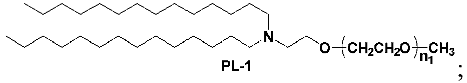

The PL-14 was obtained with reference to the preparation method disclosed by CN115515926A, and the structure is as follows:

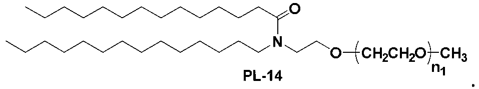

The preparation method for LNP-mRNA pharmaceutical composition is as follows:
Step a: cationic lipid, DSPC, cholesterol, and PEG-lipid were dissolved in ethanol at a molar ratio of 48:9:42:1.5 to obtain an ethanol phase solution;
Step b: Fluc-mRNA was added to 10~50 mM citrate buffer (pH=4) to obtain an aqueous solution;
Step c: the ethanol phase solution and the aqueous phase solution were mixed (1:3 v/v) to prepare LNP-mRNA, washed by multiple DPBS ultrafiltration to remove ethanol and free molecules, and finally, filtered through a 0.2 µm sterile filter to obtain the LNP-mRNA pharmaceutical composition.

### Example 32.2: Assays of Physicochemical Properties of LNP-mRNA Pharmaceutical Compositions

Determination of encapsulation rate: the encapsulation rate of LNP-mRNA compositions was determined using the Quant-it Ribogreen RNA Quantification Kit. The results showed that the lipid compositions (L-1~L-35) of the present application had high encapsulation rates for nucleic acid drugs (mRNA), all in the range of 80%-95%, and most of the encapsulation rates were in the range of 90%-95%. The results showed that the cationic lipids containing two tertiary amine groups in each experimental group can effectively encapsulate mRNA, with encapsulation rates close to or even better than that of the cationic lipid C29. There were also differences in the encapsulation rate among different cationic lipids containing two tertiary amine groups.

Determination of particle size: in the present embodiment, the particle size of LNP-mRNA was determined by dynamic light scattering (DLS). The measured LNP-mRNA had high dimensional uniformity, and the PDI values were all smaller than 0.3. The particle sizes of the LNP-mRNA prepared with the lipid compositions of the present application were in the range of 90-120 nm, which met the requirements for particle size of gene vectors.

**Table 1: Summary of the Formulation of Each Lipid Composition, and Particle Size and Encapsulation Rate of the Prepared LNP-mRNA**

| **LNP-mRNA** | **L-CT** | **L-1** | **L-2** | **L-3** | **L-4** | **L-5** |
|---|---|---|---|---|---|---|
| **Cationic Lipid, CL** | C29 | E1-1 | E2-1 | E3-1 | E4-1 | E5-1 |
| **PEGylated Lipid, PL** | DMG | | | | | |
| **Encapsulation Rate /%** | 88.73 | 89.47 | 95.11 | 90.65 | 93.26 | 91.73 |
| **Particle Size /nm** | 93.84 | 92.05 | 91.33 | 93.58 | 94.51 | 95.62 |

| **LNP-mRNA** | **L-6** | **L-7** | **L-8** | **L-9** | **L-10** | **L-11** |
|---|---|---|---|---|---|---|
| **Cationic Lipid, CL** | E6-1 | E7-1 | E8-1 | E9-1 | E10-1 | E11-1 |
| **PEGylated Lipid, PL** | DMG | | | | | |
| **Encapsulation Rate /%** | 92.10 | 91.73 | 89.47 | 93.23 | 92.16 | 93.36 |
| **Particle Size /nm** | 98.48 | 96.48 | 94.37 | 92.12 | 94.82 | 96.51 |

| **LNP-mRNA** | **L-12** | **L-13** | **L-14** | **L-15** | **L-16** | **L-17** |
|---|---|---|---|---|---|---|
| **Cationic Lipid, CL** | E12-1 | E13-1 | E14-1 | E15-1 | E16-1 | E17-1 |
| **PEGylated Lipid, PL** | DMG | | | | | |
| **Encapsulation Rate /%** | 92.18 | 93.10 | 87.26 | 95.14 | 92.22 | 94.15 |
| **Particle Size /nm** | 97.47 | 95.95 | 93.68 | 93.25 | 94.69 | 95.32 |

| **LNP-mRNA** | **L-18** | **L-19** | **L-20** | **L-21** | **L-22** | **L-23** |
|---|---|---|---|---|---|---|
| **Cationic Lipid, CL** | E17-2 | E18-1 | E19-1 | E20-1 | E21-1 | E22-1 |
| **PEGylated Lipid, PL** | DMG | | | | | |
| **Encapsulation Rate /%** | 89.88 | 93.76 | 93.47 | 93.37 | 92.19 | 93.72 |
| **Particle Size /nm** | 98.41 | 95.26 | 93.72 | 94.12 | 95.86 | 92.36 |

| **LNP-mRNA** | **L-24** | **L-25** | **L-26** | **L-27** | **L-28** | **L-29** |
|---|---|---|---|---|---|---|
| **Cationic Lipid, CL** | E23-1 | E24-1 | E25-1 | E26-1 | E27-1 | E28-1 |
| **PEGylated Lipid, PL** | DMG | | | | | |
| **Encapsulation Rate /%** | 92.17 | 91.73 | 93.39 | 91.66 | 92.42 | 91.16 |
| **Particle Size /nm** | 93.59 | 94.51 | 93.42 | 94.43 | 93.56 | 92.55 |

| **LNP-mRNA** | **L-30** | **L-31** | **L-32** | **L-33** | **L-34** | **L-35** |
|---|---|---|---|---|---|---|
| **Cationic Lipid, CL** | E28-2 | E29-1 | E30-1 | E31-1 | E9-1 | E9-1 |
| **PEGylated Lipid, PL** | DMG | | | | PL-1 | PL-14 |
| **Encapsulation Rate /%** | 92.73 | 92.22 | 90.42 | 90.19 | 93.12 | 92.88 |
| **Particle Size /nm** | 94.51 | 94.11 | 93.44 | 94.82 | 95.42 | 94.13 |

### Example 33: Biological Activity Assays of the Formulation of LNP-mRNA Pharmaceutical Composition

### (1) Assays for cytotoxicity (biocompatibility)

The cytotoxicity of the formulation of LNP-mRNA pharmaceutical composition of the present application was tested by MTT assay. The formulation of LNP-mRNA pharmaceutical composition was dissolved in the culture medium to prepare the required concentration; if necessary, an appropriate amount of co-solvent could be added. Hela cells were used as a cell model, and 100 µL/well of cell suspension was seeded into 96-well plates at a density of 1×10⁴ cells/well. After completion of inoculation, the cells were incubated in a cell culture incubator at 37°C, 4% CO₂ for 24 h, and then the culture medium was discarded. Then, mRNA was administered at a dose of 0.1-0.3 µg per well by adding 100 µL of culture medium (containing the LNP-mRNA pharmaceutical compositions prepared in **Example 32.1**) into each well; 100 µL of fresh culture medium was added to the blank control group. For each concentration (0.1 µg, 0.15 µg, 0.20 µg, 0.25 µg, and 0.3 µg) in each group, 6 replicate wells were set. After 24 h co-incubation of the formulation of LNP-mRNA pharmaceutical composition with 293T cells, 20 µL of PBS buffer containing 5 mg/mL MTT was added to each well. After 4 h incubation of MTT with cancer cells, the mixture of medium and MTT buffer was discarded, followed by adding 150 µL DMSO per well to dissolve the purple formazan crystals formed in living cells. After sufficient shaking, the absorbance was tested with a microplate reader. Calculations were performed based on the measured absorbance values. The results showed that, compared with the blank control group, the cell viability rates of the formulations of LNP-mRNA pharmaceutical composition prepared by the present application were all greater than 95%, which were in the range of 95% to 100%, indicating that the formulations of LNP-mRNA pharmaceutical composition of the present application exhibited good biocompatibility.

### (2) Serum stability evaluation

LNP-mRNA pharmaceutical compositions were added to the medium containing 10% fetal bovine serum (FBS) with agitation at 37°C. Samples were taken at regular intervals to determine the particle size change of LNP-mRNA, and the serum stability of the formulation of LNP-mRNA pharmaceutical composition was analyzed by testing its particle size change. The experimental results showed that, within 7 days, the particle size changes of the control group and the experimental groups were all less than 10%, and the particle size changes of all the experimental groups were less than 5%, indicating that the formulation of LNP-mRNA pharmaceutical composition prepared with cationic lipids of the present application exhibited good serum stability.

### (3) Study on the mRNA transfection efficiency in cells

To investigate the mRNA transfection efficiency at the cellular level of each group of LNP-mRNA pharmaceutical compositions prepared in Example 32.1 of the present application, luciferase bioluminescence was used for testing. The LNP-mRNA pharmaceutical composition was dissolved in medium to prepare the required dose. Hela cells were used as the cell model, and cell suspensions of 100 µL/well were seeded in a black-edged, clear-bottomed 96-well plate at a density of 6000 cells/well. After seeding, the cells were incubated in a cell culture incubator for 24 h. Then the cells were dosed with 0.2 µg mRNA per well, and the blank control group was added with the corresponding dose of free Fluc-mRNA. After 24 h of transfection, the old culture medium was replaced with a fresh one containing the D-fluorescein sodium substrate (1.5 mg/mL). After 5 min of incubation, bioluminescence was detected using a microplate reader. The stronger the fluorescence, the more Fluc-mRNA was transported into the cytoplasm and translated into the corresponding fluorescent protein, as shown in Table 2. Wherein, the relative value of fluorescence intensity was the ratio of the fluorescence intensity value of each group to the fluorescence intensity of the blank control group. The results showed that the LNP-mRNA pharmaceutical composition prepared by the present application had excellent in vitro transfection efficiency. Therefore, the LNPs of the experimental groups were all effective nucleic acid delivery carriers. This could be attributed to the two ionizable tertiary amine structures in the cationic lipids of the present application, which resulted in improved transfection efficiency of the corresponding LNPs compared with the L-CT group containing a lysine-core cationic lipid of the prior art. The relative fluorescence values of L-2, L-7, L-9, L-11, L-15, L-21, L-31, L-34, and L-35 were higher among the experimental groups, and there were differences in the fluorescence intensity among experimental groups, which may be due to the following differences in cationic lipids: the type and/or number of degradable linking bonds, and/or the type of hydrophobic hydrocarbon tail chain (e.g., with ether bonds or without ether bonds), and/or the degree of saturation of hydrophobic hydrocarbon tail chains. Specifically, compared among E1-1 (L-1), E2-1 (L-2) and E25-1 (L-26), the transfection efficiency of E2-1 and E25-1 containing a linking bond in the hydrophobic aliphatic tail chain was higher, and the transfection efficiency of E2-1 with an ester bond in the head group was higher than that of E25-1 with an amide bond in the head group. This could be attributed to the degradable ester bond, which could avoid the endosomal accumulation of LNP-mRNA, promote the endosomal escape of LNP-mRNA, and facilitate the release of mRNA into the cytoplasm to exert efficacy. Compared among E28-1 (L-29), E28-2 (L-30) and E30-1 (L-32), E28-2 and E30-1 containing unsaturated hydrophobic hydrocarbon tail chains had higher transfection efficiency, which could be attributed to unsaturated hydrocarbon chains that increased the membrane fluidity to enhance cellular uptake. E3-1 (L-3), E5-1 (L-5), E12-1 (L-12), E13-1 (L-13), and E14-1 (L-14) had a lower transfection efficiency than other cationic lipids containing two tertiary amine groups of the present application; this was probably due to their hydrophobic hydrocarbon tail chains, all of which contained ether bonds, and the presence of ether bonds could increase hydrophilicity, which was not conducive to the formation of conical geometry. However, some cationic lipids containing ether bonds only in part of the hydrophobic tail chains had a higher transfection efficiency. For example, E16-1 (L-13) containing an ether bond only in part of hydrophobic aliphatic tail chains had a higher transfection efficiency compared with E16-1 (L-17).

**Table 2: Results of Cell Transfection Assays**

| **Entry** | **Relative Fluorescence Value** | **Entry** | **Relative Fluorescence Value** | **Entry** | **Relative Fluorescence Value** |
|---|---|---|---|---|---|
| **Blank** | 1 | **L-14** | 6.1 | **L-29** | 7.9 |
| **L-CT** | 4.8 | **L-15** | 8.6 | **L-30** | 8.2 |
| **L-1** | 5.7 | **L-16** | 8.1 | **L-31** | 8.9 |
| **L-2** | 8.5 | **L-17** | 8.2 | **L-32** | 8.1 |
| **L-3** | 5.8 | **L-18** | 7.9 | **L-33** | 8.2 |
| **L-4** | 7.9 | **L-19** | 7.6 | **L-34** | 8.6 |
| **L-5** | 6.3 | **L-20** | 7.5 | **L-35** | 9.8 |
| **L-6** | 7.5 | **L-21** | 8.5 | | |
| **L-7** | 9.8 | **L-22** | 7.4 | | |
| **L-8** | 8.6 | **L-23** | 7.9 | | |
| **L-9** | 10.3 | **L-24** | 8.2 | | |
| **L-10** | 7.7 | **L-25** | 8.4 | | |
| **L-11** | 9.3 | **L-26** | 7.3 | | |
| **L-12** | 6.9 | **L-27** | 7.1 | | |
| **L-13** | 6.5 | **L-28** | 8.2 | | |

Those described above are only embodiments of the present application and are not for limitation. Any modification of equivalent structures or equivalent routes according to the present application, which may be applied in other related art directly or indirectly, should be included in the scope of the present application.

For those skilled in the art, without departing from the spirit and scope of the present application, and without unnecessary experimentation, the present application can be implemented in a wider range under equivalent parameters, concentrations, and conditions. While the present application has given particular examples, it should be understood that the present application can be further modified. In conclusion, under the principles of the present application, the present application is intended to cover any alterations, uses, or improvements of the present application, including changes departing from the scope disclosed in this application but made using conventional techniques known in the art.

## Claims

1. A cationic lipid, wherein the structure is represented by the general formula (1): 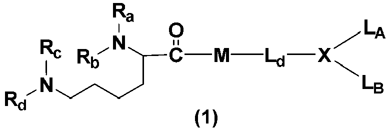
wherein, Rₐ, R_{b}, R_{c} and R_{d} are each independently a C₁₋₃ alkyl group; or, Rₐ and R_{b}, together with the nitrogen atom to which they are connected, form a nitrogen-containing heterocyclic group; or, R_{c} and R_{d}, together with the nitrogen atom to which they are connected, form a nitrogen-containing heterocyclic group;
M is O, S, or NH;
L_{d} is a linking bond or a divalent linking group;
X is N or CRₘ, and the Rₘ is H or a C₁₋₁₂ alkyl group;
L_{A} is represented by -B₃-L₃-B₁-L₁-R₁, and L_{B} is represented by -B₄-L₄-B₂-L₂-R₂; wherein, L₁, L₂, L₃, and L₄ are each independently a linking bond or a divalent linking group L; B₁, B₂, B₃, and B₄ are each independently a linking bond or a C₁₋₂₀ alkylene group; R₁ and R₂ are each independently a C₁₋₃₀ hydrocarbon group or a C₁₋₃₀ hydrocarbon derivative residue containing 1 to 4 units of -O-;
or a pharmaceutically acceptable salt, tautomer, stereoisomer, deuterated derivative, or solvate thereof.

2. The cationic lipid according to claim **1,** wherein L_{d} is selected from the group consisting of a linking bond, -(CH₂)ₜ-, -(CH₂)ₜZ-, -(CH₂)ₜZ-(CH₂)ₜZ-, and -(CH₂)ₜZ(CH₂)ₜZ-; wherein, t is independently an integer from 1 to 12 at each occurrence; Z is independently selected from the group consisting of -C(=O)-, -OC(=O)-, -C(=O)O-, -OC(=O)O-, -O-, -S-, -C(=O)S-, -SC(=O)-, -NRC(=O)-, -C(=O)NR_{g}, -NR_{g}C(=O)NR_{g}-, -OC(=O)NR_{g}-, -NR_{g}C(=O)O-, -SC(=O)NR_{g}-, and -NR_{g}C(=O)S- at each occurrence; wherein, R_{g} is independently H or a C₁₋₁₂ alkyl group at each occurrence; L_{d} is preferably selected from the group consisting of a linking bond, -(CH₂)ₜ-, -(CH₂)ₜO-, -(CH₂)ₜC(=O)-, -(CH₂)ₜC(=O)O-, -(CH₂)ₜOC(=O)-, -(CH₂)ₜC(=O)NH-, -(CH₂)ₜNHC(=O)-, -(CH₂)ₜOC(=O)O-, -(CH₂)ₜNHC(=O)O-, -(CH₂)ₜOC(=O)NH-, -(CH₂)ₜNHC(=O)NH-, -(CH₂)ₜO(CH₂)ₜ-, -(CH₂)ₜC(=O)(CH₂)ₜ-, -(CH₂)ₜC(=O)O(CH₂)ₜ-, -(CH₂)ₜOC(=O)(CH₂)ₜ-, -(CH₂)ₜC(=O)NH(CH₂)ₜ-, -(CH₂)ₜNHC(=O)(CH₂)ₜ-, -(CH₂)ₜOC(=O)O(CH₂)ₜ-, -(CH₂)ₜNHC(=O)O(CH₂)ₜ-, -(CH₂)ₜOC(=O)NH(CH₂)ₜ-, and -(CH₂)ₜNHC(=O)NH(CH₂)ₜ-; most preferably, L_{d} is a linking bond or -(CH₂)ₜ-.

3. The cationic lipid according to claim **1,** wherein L is independently selected from the group consisting of -OC(=O)-, -C(=O)O-, -OC(=O)O-, -C(=O)-, -O-, -NH-, -O(CR_{g}R_{g})ₛO-, -S-, -C(=O)S-, -SC(=O)-, -NR_{g}C(=O)-, -C(=O)NR_{g}-, -NR_{g}C(=O)NR_{g}-, -OC(=O)NR_{g}-, -NR_{g}C(=O)O-, -SC(=O)NR_{g}-, -NR_{g}C(=O)S-, -C(=S)-, -OC(=S)-, -C(=S)O-, -OC(=S)O-, -NR_{g}C(=S)-, -C(=S)NR_{g}-, -NR_{g}C(=S)NR_{g}-, -OC(=S)NR_{g}-, and -NR_{g}C(=S)O- at each occurrence; wherein, R_{g} is independently a hydrogen atom or a C₁₋₁₂ alkyl group at each occurrence, and s is 2, 3 or 4; preferably, L₁, L₂, L₃, and L₄ are selected from any of the following cases:
Case (1): L₁, L₂, L₃, and L₄ are each independently L;
Case (2): any one of L₁, L₂, L₃, and L₄ is a linking bond, and the other three are each independently L; preferably, L₃ is a linking bond, and L₁, L₂, and L₄ are each independently L;
Case (3): any two of L₁, L₂, L₃, and L₄ are linking bonds, and the other two are each independently L; preferably, L₃ and L₄ are linking bonds, and L₁ and L₂ are each independently L;
Case (4): any three of L₁, L₂, L₃, and L₄ are linking bonds, and the other is L;
Case (5): L₁, L₂, L₃, and L₄ are all linking bonds;
more preferably, L₁, L₂, L₃, and L₄ are selected from any one of the aforementioned Case (1) to Case (3);
most preferably, L₁, L₂, L₃, and L₄ are selected from any of the following cases:
Case (a): L₁, L₂, L₃, and L₄ are each independently selected from the group consisting of -OC(=O)-, -C(=O)O-, -C(=O)NH-, -OC(=O)O-, and -NHC(=O)O-;
Case (b): L₃ is a linking bond; L₁, L₂, and L₄ are each independently selected from the group consisting of -OC(=O)-, -C(=O)O-, -C(=O)NH-, -OC(=O)O-, and -NHC(=O)O-;
Case (c): L₃ and L₄ are linking bonds; L₁ and L₂ are each independently selected from the group consisting of -OC(=O)-, -C(=O)O-, -C(=O)NH-, -OC(=O)O-, and -NHC(=O)O-.

4. The cationic lipid according to claim **1,** wherein B₁, B₂, B₃, and B₄ are selected from any of the following cases:
Case (1): B₁, B₂, B₃, and B₄ are each independently a C₁₋₂₀ alkylene group, more preferably a C₁₋₁₀ alkylene group;
Case (2): any one of B₁, B₂, B₃, and B₄ is a linking bond, and the other three are C₁₋₁₀ alkylene groups; preferably, B₃ is a linking bond, with B₁, B₂, and B₄ each independently being a C₁₋₁₀ alkylene group;
Case (3): any two of B₁, B₂, B₃, and B₄ are linking bonds, and the other two are C₁₋₁₀ alkylene groups; preferably, B₃ and B₄ are linking bonds, with B₁ and B₂ each independently being a C₁₋₁₀ alkylene group;
Case (4): any three of B₁, B₂, B₃, and B₄ are linking bonds, and the other is a C₁₋₁₀ alkylene group;
`Case (5): B₁, B₂, B₃, and B₄ are all linking bonds;
more preferably, B₁, B₂, B₃, and B₄ are selected from any one of the aforementioned Case (1) to Case (3);
wherein, the aforementioned C₁₋₁₀ alkylene group is preferably selected from the group consisting of a methylene group, an ethylene group, a propylene group, a butylene group, a pentylene group, a hexylene group, a heptylene group, and an octylene group.

5. The cationic lipid according to claim **1,** wherein R₁ and R₂ are selected from any of the following cases:
Case (1): both R₁ and R₂ are C₁₋₃₀ hydrocarbon groups;
Case (2): both R₁ and R₂ are C₁₋₃₀ hydrocarbon derivative residues;
Case (3): one of R₁ and R₂ is a C₁₋₃₀ hydrocarbon group, and the other is a C₁₋₃₀ hydrocarbon derivative residue;
the C₁₋₃₀ hydrocarbon derivative residue is represented by 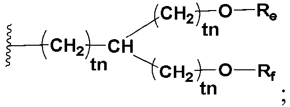 wherein, the tn is independently an integer from 0 to 12 at each occurrence; Rₑ and R_{f} are each independently selected from the group consisting of a C₁₋₁₅ alkyl group, a C₂₋₁₅ alkenyl group, and a C₂₋₁₅ alkynyl group.

6. The cationic lipid according to claim **5,** wherein the C₁₋₃₀ hydrocarbon group is a linear C₁₋₃₀ hydrocarbon group or a branched C₁₋₃₀ hydrocarbon group;
the linear C₁₋₃₀ hydrocarbon group is selected from the group consisting of a linear C₁₋₃₀ alkyl group, a linear C₂₋₃₀ alkenyl group, and a linear C₂₋₃₀ alkynyl group; preferably, the linear C₁₋₃₀ hydrocarbon group is selected from the group consisting of a linear C₁₋₂₅ alkyl group, a linear C₂₋₂₅ alkenyl group, and a linear C₂₋₂₅ alkynyl group; more preferably, the linear C₁₋₃₀ hydrocarbon group is a linear C₂₋₂₅ alkenyl group;
the branched C₁₋₃₀ hydrocarbon group is selected from the group consisting of a branched C₁₋₃₀ alkyl group, a branched C₂₋₃₀ alkenyl group, and a branched C₂₋₃₀ alkynyl group, each independently represented by 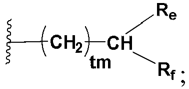 wherein, tm is an integer from 0 to 12; Rₑ and R_{f} are each independently selected from the group consisting of a C₁₋₁₅ alkyl group, a C₂₋₁₅ alkenyl group, and a C₂₋₁₅ alkynyl group; the branched C₁₋₃₀ hydrocarbon group is preferably selected from the group consisting of the following structures: 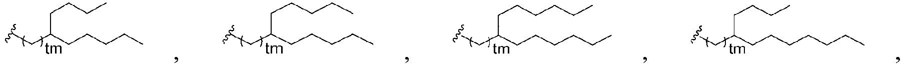 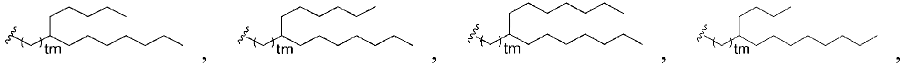 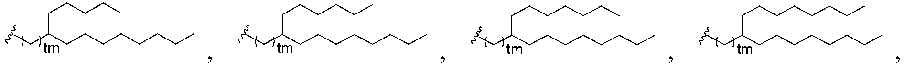 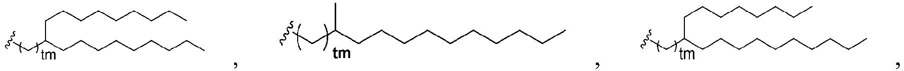 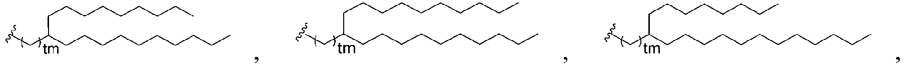 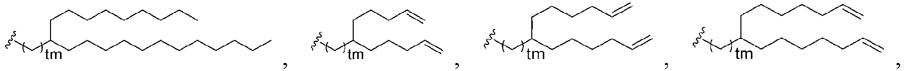 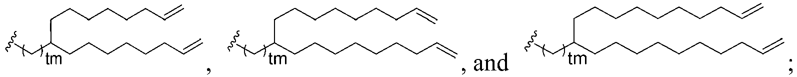 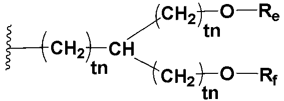 is preferably selected from the group consisting of 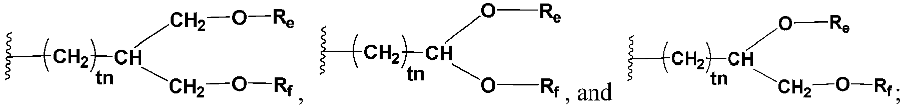 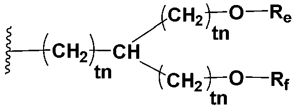 is further preferably selected from the group consisting of the following structures:

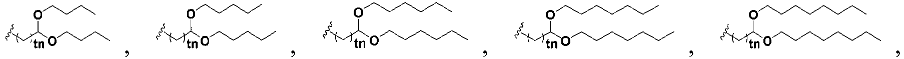

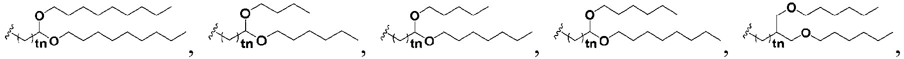

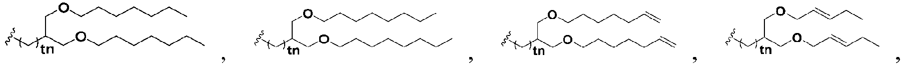

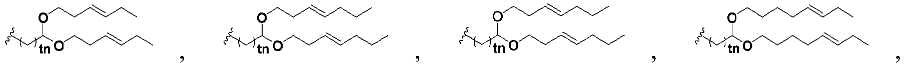

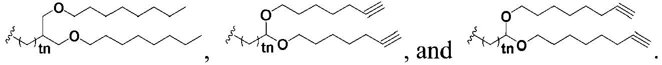

7. The cationic lipid according to claim 1, wherein L_{A} and L_{B} are each independently selected from the group consisting of the following structures: 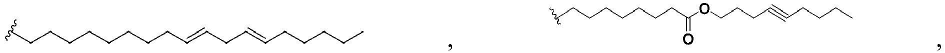 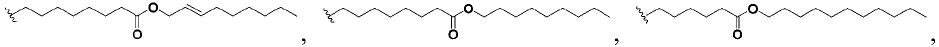 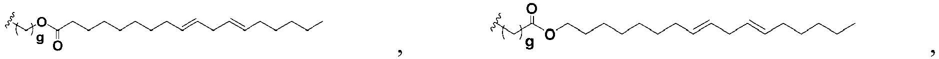 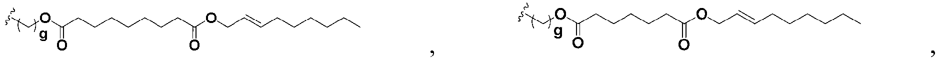 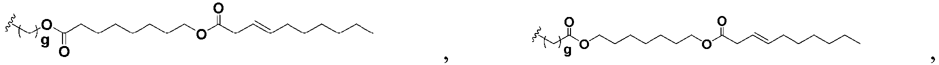 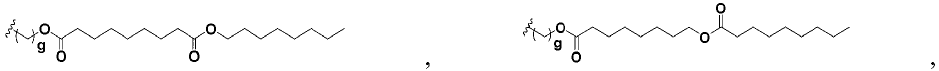 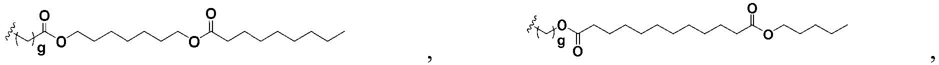 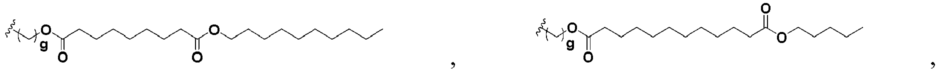 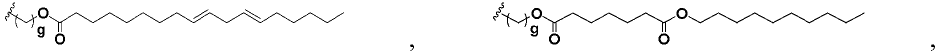 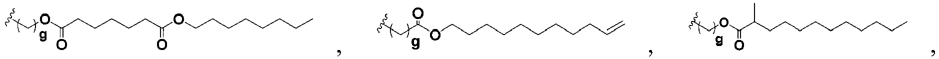 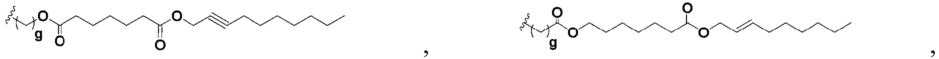 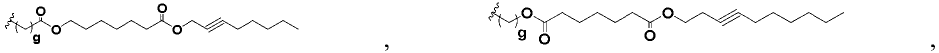 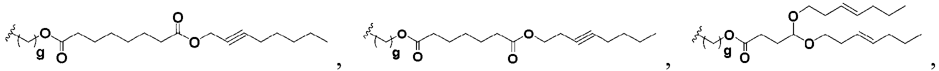 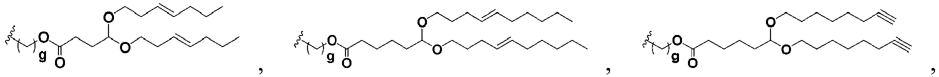 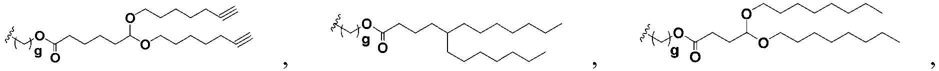 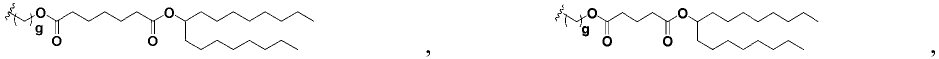 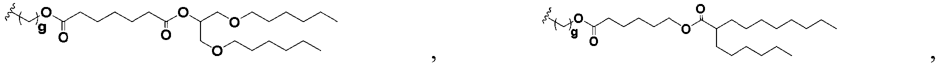 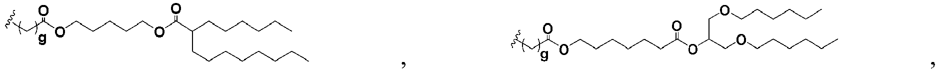 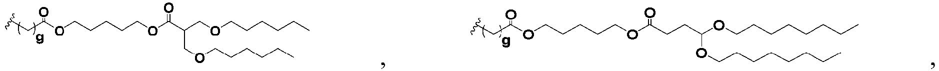 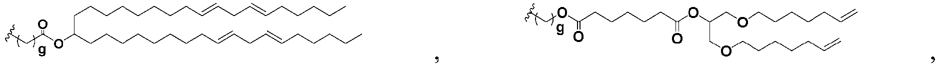 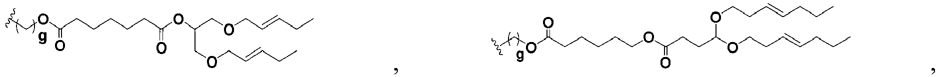 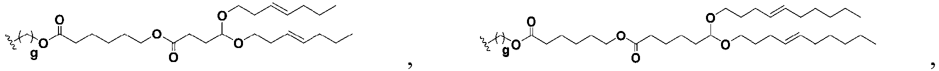 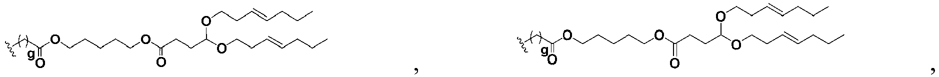 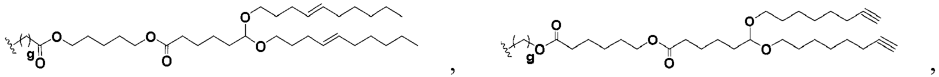 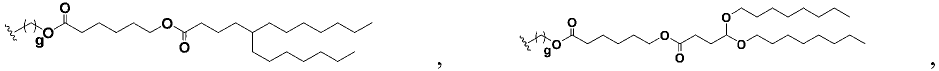 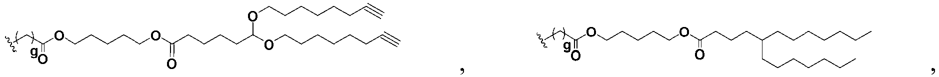 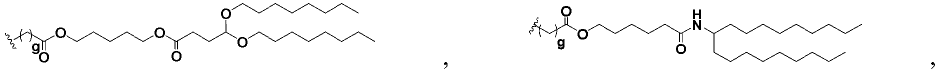 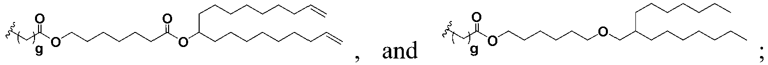 wherein, g is independently an integer from 1 to 10 at each occurrence, more preferably 1, 2, or 3.

8. The cationic lipid according to claim **1,** wherein Rₐ and R_{b} are identical and are both methyl groups or ethyl groups; R_{c} and R_{d} are identical and are both methyl groups or ethyl groups;
or, Rₐ and R_{b}, together with the nitrogen atom to which they are connected, form a nitrogen-containing heterocyclic group; R_{c} and R_{d}, together with the nitrogen atom to which they are connected, form a nitrogen-containing heterocyclic group; the nitrogen-containing heterocyclic group is 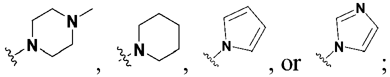
more preferably, Rₐ, R_{b}, R_{c}, and R_{d} are all methyl groups.

9. The cationic lipid according to claim 1, wherein the structure of the cationic lipid is represented by the following general formula (1-A) or (1-B): 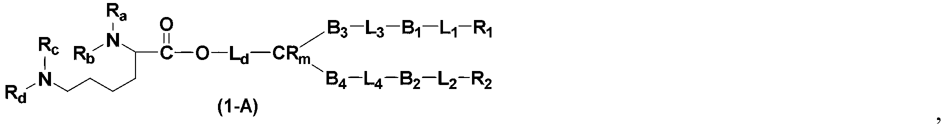 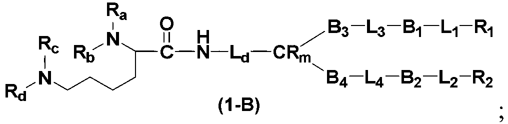 preferably, the structure of the cationic lipid is represented by general formula (2-1) or (2-2): 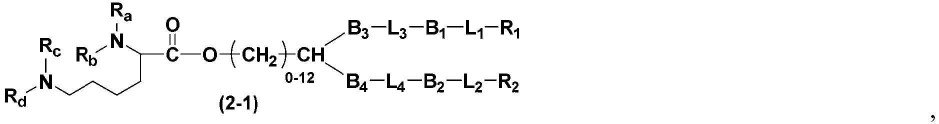 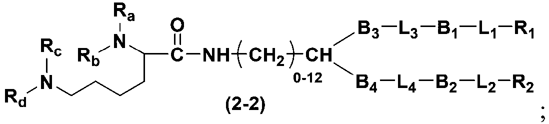 more preferably, the structure of the cationic lipid is selected from the group consisting of the following general formulas: 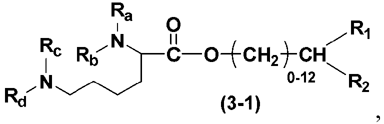 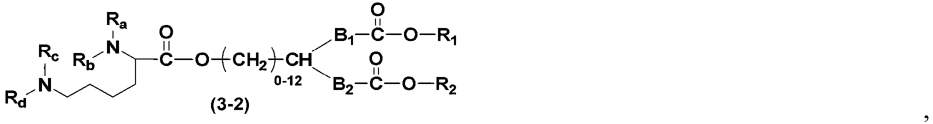 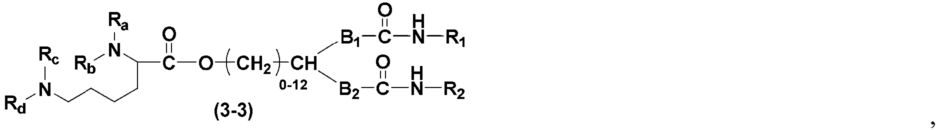 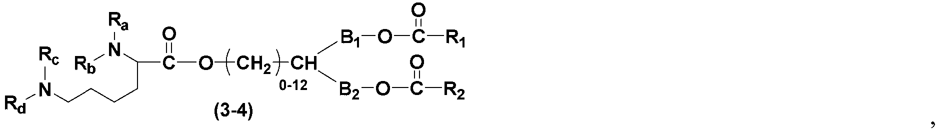 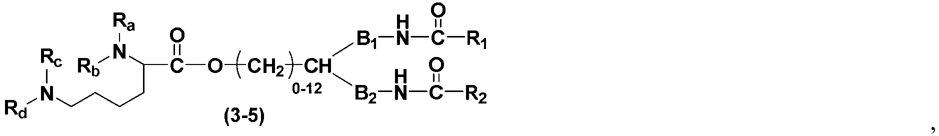 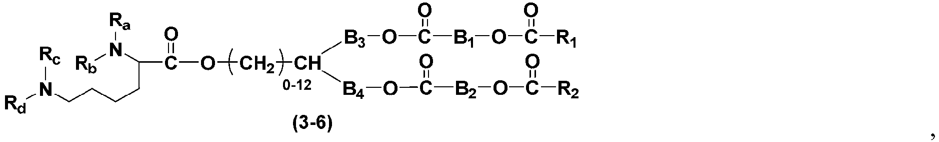 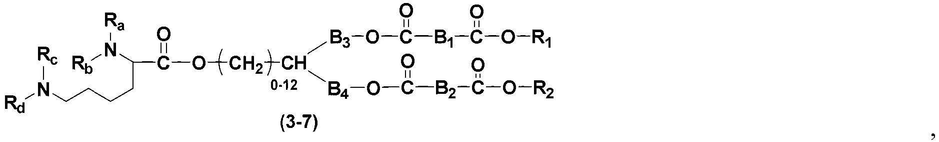 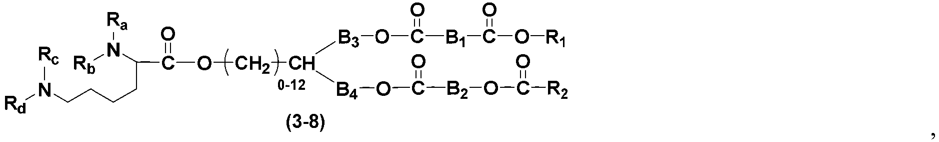 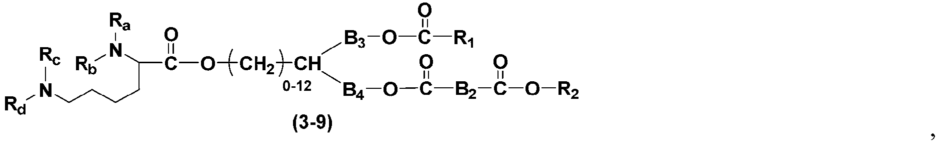 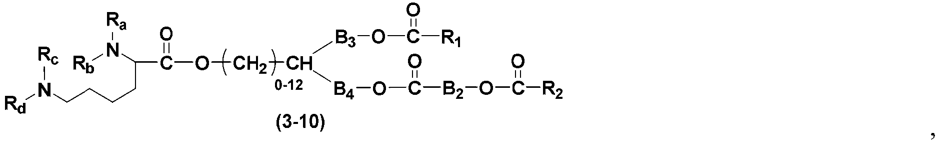 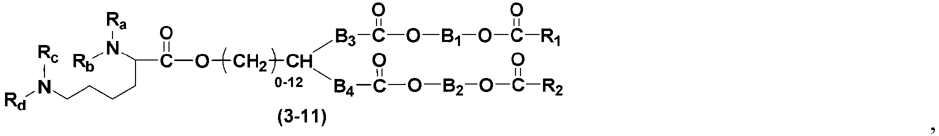 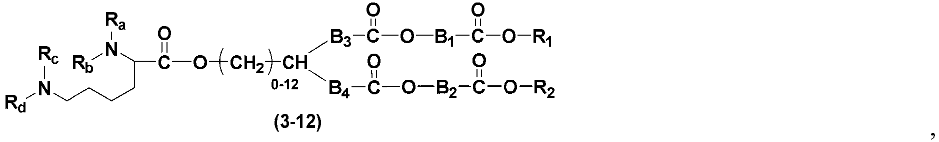 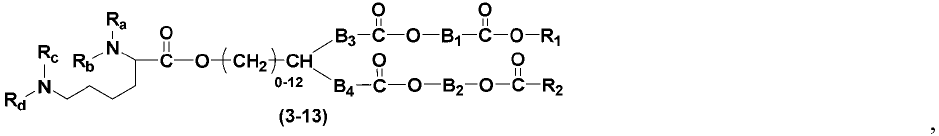 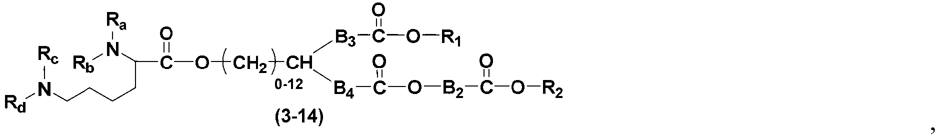 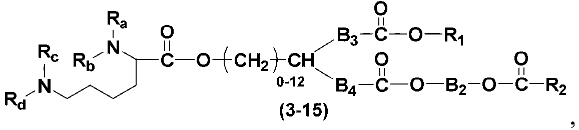 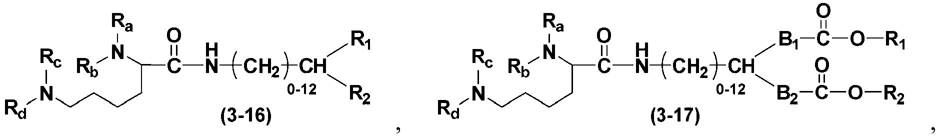 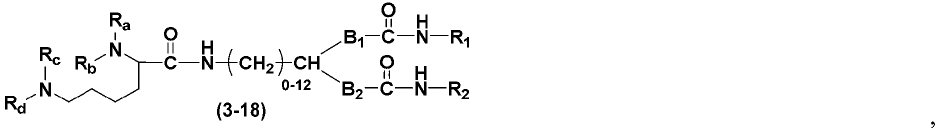 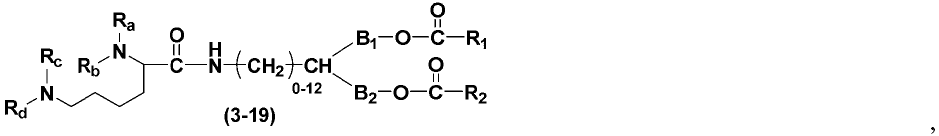 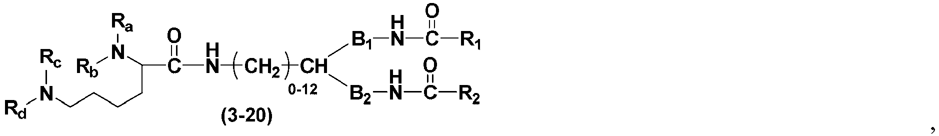 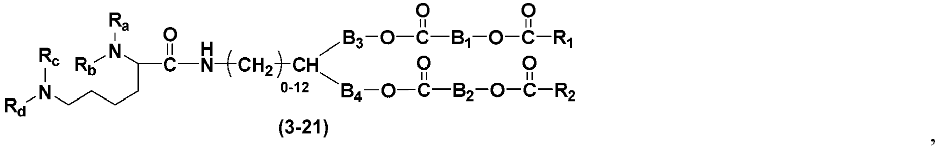 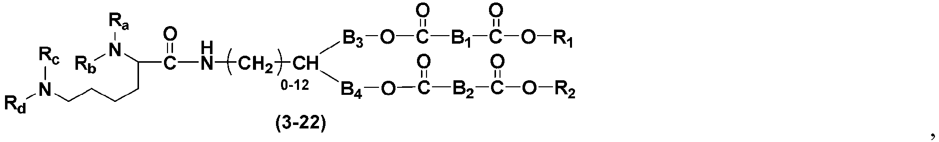 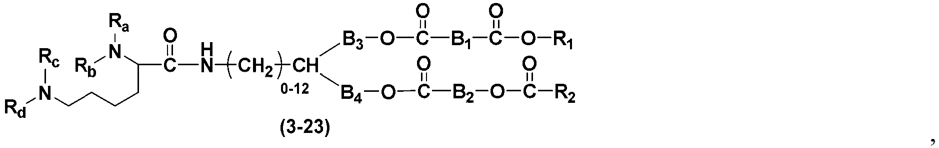 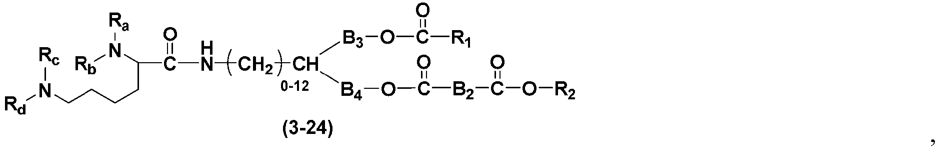 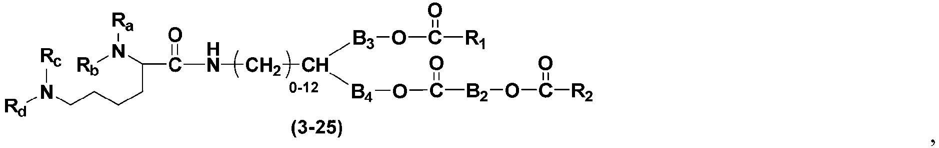 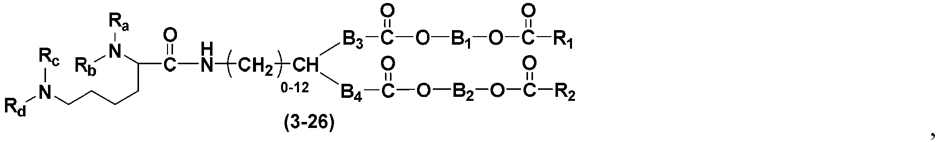 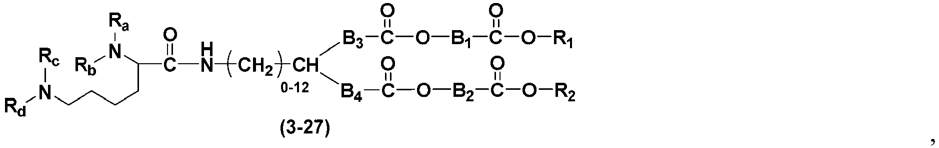 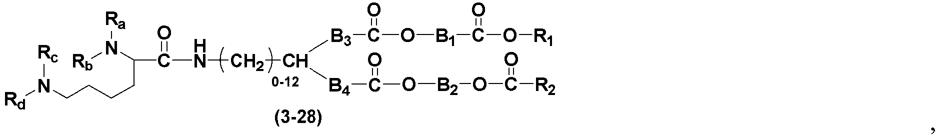 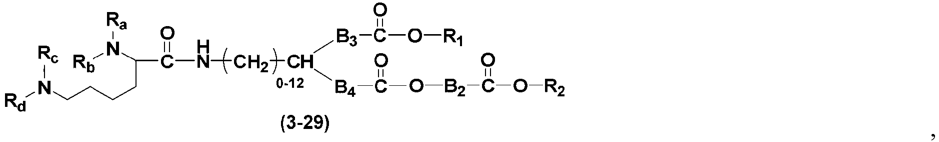 and 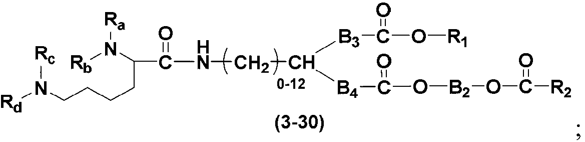 in the formulas (3-1) to (3-30), B₁, B₂, B₃, and B₄ are not linking bonds; more preferably, B₁, B₂, B₃, and B₄ are each independently a C₁₋₁₀ alkylene group at each occurrence.

10. The cationic lipid according to claim **1,** wherein the structure of the cationic lipid is selected from the group consisting of the following structures: 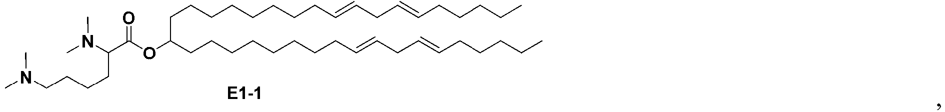 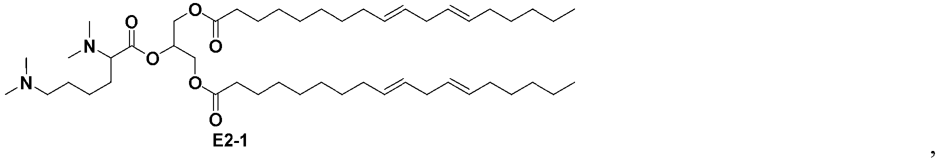 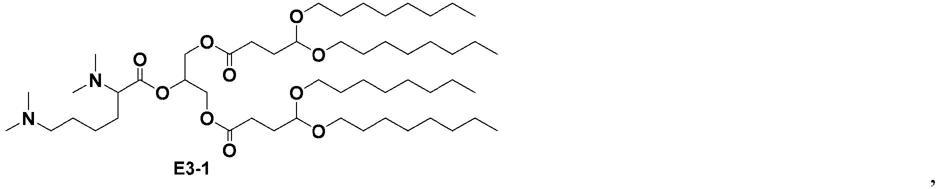 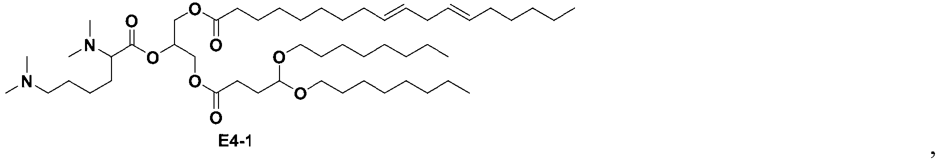 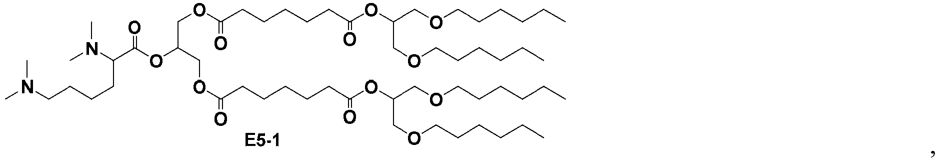 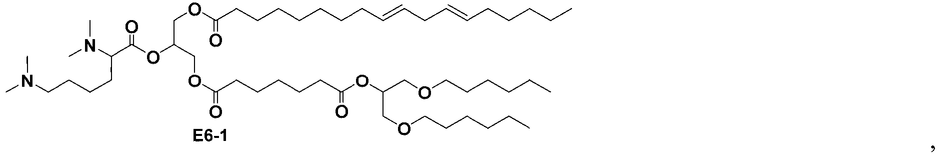 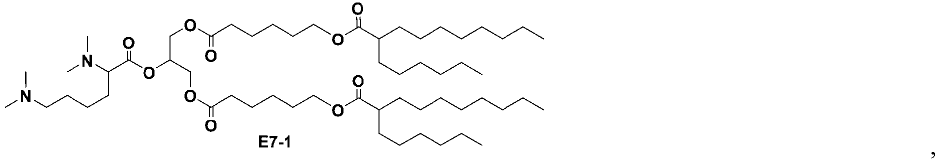 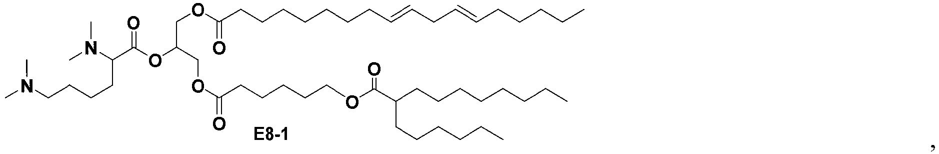 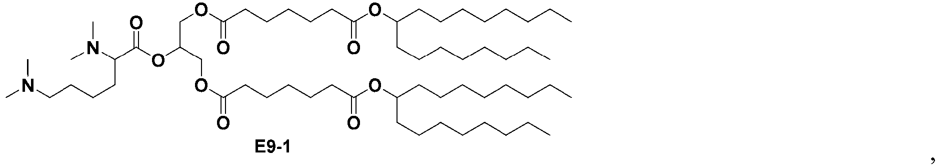 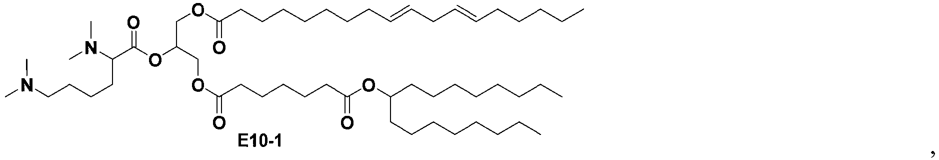 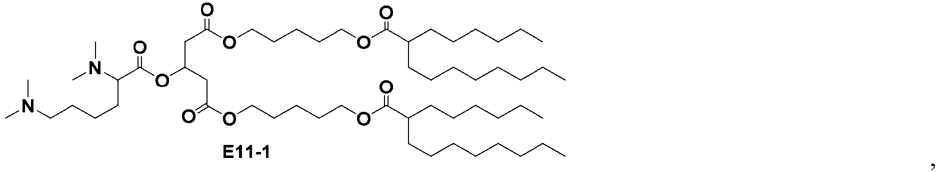 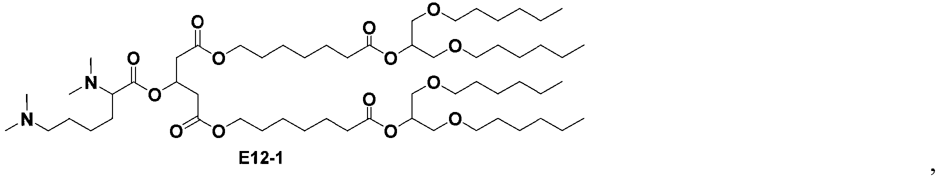 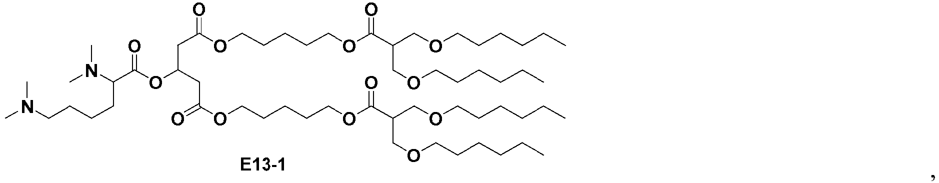 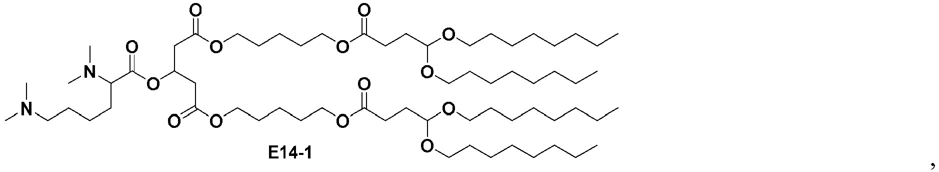 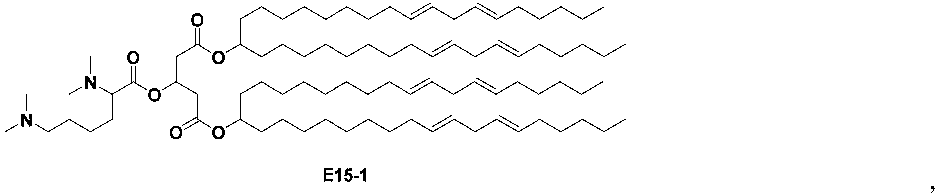 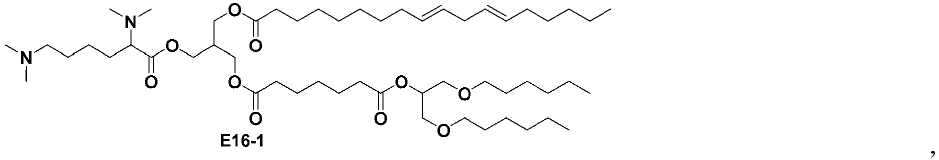 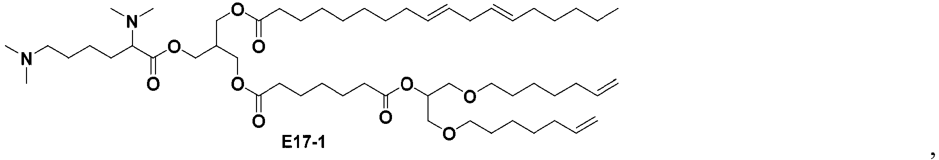 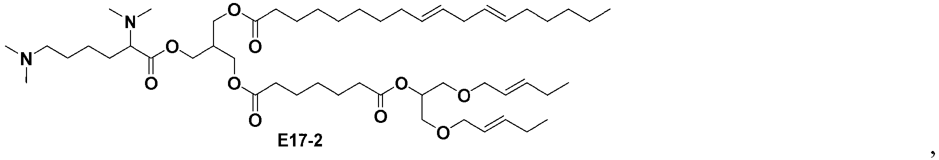 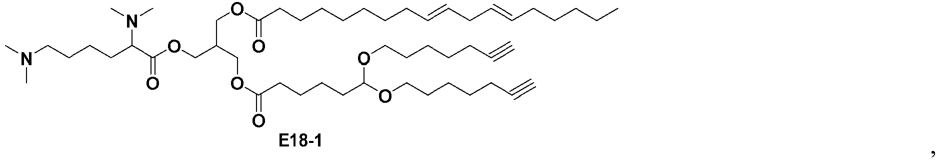 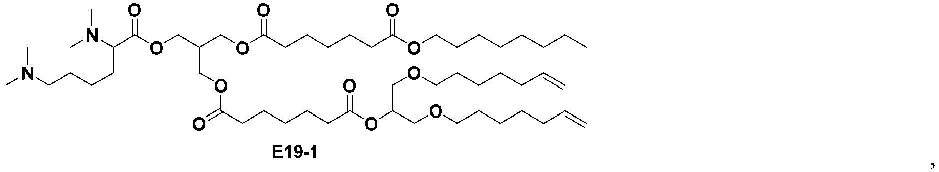 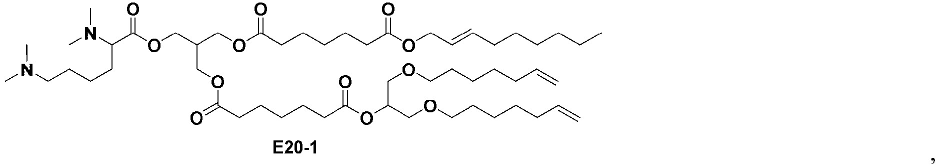 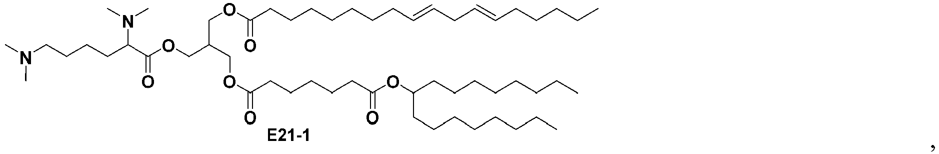 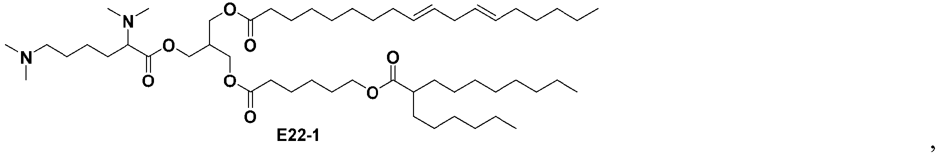 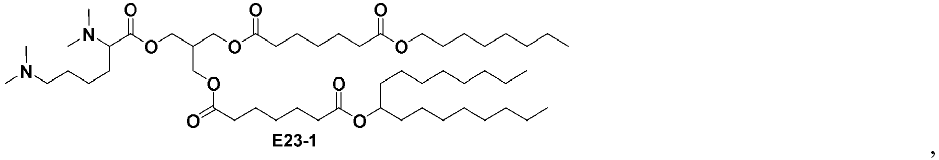 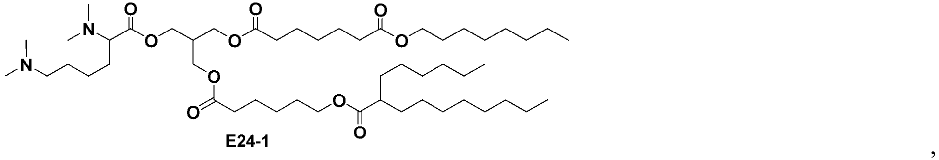 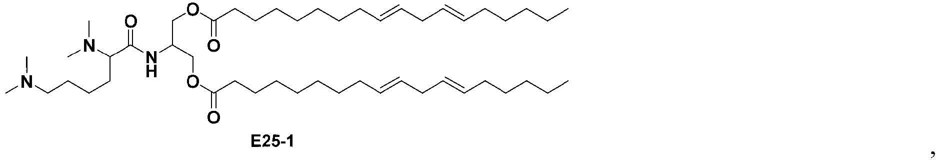 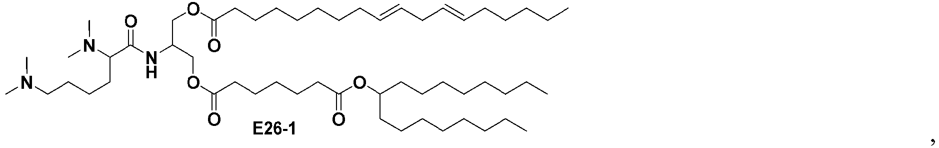 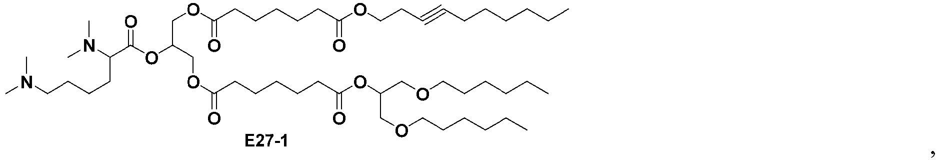 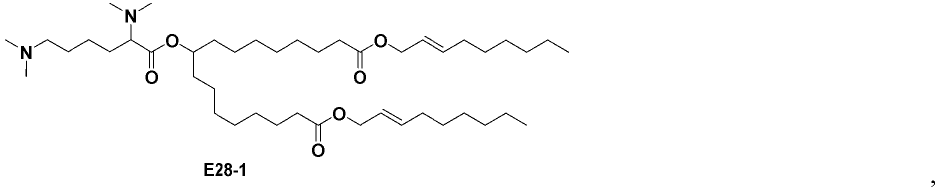 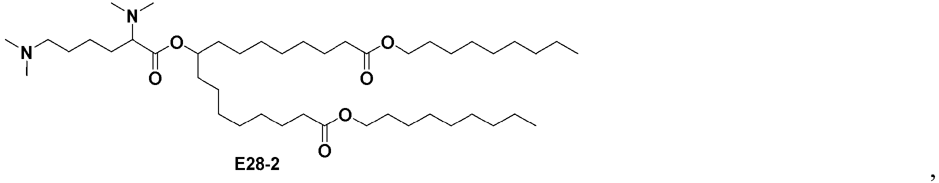 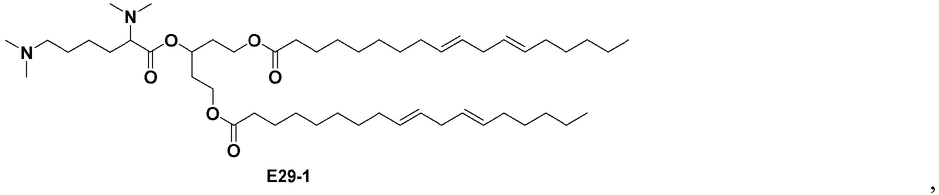 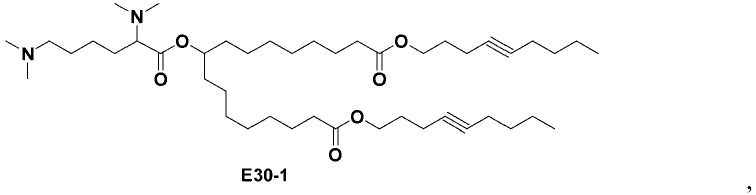 and 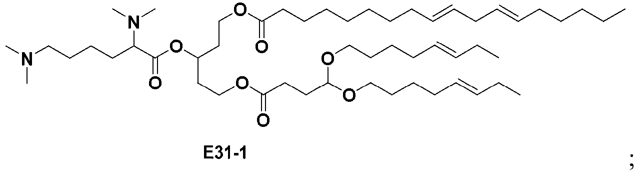 or, the structure of the cationic lipid is selected from the group consisting of the following structures: 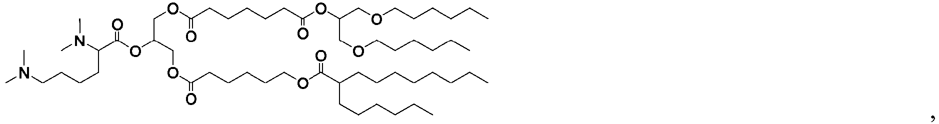 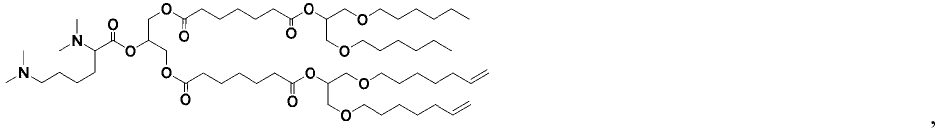 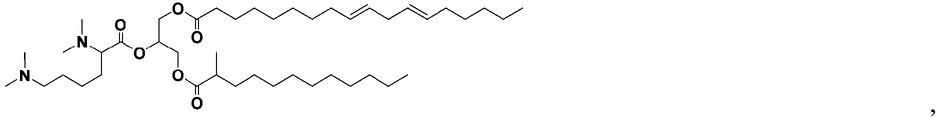 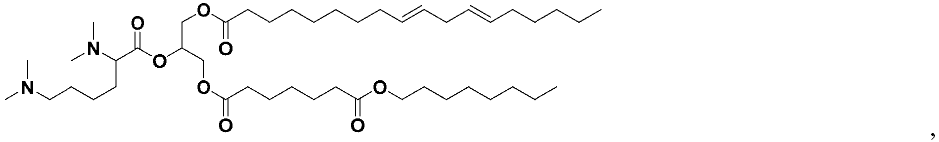 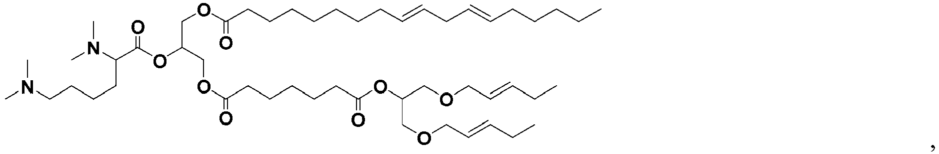 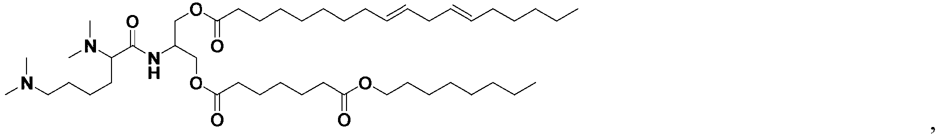 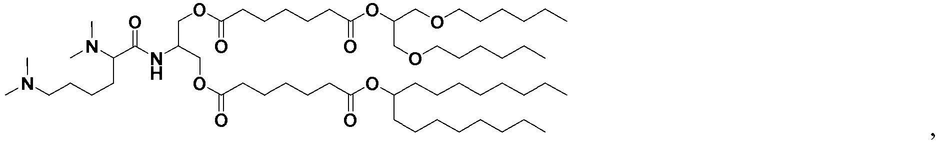 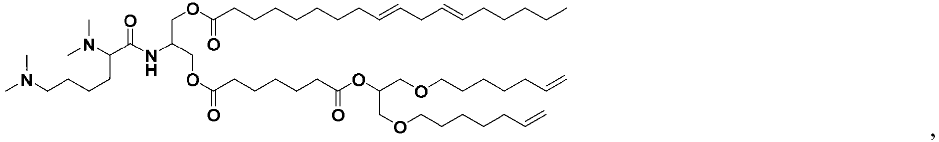 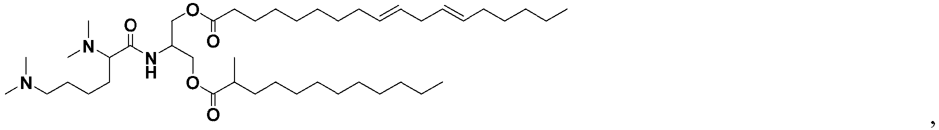 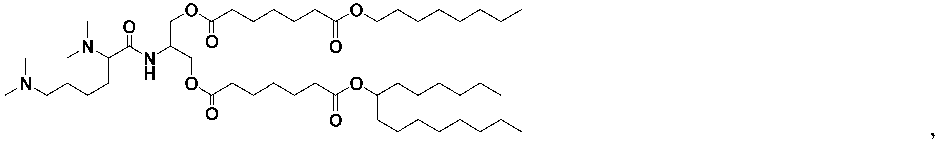 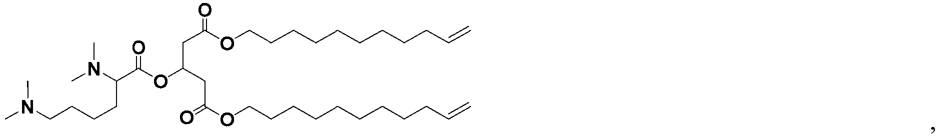 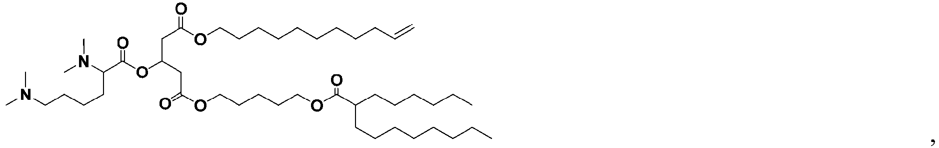 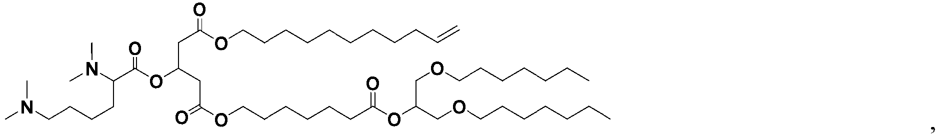 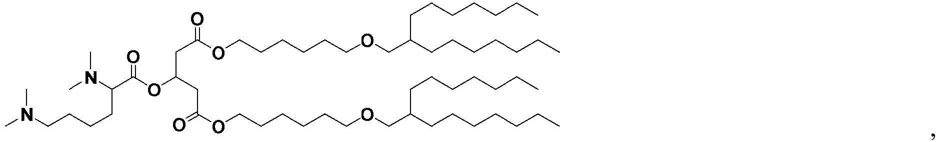 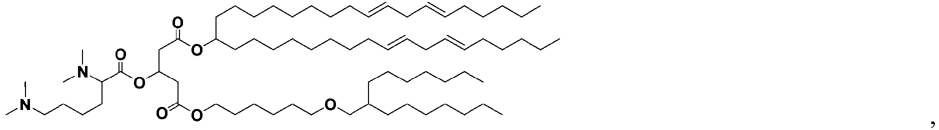 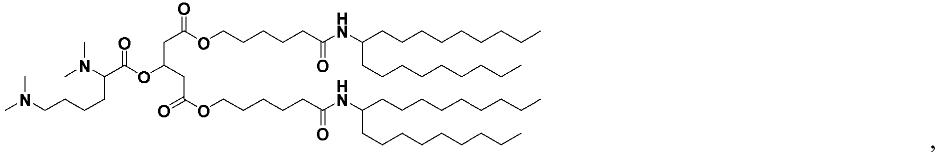 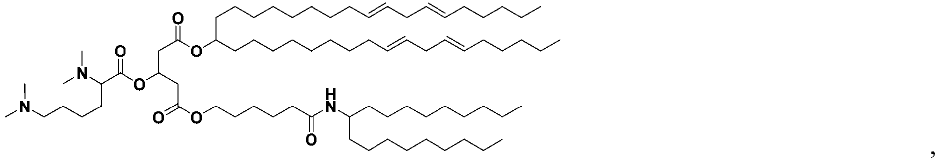 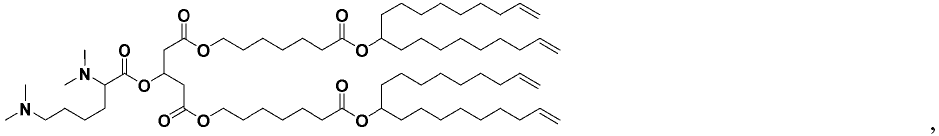 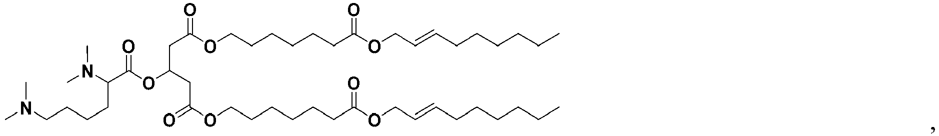 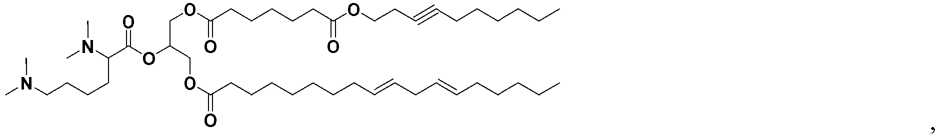 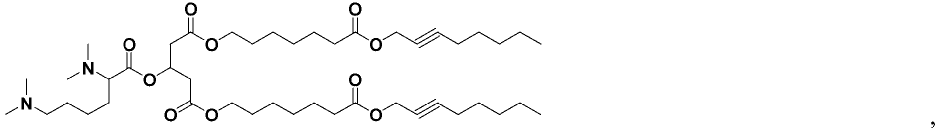 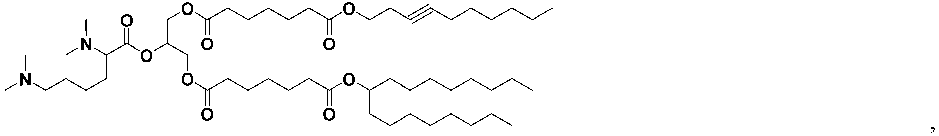 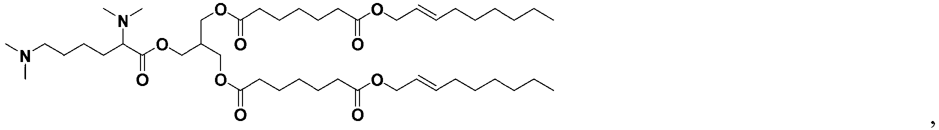 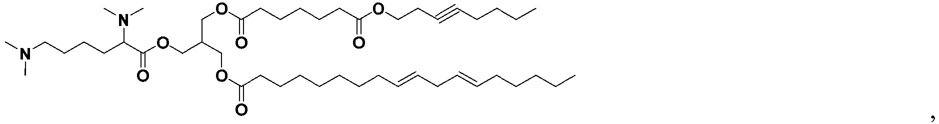 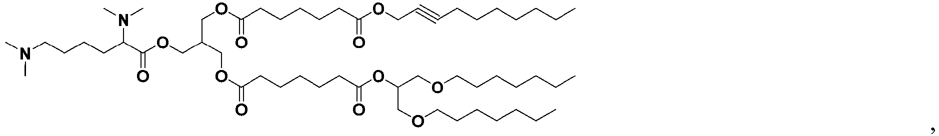 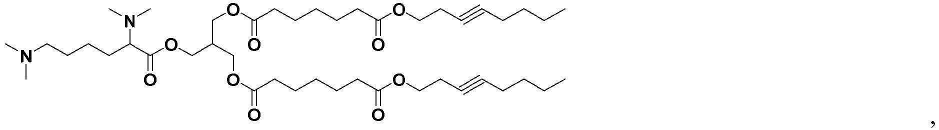 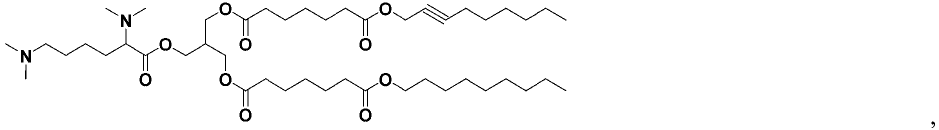 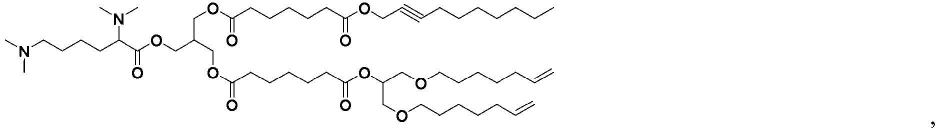 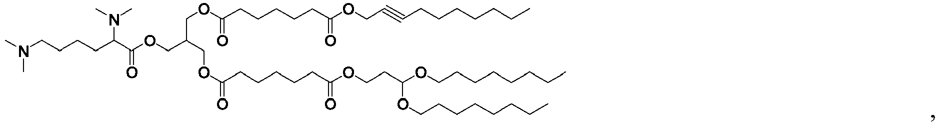 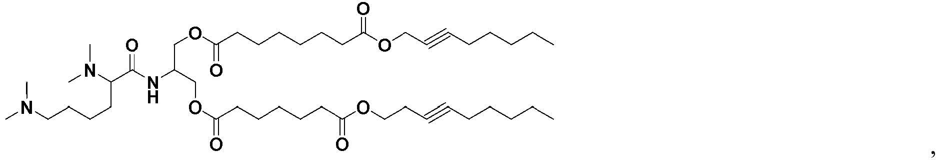 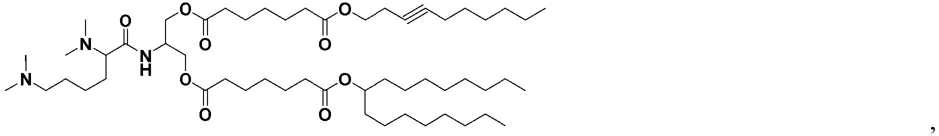 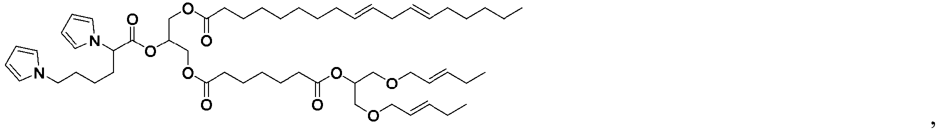 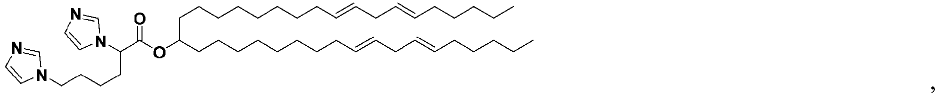 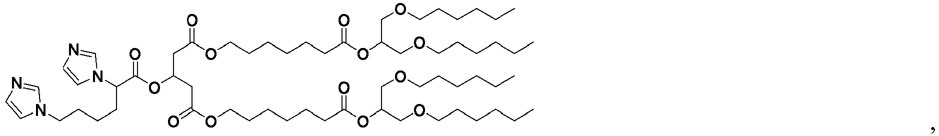 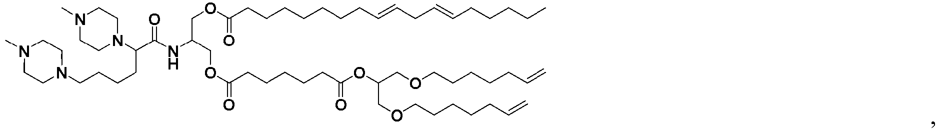 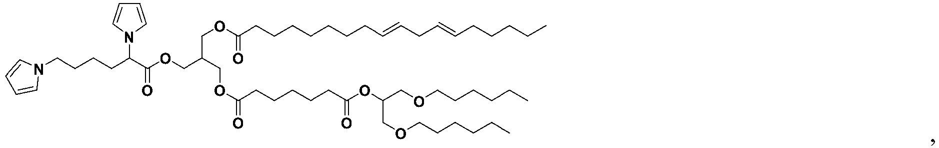 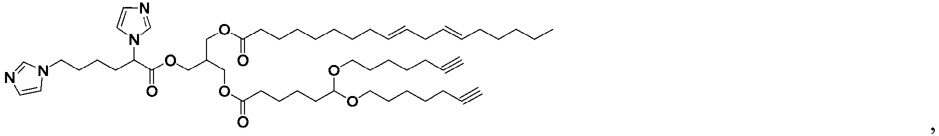 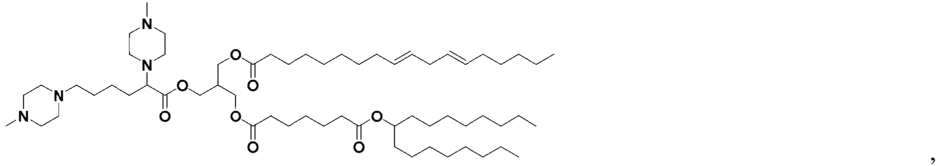 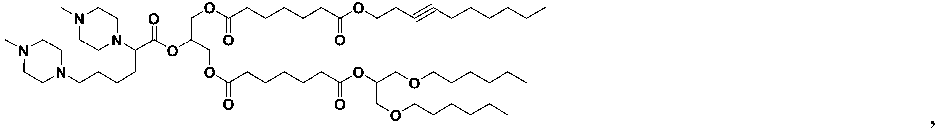 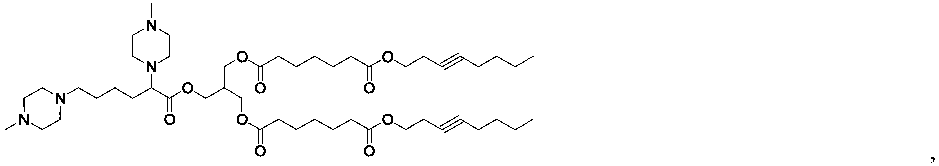 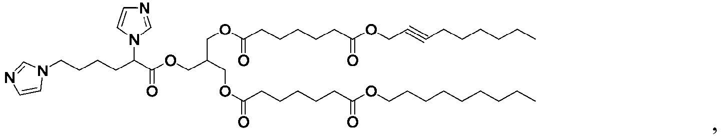 and

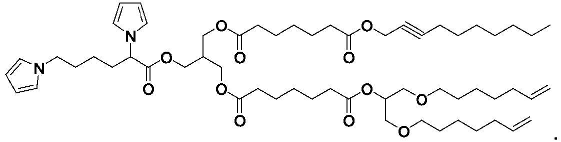

11. A lipid composition containing a cationic lipid of any one of claims **1** to **10.**

12. The lipid composition according to claim **11,** which additionally contains one or more types of lipids selected from the group consisting of phospholipid, steroid lipid, and PEGylated lipid, and is selected from any one of the following cases:
Case (1): the lipid composition additionally contains a phospholipid;
Case (2): the lipid composition additionally contains a steroid lipid;
Case (3): the lipid composition additionally contains a PEGylated lipid;
Case (4): the lipid composition additionally contains a phospholipid and a steroid lipid;
Case (5): the lipid composition additionally contains a phospholipid and a PEGylated lipid;
Case (6): the lipid composition additionally contains a steroid lipid and a PEGylated lipid;
Case (7): the lipid composition additionally contains a phospholipid, a steroid lipid, and a PEGylated lipid;
more preferably, the lipid composition additionally contains a phospholipid lipid, a steroid lipid, and a PEGylated lipid, simultaneously.

13. The lipid composition according to claim **12,** wherein the phospholipid is selected from the group consisting of 1,2-dilinoleoyl-sn-glycero-3-phosphocholine, 1,2-dimyristoleoyl-sn-glycero-phosphocholine, 1,2-dioleoyl-sn-glycero-3-phosphocholine, 1,2-dipalmitoyl-sn-glycero-3-phosphocholine, 1,2-distearoyl-sn-glycero-3-phosphocholine, 1,2-diundecanoyl-sn-glycero-3-phosphocholine, 1-plamitoyl-2-oleoyl-sn-glycero-3-phosphocholine, 1,2-di-*O*-octadecenyl-sn-glycero-3-phosphocholine, 1-oleoyl-2-cholesterylhemisuccinyl-sn-glycero-3-phosphocholine, 1-hexadecyl-sn-glycero-3-phosphocholine, 1,2-dilinolenoyl-sn-glycero-3-phosphocholine, 1,2-diarachidonoyl-sn-glycero-3-phosphocholine, 1,2-didecosahexaenoyl-sn-glycero-3-phosphocholine, 1,2-dioleoyl-sn-glycero-3-phosphoethanolamine, 1,2-diphytanyl-sn-glycero-3-phosphoethanolamine, 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didecosahexaenoyl-sn-glycero-3-phosphoethanolamine, 1,2-dioleoyl-sn-glycero-3-phospho-rac-(1-glycerol) sodium salt, dioleoyl phosphatidylserine, dipalmitoyl phosphatidylglycerol, palmitoyloleoyl phosphatidylethanolamine, distearoyl phosphatidylethanolamine, dipalmitoyl phosphatidylethanolamine, dimyristoleoyl phosphoethanolamine, 1-stearoyl-2-oleoyl-stearoylethanolamine, 1-stearoyl-2-oleoyl-phosphatidylcholine, sphingomyelin, phosphatidylcholine, phosphatidylethnolamine, phosphatidylserine, phosphatidylinositol, phosphatidic acid, palmitoyloleoyl phosphatidylcholine, lysophosphatidylcholine, lysophosphatidylethanolamine, and combinations thereof;
or, the steroid lipid is selected from the group consisting of cholesterol, coprostanol, sitosterol, ergosterol, campesterol, stigmasterol, brassicasterol, tomatine, ursolic acid, α-tocopherol, and combinations thereof;
or, the PEGylated lipid is selected from the group consisting of polyethylene glycol-1,2-dimyristoylglycerol, polyethylene glycol-distearoylphosphatidylethanolamine, PEG-cholesterol, polyethylene glycol-diacylglycerol, and polyethylene glycol-dialkyloxypropyl, and is specifically selected from the group consisting of polyethylene glycol 500-dipalmitoylphosphatidylcholine, polyethylene glycol 2000-dipalmitoylphosphatidylcholine, polyethylene glycol 500-distearylphosphatidylethanolamine polyethylene glycol 2000-distearylphosphatidylethanolamine, polyethylene glycol 500-1,2-oleoylphosphatidylethanolamine, polyethylene glycol 2000-1,2-oleoylphosphatidylethanolamine, polyethylene glycol 2000-2,3-dimyristoylglycerol, and combinations thereof;
or, the PEGylated lipid is selected from the group consisting of the following structures: 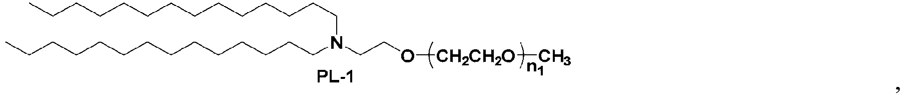 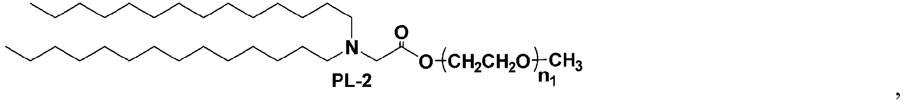 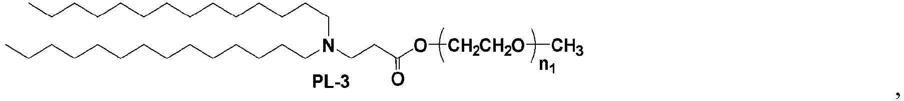 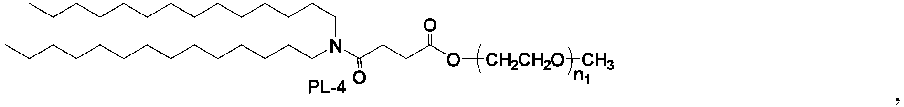 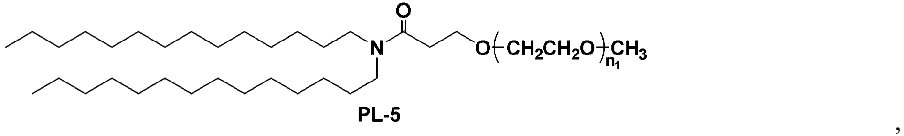 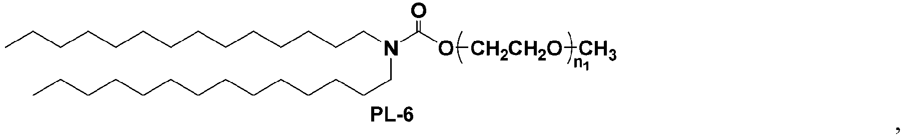 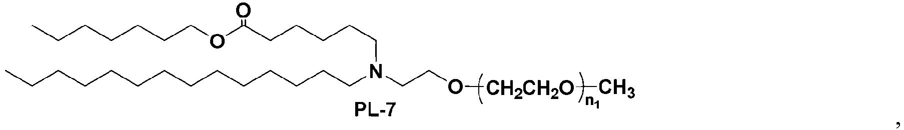 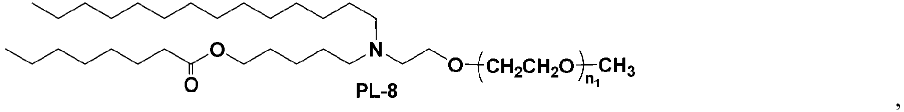 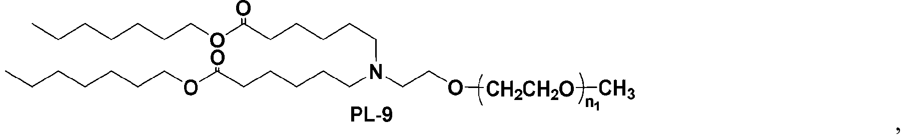 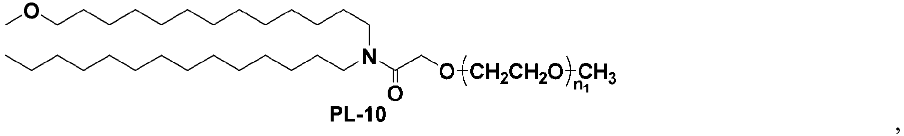 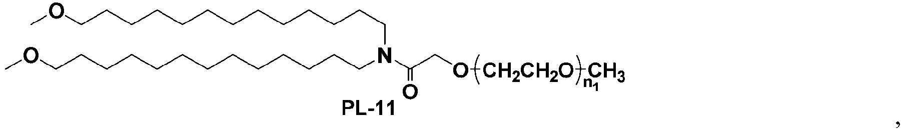 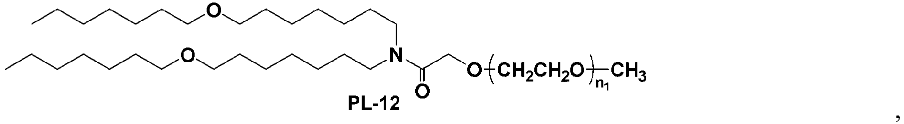 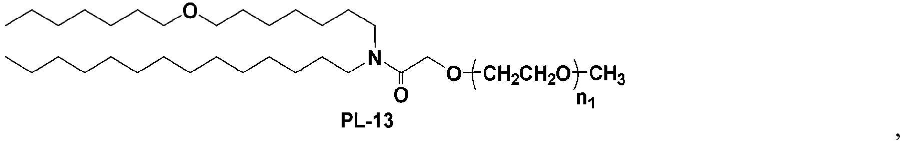 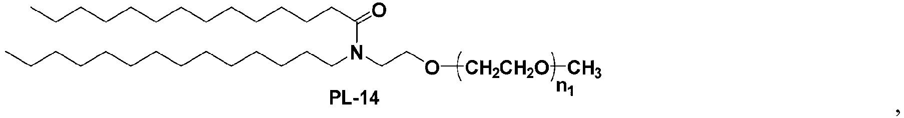 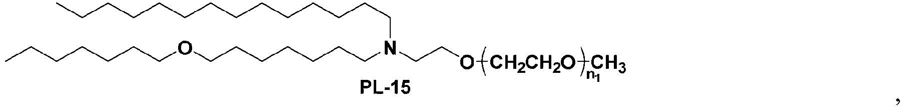 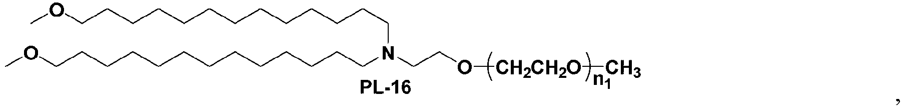 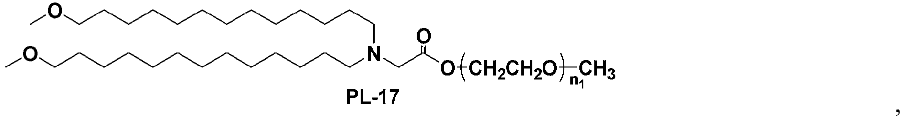 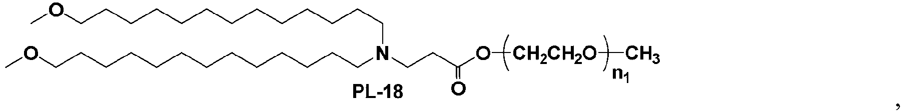 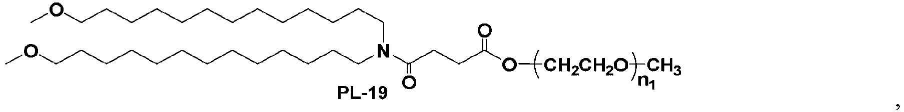 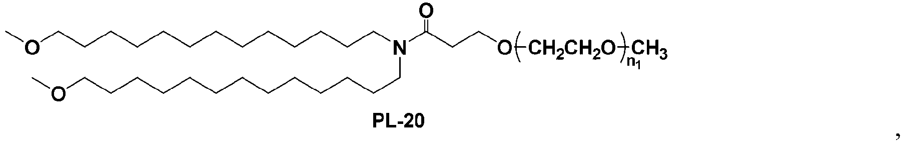 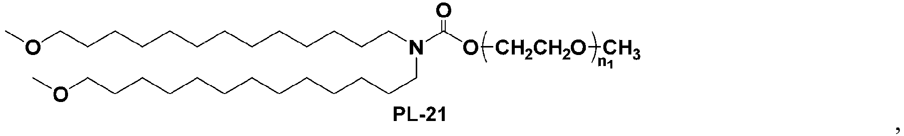 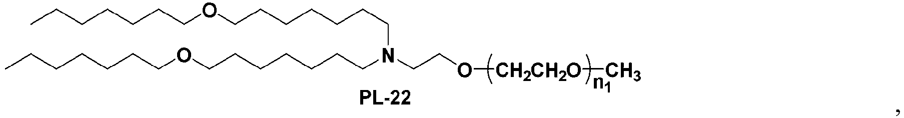 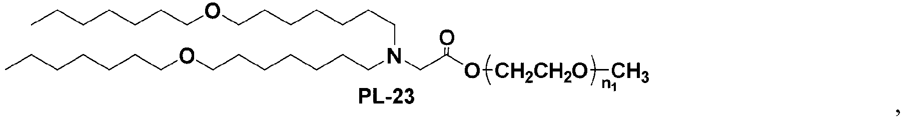 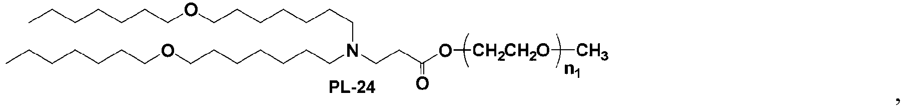 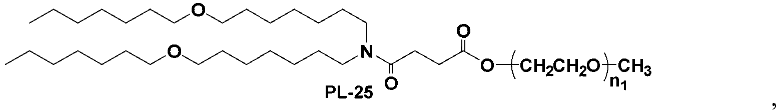 and 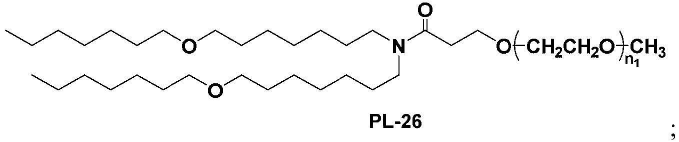 wherein, n₁ is an integer from 25 to 300, and more preferably, n₁ is selected from the group consisting of 44, 45, 46, 47 and 48.

14. The lipid composition according to any one of claims **11** to **13,** which contains 20% to 80% cationic lipid, 5% to 15% phospholipid, 25% to 55% steroid lipid, and 0.5% to 10% PEGylated lipid, wherein the percentages are the molar percentages of each lipid in the total lipids.

15. The lipid composition according to claim **12,** wherein the molar percentage of cationic lipid in the total lipids is 30% to 65%, more preferably 35%, 40%, 45%, 46%, 47%, 48%, 49%, 50%, or 55%;
or, the molar percentage of phospholipid in the total lipids is 7.5% to 13%, more preferably 8%, 9%, 10%, 11%, or 12%;
or, the molar percentage of steroid lipid in the total lipids is 35% to 50%, more preferably 40%, 41%, 42%, 43%, 44%, 45%, 46%, 47%, 48%, 49%, or 50%;
or, the molar percentage of PEGylated lipid in the total is 0.5% to 5%, preferably 1% to 3%, and more preferably 1.5%, 1.6%, 1.7%, 1.8%, or 1.9%.

16. A lipid pharmaceutical composition containing a lipid composition of any one of claims **11** to **15** and a drug, wherein the drug is selected from the group consisting of a nucleic acid drug, a gene vaccine, an antitumor drug, a small molecule drug, a peptide drug, and a protein drug.

17. The lipid pharmaceutical composition according to claim **16,** wherein the nucleic acid drug is selected from the group consisting of RNA, DNA, antisense nucleic acid, plasmid, interfering nucleic acid, aptamer, antagomir, and ribozyme; wherein, the RNA is selected from the group consisting of mRNA, saRNA, circRNA, miRNA, and siRNA; preferably, the nucleic acid drug is selected from the group consisting of DNA, mRNA, miRNA, and siRNA.

18. A lipid pharmaceutical composition according to any one of claims **16** to **17,** wherein the lipid pharmaceutical composition is used as a drug, and is selected from the group consisting of the following drugs: an antineoplastic agent, an antiviral agent, an antifungal agent, and a vaccine.

19. A formulation of lipid pharmaceutical composition containing a lipid pharmaceutical composition of any one of claims **15** to **17** and a pharmaceutically acceptable diluent or excipient; the diluent or excipient is preferably deionized water, ultrapure water, phosphate buffer, or normal saline, more preferably phosphate buffer or normal saline, and most preferably normal saline.

20. A liposome or lipid nanoparticle containing a lipid composition of any one of claims **11** to **15.**

21. The liposome or lipid nanoparticle according to claim **20,** wherein the lipid nanoparticle is an LNP pharmaceutical composition, an LPP pharmaceutical composition or a PNP pharmaceutical composition, preferably an LNP pharmaceutical composition, more preferably an LNP-nucleic acid pharmaceutical composition, more preferably an LNP-mRNA pharmaceutical composition.
